# EUROPÄISCHE PATENTSCHRIFT

(11) **EP 4 244 910 B1**
(45) Veröffentlichungstag und Bekanntmachung des Hinweises auf die Patenterteilung: **11.12.2024**
(21) Anmeldenummer: 21814717.1
(22) Anmeldetag: 11.11.2021
(51) Int. Cl.: C07C 251/12, C07C 251/24, C07C 255/61, H10K 85/30, H10K 50/155, H10K 50/17

(54) **CER-ETHYLENDIAMINKETONARTIGE- UND CER-SALENARTIGE-KOMPLEXE UND DEREN VERWENDUNG IN DER ORGANISCHEN ELEKTRONIK**
CERIUM-ETHYLENEDIAMINE KETONE-TYPE AND CERIUM-SALEN-TYPE COMPLEXES AND USE THEREOF IN ORGANIC ELECTRONICS
COMPLEXES DE TYPE CÉRIUM-ÉTHYLÈNEDIAMINE CÉTONE ET DE TYPE CÉRIUM-SALEN ET LEUR UTILISATION DANS L'ÉLECTRONIQUE ORGANIQUE

(30) Priorität: 16.11.2020 DE 102020130218
(43) Veröffentlichungstag der Anmeldung: 20.09.2023
(73) Patentinhaber: Credoxys GmbH c/o Institut for Applied Physics, 01187 Dresden (DE)
(72) Erfinder: DOROK, Sascha, 01157 Dresden (DE); PAPMEYER, Marcus, 01683 Nossen (DE); EYMANN, Leonard, 01097 Dresden (DE); STOLZ, Julia, 01277 Dresden (DE)
(74) Vertreter: Zellentin & Partner mbB Patentanwälte
(86) Internationale Anmeldenummer: PCT/EP2021/081392
(87) Internationale Veröffentlichungsnummer: WO 2022/101343

(56) Entgegenhaltungen:
- WO-A1-00/32719
- SCHLÄFER J. ET AL: "Fluorinated Cerium(IV) Enaminolates: Alternative Precursors for Chemical Vapor Deposition of CeO 2 Thin Films", INORGANIC CHEMISTRY, vol. 55, no. 11, 6 June 2016 (2016-06-06), Easton , US, pages 5422 - 5429, XP055888325, ISSN: 0020-1669, Retrieved from the Internet <URL:https://pubs.acs.org/doi/pdf/10.1021/acs.inorgchem.6b00348> DOI: 10.1021/acs.inorgchem.6b00348

## Beschreibung

Die vorliegende Erfindung betrifft ein elektronisch dotiertes Halbleitermaterial und ein elektronisches Bauteil, umfassend Cer-ethylendiaminketonartige - und Cer-salenartige-Komplexe. Ein weiterer Gegenstand der Erfindung ist die Verwendung der Cerethylendiaminketonartigen - und Cer-salenartigen-Komplexe als Elektronenakzeptoren, speziell als p-Dotanden und Elektronentransportmaterialien in organisch elektronischen Bauteilen. Ein weiterer Gegenstand der Erfindung sind neue Cer-ethylendiaminketonartige - und Cer-salenartige-Komplexe.

### HINTERGRUND DER ERFINDUNG

Die organische Elektronik beschäftigt sich mit der Entwicklung, Charakterisierung und Anwendung neuer Materialien, sowohl auf Basis von kleinen organischen Molekülen als auch von Polymeren mit bestimmten gewünschten elektronischen Eigenschaften für die Herstellung von elektronischen Bauteilen. Dazu gehören beispielsweise organische Feldeffekttransistoren (OFETs), wie organische Dünnschichttransistoren (OTFTs), organische Elektrolumineszenzvorrichtungen, wie organische lichtemittierende Dioden (OLEDs), organische Solarzellen (OSCs), z. B. Exzitonsolarzellen, farbstoffsensibilisierte Solarzellen (DSSCs) oder Perowskitsolarzellen, die Elektrofotografie, z. B. fotoleitfähige Materialien in organischen Fotoleitern (OPC), organische optische Detektoren, organische Fotorezeptoren, lichtemittierende elektrochemische Zellen (LECs) und organische Laserdioden.

Es ist bekannt, dass organische Halbleitermatrizes durch Dotierung hinsichtlich ihrer elektrischen Leitfähigkeit stark beeinflusst werden können. Solche organischen Halbleitermatrixmaterialien können entweder aus Verbindungen mit Elektronendonoreigenschaften (p-Leiter) oder aus Verbindungen mit Elektronenakzeptoreigenschaften (n-Leiter) aufgebaut werden. Anders als anorganische Halbleiter, weisen organische Halbleiter nur eine sehr geringe intrinsische Ladungsträgerkonzentration auf. Zur Erzielung guter Halbleitereigenschaften werden organische Halbleitermatrixmaterialien daher in der Regel dotiert. Dabei werden zur n-Dotierung starke Elektronendonatoren (n-Dotanden) eingesetzt, die ein Elektron auf das LUMO der Halbleitermatrix übertragen (n-Dotierung), wodurch ein freies Elektron auf der Matrix entsteht (SOMO). Zur p-Dotierung werden starke Elektronenakzeptoren (p-Dotanden) eingesetzt, die ein Elektron vom HOMO der Halbleitermatrix entfernen (p-Dotierung), wodurch ein Loch zurückbleibt. Mit anderen Worten muss bei der p-Dotierung das LUMO des Dotanden unter der HOMO-Energie der Matrix liegen. Der Dotand agiert als Akzeptor und hinterlässt ein bewegliches Loch (SOMO) in der Matrix.

Bekannte p-Dotanden für Elektronendonormaterialien sind Elektronenakzeptoren wie Tetracyanochinonmethan (TCNQ), 2,3,5,6-Tetrafluortetracyano-1,4-benzochinonmethan (F4TCNQ), Trinaphthylene (HATNA), Metalloxide, wie MoOs oder WO₃, oder Radialene, wie sie in EP 2180029 beschrieben werden. Die Akzeptormoleküle erzeugen durch Elektronentransferprozesse in den Halbleitermatrixmaterialien sogenannte Löcher (Lochtransportmaterialien), und die Leitfähigkeit des Halbleitermatrixmaterials (Lochtransportmaterials) wird je nach Anzahl und Mobilität der Löcher mehr oder weniger stark verändert.

Die zuvor beschriebenen Verbindungen bzw. Verbindungsklassen haben jedoch Nachteile für einen technischen Einsatz bei der Herstellung dotierter Halbeiter oder entsprechender elektronischer Bauteile mit solchen dotierten Schichten. Die genannten Verbindungen bzw. Verbindungsklassen sind beispielsweise zu leicht flüchtig, haben einen zu hohen Absorptionskoeffizienten, weisen eine instabile Verdampfungsrate auf und/oder zeigen eine geringe Thermostabilität. Hinzu kommt, dass einige dieser Substanzklassen sehr hohe Herstellungskosten haben.

Es besteht deshalb weiterhin ein großer Bedarf an Verbindungen, die leicht verfügbar oder herstellbar sind, sich zur Dotierung von Elektronendonormaterialien eignen und die oben beschriebenen Nachteile nicht aufweisen.

Es sind nur wenige Cer-ethylendiaminketonartige-Komplexe und keine Cer-salenartigen (Cer-bis(salicyliden)ethylendiaminartige-) Komplexe bekannt. So werden einige wenige Komplexe des Cer-(IV) in der Literatur beschrieben. J. Schläfer et al., Inorg. Chem., 2016, 55, 5422-5429 beschreibt homopleptische Cer(IV) Komplexe, die folgende Liganden aufweisen: N,N'-bis(4,4,4,-trifluorobut-1-en-3-on)-ethylendiamin, N,N'-bis(4,4,5,5,5-pentafluoropent-1-en-3-on)-ethylendiamin, N,N'-bis(4,4,5,5,6,6,6-heptafluorohex-1-en-3-on)-ethylendiamin und N,N'-bis(4,4,4,-trifluorobut-1-en-3-on)-propylendiamin.

SCHLÄFER J. ET AL: "Fluorinated Cerium(IV) Enaminolates: Alternative Precursors for Chemical Vapor Deposition of CeO 2 Thin Films",INORGANIC CHEMISTRY,Bd. 55, Nr. 11 6. Juni 2016 (2016-06-06), Seiten 5422-5429 offenbart Cer (IV) Komplexe.

WO00/32719 (A1) offenbart ein elektronisches Bauteil umfassend eine Cer (IV) Verbindung.

In D.W. Wester et.al: "Electrochemical studies of cerium chelate complexes", Inorg. Chem. 1985, 24, 4435-4437, werden folgende homopleptische Cer(IV) Komplexe beschrieben:

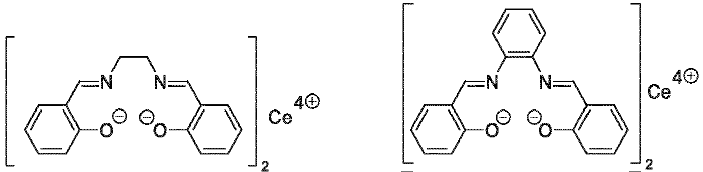

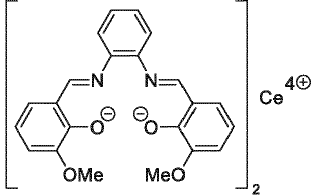

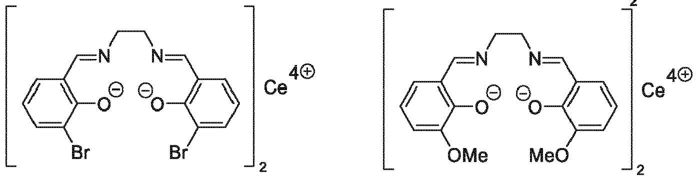

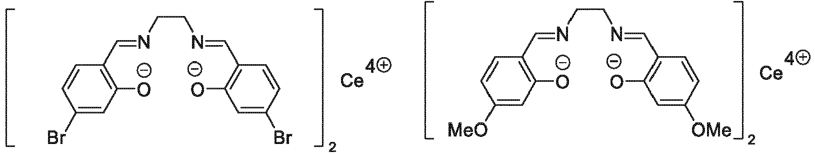

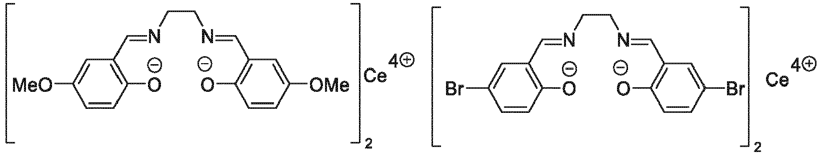

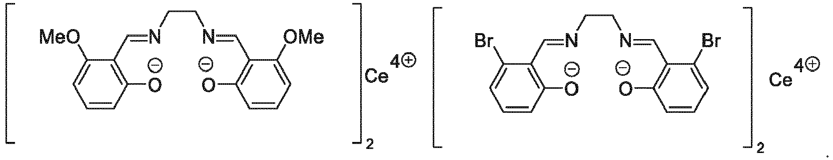

N. K. Dutt et al. (Chemistry of Lanthanons- XIX Ethylenediamine bis-Acetylacetone Complexes of Rare-Earths, J. Inorg. Nucl. Chem., 1968, Vol. 30, S. 3273 - 3280) beschreibt die Synthese von Ethylendiamin-bis-acetylaceton Komplexen der seltenen Erden. Unter anderem werden Ce(III)-Komplexe genannt, [Ce(Ethylendiamin-bis-aceylaceton))Cl₃] und [Ce(Ethylendiamin-bis-acetylaceton))NO₃]. In diesen Komplexen ist der Ligand neutral und trägt somit keine Ladung. Erfindungsgemäß ist der Ligand zweifach negativ geladen.

V. Balasubramanian et al. (Synthesis and Reactivity of Quadridentate Schiff Base Chelates of Lanthanide(III) Ions, Asian Journal of Chemistry 2003, Vol. 15, No. 1, S. 139 - 143) beschreibt die Synthese von Lathanid (III) Chelatkomplexe. Unter anderem werden Ce(III)-Komplexe genannt, [Ce(Ethylendiamin-bis-acytylaceton)Cl₃] und [Ce(Ethylendiamin-bis-acytylaceton-α-naphtylisocyanat))Cl₃], wobei Ethylendiamin-bisacytylaceton-Ligand in der Keto-Form vorliegt. In diesen Komplexen ist der Ligand neutral und trägt somit keine Ladung. Erfindungsgemäß ist der Ligand zweifach negativ geladen.

J. H. Timmons (Template Synthesis and Crystal and Molecular Structure of Bis[1,1,1,12,12,12-hexafluoro-2, 11-bis(trifluoromethyl)-4,9-dimethyl-2,11-diolato-5,8-diazadodeca-4,8-diene(2-)]cerium(IV), CeC28H28F24O4N4, a fluorinated schiff base complex of eight-coordinate Cerium (IV), Inorganic Chemistry 1980, Vol. 19, No. 12, S. 3553 - 3557) beschreibt einen Cer (IV)-Komplex. Bei dem Liganden dieses Komplex liegt eine andere Konnektivität der Atome vor. Mit anderen Worten, der Ligand beruht nicht auf einer Acetylaceton-Struktur.

WO 2021/048044 beschreibt Cer-(IV) Diketonatkomplexe als Elektronenakzeptoren, speziell als p-Dotanden und Elektronentransportmaterialien in organisch elektronischen Bauteilen eingesetzt werden können.

Bislang war es unbekannt, Cer-(IV)-ethylendiaminketonartige- und/oder Cer-(IV)-salenartige- und/oder (Cer-(IV)-bis(salicyliden)ethylendiaminartige-) Komplexe (im Folgenden Cer-(IV)-Komplexe genannt) in organischen Halbleitermaterialien einzusetzen. Insbesondere ist bislang nicht beschrieben, derartige Cer (IV)-Komplexe als p-Dotanden, als Elektronentransportmaterialien oder als Elektronenakzeptoren zu verwenden.

Überraschenderweise wurde jetzt gefunden, dass sich derartige Cer-(IV)-Komplexe hervorragend als p-Dotanden einsetzen lassen. Darüber hinaus wurde gefunden, derartige Cer-(IV)-Komplexe als Elektronentransportmaterialien (ETM) in organisch-elektronischen Bauteilen, wie organischen lichtemittierenden Dioden (OLED), Photovoltaikzellen, organischen Solarzellen (OPV), organischen Dioden oder organischen Transistoren, zu verwenden.

Darüber hinaus lassen sich viele dieser Cer-(IV)-Kemplexe sehr gut im Vakuum verdampfen und weisen mitunter eine hohe Thermostabilität auf. Somit eignen diese sich grundsätzlich für beide Varianten der Prozessierung organisch-elektronischer Bauteile, die Vakuumbeschichtung (vapour deposition) und die Lösungsmittel-basierte Prozessierung (solution processing).

### ZUSAMMENFASSUNG DER ERFINDUNG

Ein erster Gegenstand der Erfindung ist ein elektronisches Bauteil, umfassend mindestens eine Verbindung der allgemeinen Formel (I)

Ce⁴⁺[L₁L₂]⁴⁻ (I),

wobei L₁ und L₂ jeweils ein tetradentater Ligand ist, die unabhängig voneinander ausgewählt sind unter Liganden der Formel (I.1) 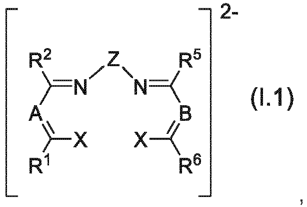 wobei
- X: unabhängig voneinander für O oder S stehen;
- Z: für eine Brückengruppe steht, die 2 oder 3 Kohlenstoffatome zwischen den Stickstoffatomen aufweist, wobei die Kohlenstoffatome jeweils unsubstituiert oder mit 1, 2, 3, 4, 5 oder 6 identischen oder verschiedenen Resten R¹² substituiert sind, wobei zwei benachbarte Kohlenstoffatome auch durch eine Doppelbindung miteinander verbunden sein können, oder 2 oder 3 der Kohlenstoffatome Teil eines 1-kernigen, 2- kernigen oder 3- kernigen aromatischen oder heteroaromatischen C₆-C₁₄-Ringsystems sind, wobei das heteroaromatische Ringsystem 4 bis 13 Kohlenstoffatomen aufweist und 1, 2 oder 3 identische oder verschiedene Heteroatome oder heteroatomhaltige Gruppen als Ringglieder hat, die ausgewählt sind unter N, NR⁸, O, S, SO und SO₂ und wobei das aromatische oder heteroaromatische Ringsystem unsubstituiert oder mit 1, 2, 3, 4 oder 5 identischen oder verschiedenen Resten R⁹ substituiert ist;
- A und B: unabhängig voneinander für N oder CR⁷ stehen;
- R¹ und R⁶: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind; oder
- R¹ und A: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
- R⁶ und B: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind;
- R² und R⁵: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
- R² und A: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
- R⁵ und B: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R^{4a}R^{4b}: unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
- R⁷: ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
- R⁸: ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert ist;
- R⁹: ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
- R¹⁰: ausgewählt ist unter CN, Halogen, C₁-C₄-Alkyl und C₁-C₄-Haloalkyl;
- R¹¹: ausgewählt ist unter CN, Halogen, C₁-C₄-Alkyl und C₁-C₄-Haloalkyl;
- R¹²: unabhängig voneinander ausgewählt sind unter Halogen, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind.

Ein weiterer Gegenstand der Erfindung ist ein elektronisches Bauteil, umfassend mindestens eine Verbindung der allgemeinen Formel (I)

Ce⁴⁺[L₁L₂]⁴⁻ (I),

wobei L₁ und L₂ jeweils ein tetradentater Ligand ist, die unabhängig voneinander ausgewählt sind unter Liganden der Formel (II) 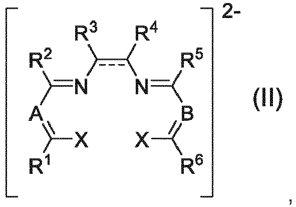 wobei
- -̅ -̅ -̅ -̅ -̅ -̅: zwischen der Gruppe CR³-CR⁴ für eine Einfach- oder Doppelbindung steht,
- X: unabhängig voneinander für O oder S stehen;
- A und B: unabhängig voneinander für N oder CR⁷ stehen;
- R¹ und R⁶: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
- R¹ und A: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
- R⁶ und B: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind;
- R³ und R⁴: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
- R³ und R⁴: gemeinsam mit den Kohlenstoffen, an denen sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind;
- R² und R⁵: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
- R^{4a}R^{4b}: unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
- R⁷: ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
- R⁸: ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert sind;
- R⁹: ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
- R¹⁰: ausgewählt ist unter CN, Halogen, C₁-C₄-Alkyl und C₁-C₄-Haloalkyl.

Ein weiterer Gegenstand der Erfindung ist ein dotiertes Halbleitermatrixmaterial, umfassend mindestens einen Elektronendonor und mindestens eine Verbindung der Formel (I), wobei L₁ und L₂ jeweils ein tetradentater Ligand ist, die unabhängig voneinander ausgewählt sind unter Liganden der Formel (1.1) oder Formel (II), wobei die Reste X, Z, A, B, R¹, R², R³, R⁴, R⁵ und R⁶ die zuvor und wie im Folgenden definierten Bedeutungen haben.

Ein weiterer Gegenstand der Erfindung ist die Verwendung einer Verbindung (I), wobei L₁ und L₂ jeweils ein tetradentater Ligand ist, die unabhängig voneinander ausgewählt sind unter Liganden der Formel (1.1) oder Formel (II), wobei die Reste X, Z, A, B, R¹, R², R³, R⁴, R⁵ und R⁶ die zuvor und wie im Folgenden definierten Bedeutungen haben,
- als organischer Halbleiter,
- als Dotierstoff in organischen Halbleitermatrixmaterialien, insbesondere als p-Dotand in Lochtransportschichten,
- als Ladungsinjektor in einer Ladungsinjektionsschicht,
- als Elektronentransportschicht,
- als Kathodenmaterial in organischen Batterien,
- als elektrochromes Material,
- als Elektronenakzeptor.

Ein weiterer Gegenstand der Erfindung ist die Verwendung von durch Reduktion einer Verbindung (I), wie zuvor und im Folgenden definiert, erhaltenen Ce(III)-Komplexanionen oder von Ladungstransferkomplexen einer Verbindung (I), wie zuvor und im Folgenden definiert, mit Elektronendonoren, als organischer Leiter, insbesondere als organischer Leiter, als elektrochromes Material, als Charge-TransferKomplex oder als Ferromagneten.

Ein weiterer Gegenstand der Erfindung sind Verbindungen der allgemeinen Formel (I)

Ce⁴⁺[L₁L₂]⁴⁻ (I),

deren Ladungstransferkomplexe, deren Reduktionsprodukte, und Mischungen davon, wobei L₁ und L₂ jeweils ein tetradentater Ligand ist, die unabhängig voneinander ausgewählt sind unter Liganden der Formel (I.1) 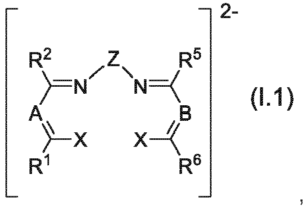 wobei
- X: unabhängig voneinander für O oder S stehen;
- Z: für eine Brückengruppe steht, die 2 oder 3 Kohlenstoffatome zwischen den Stickstoffatomen aufweist, wobei die Kohlenstoffatome jeweils unsubstituiert oder mit 1, 2, 3, 4, 5 oder 6 identischen oder verschiedenen Resten R¹² substituiert sind, wobei zwei benachbarte Kohlenstoffatome auch durch eine Doppelbindung miteinander verbunden sein können, oder 2 oder 3 der Kohlenstoffatome Teil eines 1-kernigen, 2-kernigen oder 3-kernigen aromatischen oder heteroaromatischen C₆-C₁₄-Ringsystems sind, wobei das heteroaromatische Ringsystem 4 bis 13 Kohlenstoffatomen aufweist und 1, 2 oder 3 identische oder verschiedene Heteroatome oder heteroatomhaltige Gruppen als Ringglieder hat, die ausgewählt sind unter N, NR⁸, O, S, SO und SO₂ und wobei das aromatische oder heteroaromatische Ringsystem unsubstituiert oder mit 1, 2, 3, 4 oder 5 identischen oder verschiedenen Resten R⁹ substituiert ist;
- A und B: unabhängig voneinander für N oder CR⁷ stehen;
- R¹ und R⁶: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
- R¹ und A: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind, oder
- R⁶ und B: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R² und R⁵: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
R² und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
R⁵ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R^{4a}R^{4b}: unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
- R⁷: ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
- R⁸: ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert sind;

- R⁹: ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
- R¹⁰: ausgewählt ist unter CN, Halogen, C₁-C₄-Alkyl und C₁-C₄-Haloalkyl;
- R¹¹: ausgewählt ist unter CN, Halogen, C₁-C₄-Alkyl und C₁-C₄-Haloalkyl;
- R¹²: unabhängig voneinander ausgewählt sind unter Halogen, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
wobei folgende Verbindungen und deren Reduktionsprodukte ausgenommen sind:

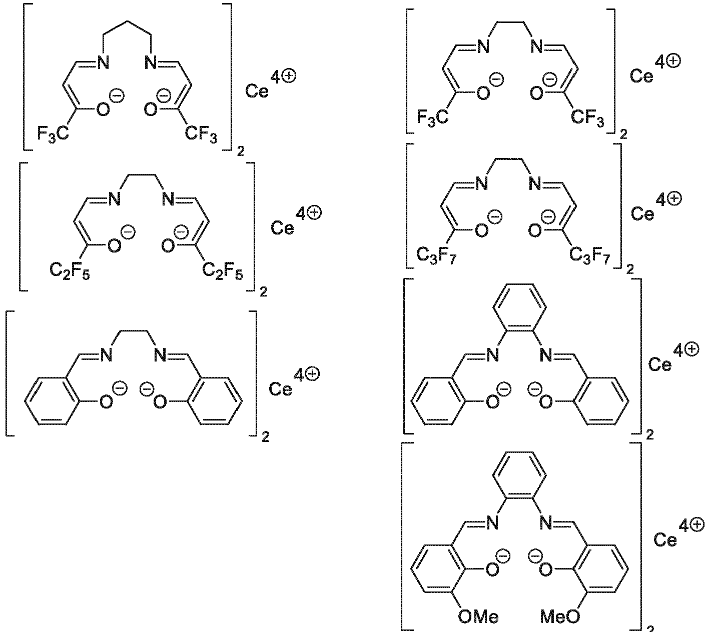

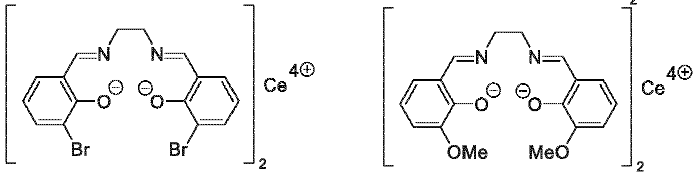

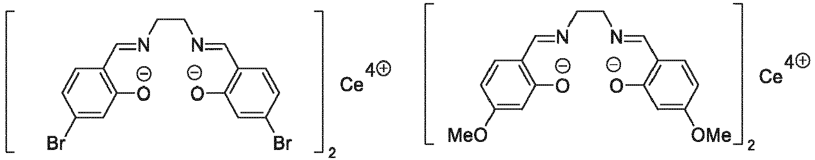

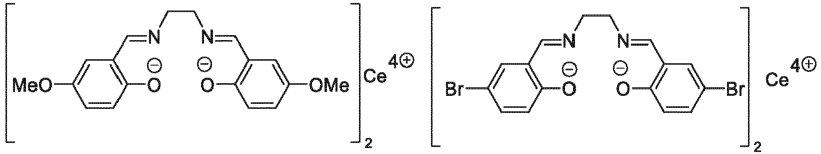

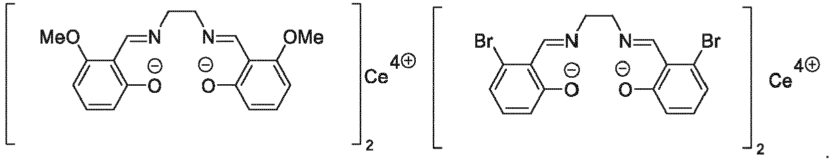

Ein weiterer Gegenstand der Erfindung sind Verbindungen der allgemeinen Formel (I)

Ce⁴⁺[L₁L₂]⁴⁻ (I),

deren Ladungstransferkomplexe, deren Reduktionsprodukte, und Mischungen davon, wobei L₁ und L₂ jeweils ein tetradentater Ligand ist, die unabhängig voneinander ausgewählt sind unter Liganden der Formel (II) 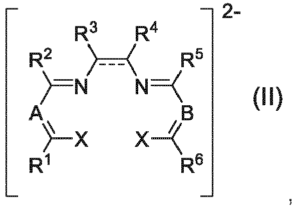 wobei
- -̅ -̅ -̅ -̅ -̅ -̅: zwischen der Gruppe CR³-CR⁴ für eine Einfach- oder Doppelbindung steht,
- X: unabhängig voneinander für O oder S stehen;
- A und B: unabhängig voneinander für N oder CR⁷ stehen;
- R¹ und R⁶: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R¹ und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind, oder
R⁶ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R³ und R⁴: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R³ und R⁴ gemeinsam mit den Kohlenstoffen, an denen sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R² und R⁵: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;

- R^{4a}R^{4b}: unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
- R⁷: ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
- R⁸: ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert sind;
- R⁹: ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl und C₁-C₄-Haloalkyl substituiert ist;
- R¹⁰: ausgewählt ist unter CN, Halogen, C₁-C₄-Alkyl und C₁-C₄-Haloalkyl,

wobei folgende Verbindungen und deren Reduktionsprodukte der Formel (I) ausgenommen sind, wobei
L₁ = L₂ = N,N'-bis(4,4,4,-trifluorobut-1-en-3-on)-ethylendiamin,
L₁ = L₂ = N,N'-bis(4,4,5,5,5-pentafluoropent-1-en-3-on)-ethylendiamin,
L₁ = L₂ = N,N'-bis(4,4,5,5,6,6,6-heptafluorohex-1-en-3-on)-ethylendiamin und folgende Verbindungen und deren Reduktionsprodukte

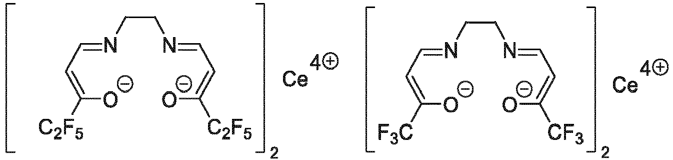

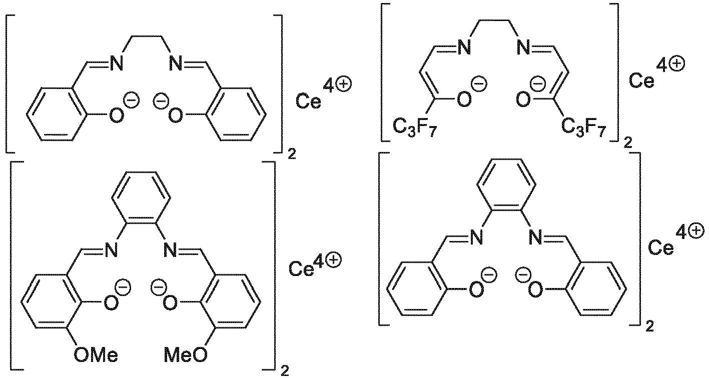

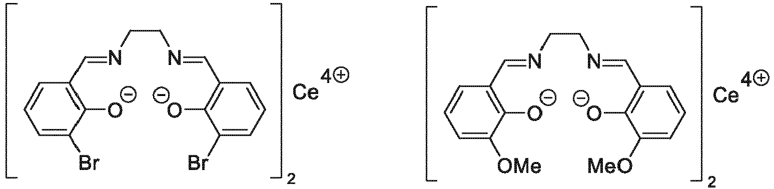

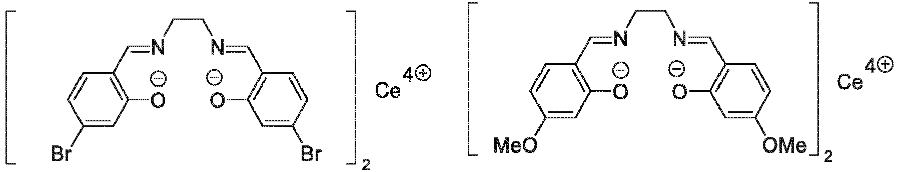

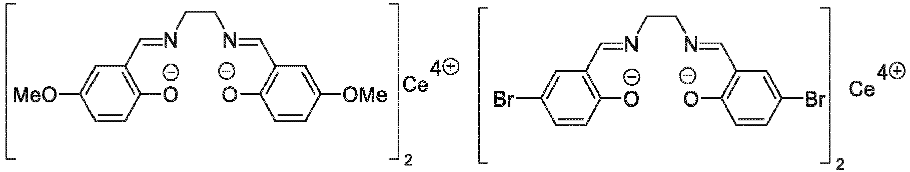

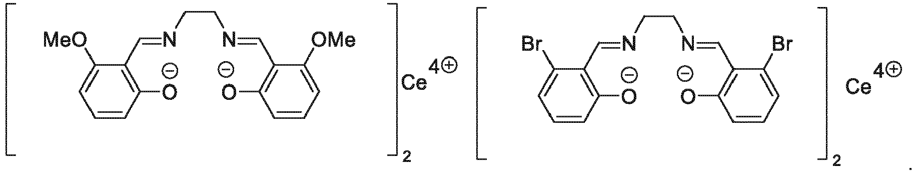

Die Erfindung hat folgende Vorteile:
- Die erfindungsgemäßen Cer-(IV)-Komplexe haben nur geringe Herstellungskosten.
- Die erfindungsgemäßen Cer-(IV)-Komplexe eigenen sich in vorteilhafter Weise als Elektronenakzeptoren zur Verwendung als p-Dotanden sowie als Elektronentransportmaterialien in organisch-elektronischen Bauteilen.
- Die erfindungsgemäßen Cer-(IV)-Komplexe weisen eine bessere Leitfähigkeit gegenüber bekannten Elektronenakzeptoren auf.
- Die erfindungsgemäßen Cer-(IV)-Komplexe zeigen eine verbesserte Thermostabilität der dotierten Schichten im Vergleich zum Stand der Technik.
- Außerdem zeichnen sich die erfindungsgemäßen Cer-(IV)-komplexe durch eine höhere Dotiereffizienz aus.
- Die erfindungsgemäßen Cer-(IV)-Komplexe weisen nur eine geringe Absorption der dotierten Schicht auf. Somit können parasitäre Absorptionen und Emissionen verringert oder sogar verhindert werden.
- Die erfindungsgemäßen Cer-(IV)-Komplexe eignen sich für die Herstellung von organischen und hybriden opto-elektronischen Bauteilen, sowohl mittels Lösungsmittelprozessierung als auch durch Vakuumsprozessierung.

Die Begriffe Cer- Verbindung und Cer- Komplex werden synonym verwendet und werden durch die Formel (I) definiert. Die Liganden in Abwesenheit des Metallatoms (Cer-Atom), werden durch die Formel (II) definiert.

Der Begriff Reduktionsprodukte einer Verbindung (I) sind Anionen-Komplexe, insbesonder Ce(III)-anionen-Komplexe, wobei das entsprechende Gegenion aus dem lochleitenden Material (HTL) der Matrix gespendet wird. Halbleitermatrixmaterialien sind unten definiert.

Im Sinne der Erfindung sind [Ce(ac₂en)Cl₃], [Ce(enac)₂Cl₃] und [Ce(enac)₂(NO₃)₃] keine Reduktionsprodukte der Verbindung (I), wobei ac₂en und enac die gleichen Bedeutungen haben und insbesonde ac₂en für Bis(acetylaceton)ethylendiamin in der Keto-form und enac für Ethylendiamin-bis-acetylaceton steht.

Unter dem Begriff Ladungstransferkomplexe einer Verbindung (I) sind deren ionische Paarungen mit den Radikal-Kationen des Matrixmaterials (Lochtransportmaterials, HTL) zu verstehen.

Im Rahmen der Erfindung representiert die Formel (II) exemplarisch eine mesomere Grenzstruktur, auf die die Liganden L₁ und L₂ nicht beschränkt sind. Weitere mesomere Genzstrukturen sind ausgewählt unter der Formel a, b, c und d

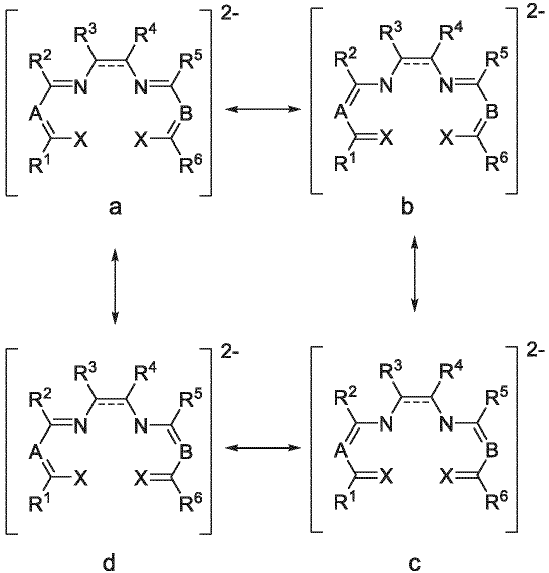

Die mesomere Grenzstrukture a, b, c und d gelten analog auch für die Liganden der Formel (I.1).

Bevorzugt haben L₁ und L₂ die mesomere Grenzstruktur der Formel a.

Im Rahmen der Erfindung ist ein vierzähniger Ligand (auch Tetradentat genannt) ein Ligand, der mit vier Atomen an das Metallatom (Cer-Atom) bindet.

Im Rahmen der Erfindung ist eine homoleptische Cer(IV)-Verbindung ein Komplex, bei dem alle Liganden identisch sind (L₁ = L₂).

Im Rahmen der Erfindung ist eine heteroleptische Cer(IV)-Verbindung ein Komplex, wobei ein Ligand eine andere Bedeutung hat als der andere Ligand (L₁ ≠ L₂).

Im Rahmen der Erfindung gibt das Präfix Cₙ-Cₘ die Anzahl der Kohlenstoffatome an, die ein damit bezeichnetes Molekül oder ein damit bezeichneter Rest aufweisen kann.

Im Rahmen der vorliegenden Erfindung steht der Ausdruck "C₁-C₆-Alkyl" für unverzweigte und verzweigte gesättigte Kohlenwasserstoffreste mit 1 bis 6 Kohlenstoffatomen. C₁-C₆-Alkyl sind z.B. Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl. C₁-C₄-Alkyl bedeutet beispielsweise Methyl, Ethyl, Propyl, 1-Methylethyl, Butyl, 1-Methylpropyl, 2-Methylpropyl oder 1,1-Dimethylethyl.

Im Rahmen der vorliegenden Erfindung steht der Ausdruck "C₁-C₆-Alkoxy" für eine unverzweigte oder verzweigte gesättigte C₁-C₆-Alkylgruppe, wie vorstehend definiert, die über ein Sauerstoffatom gebunden ist. Bevorzugt sind Alkoxyreste mit 1 bis 4 Kohlenstoffatomen, besonders bevorzugt mit 1 oder 2 Kohlenstoffatomen. C₁-C₂-Alkoxy ist Methoxy oder Ethoxy. C₁-C₄-Alkoxy ist z. B. Methoxy, Ethoxy, n-Propoxy, 1-Methylethoxy (Isopropoxy), Butoxy, 1-Methylpropoxy (sec-Butoxy), 2-Methylpropoxy (Isobutoxy) oder 1,1-Dimethylethoxy (tert-Butoxy). C₁-C₆-Alkoxy beinhaltet die für C₁-C₄-Alkoxy angegebenen Bedeutungen und zusätzlich z. B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexyloxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy und 3,3-Dimethylbutoxy.

Im Rahmen der vorliegenden Erfindung steht der Ausdruck "C₁-C₆-Alkylsulfanyl" für eine unverzweigte oder verzweigte gesättigte C₁-C₆-Alkylgruppe, wie vorstehend definiert, die über ein Schwefelatom gebunden ist. Bevorzugt sind Alkylsulfanylreste mit 1 bis 4 Kohlenstoffatomen, besonders bevorzugt mit 1 oder 2 Kohlenstoffatomen. C₁-C₂-Alkylsulfanyl ist Methylsulfanyl oder Ethylsulfanyl. C₁-C₄-Alkylsulfanyl ist beispielsweise Methylsulfanyl, Ethylsulfanyl, n-Propylsulfanyl, 1-Methylethylsulfanyl (Isopropylsulfanyl), Butylsulfanyl, 1-Methylpropylsulfanyl (sec-Butylsulfanyl), 2-Methylpropylsulfanyl (Isobutylsulfanyl) oder 1,1-Dimethylethylsulfanyl (tert-Butylsulfanyl). C₁-C₆-Alkylthio beinhaltet die für C₁-C₄-Alkylsulfanyl angegebenen Bedeutungen und zusätzlich beispielsweise auch Pentylsulfanyl, 1-Methylbutylsulfanyl, 2-Methylbutylsulfanyl, 3-Methylbutylsulfanyl, 1,1-Dimethylpropylsulfanyl, 1,2-Dimethylpropylsulfanyl, 2,2-Dimethylpropylsulfanyl, 1-Ethylpropylsulfanyl, Hexylsulfanyl, 1-Methylpentylsulfanyl, 2-Methylpentylsulfanyl, 3-Methylpentylsulfanyl, 4-Methylpentylsulfanyl, 1,1-Dimethylbutylsulfanyl, 1,2-Dimethylbutylsulfanyl, 1,3-Dimethylbutylsulfanyl, 2,2-Dimethylbutylsulfanyl, 2,3-Dimethylbutylsulfanyl, 3,3-Dimethylbutylsulfanyl, 1-Ethylbutylsulfanyl, 2-Ethylbutylsulfanyl, 1,1,2-Trimethylpropylsulfanyl, 1,2,2-Trimethylpropylsulfanyl, 1-Ethyl-1-methylpropylsulfanyl oder 1-Ethyl-2-methylpropylsulfanyl.

Im Rahmen der Erfindung stehen die Ausdrücke Haloalkyl, Haloalkoxy und Hakoalkylsulfanyl für teilweise oder vollständig halogeniertes Alkyl, Alkoxy oder Alkylsulfanyl. Mit anderen Worten, ist ein Wasserstoffatom oder sind mehrere Wasserstoffatome, z. B. 1, 2, 3, 4 oder 5, Wasserstoffatome, die an ein Kohlenstoffatom oder mehrere Kohlenstoffatome von Alkyl, Alkoxy oder Alkylsulfanyl gebunden sind, durch ein Halogenatom, insbesondere durch Fluor oder Chlor, ersetzt.

Im Rahmen der Erfindung steht der Ausdruck "Cycloalkyl" steht für carbocyclische, monocyclische Reste mit 3 bis 7 Kohlenstoffen, wie Cyclopropyl, Cyclobutyl, Cyclopentyl, Cyclohexyl, Cycloheptyl; bevorzugt sind Cyclopentyl, Cyclohexyl und Cycloheptyl, wobei die Anbindung an den Rest des Moleküls über jegliches geeignetes C-Atom erfolgen kann. Im Falle einer Substitution können diese im Allgemeinen 1, 2, 3, 4, 4, 5 oder 6, bevorzugt 1, 2 oder 3 und besonders bevorzugt 1 "Substituenten" gemäß obiger Definition tragen.

Im Rahmen der Erfindung steht der Ausdruck "Alkylen" für eine Alkandiyl- Gruppen, d.h. Kohlenwasserstoff-Brückengruppen mit 2 oder 3 Kohlenstoffatomen, wie z.B. -(CH₂)₂-, - (CH₂)₃-, -CH₂-CH-CH₂-. Im Falle einer Substitution können diese im Allgemeinen 1, 2 oder, 3 Substituenten R¹² gemäß obiger Definition tragen.

Im Rahmen der Erfindung steht der Ausdruck "Alkenylen" steht für die ein- oder mehrfach, insbesondere einfach ungesättigten Analoga de Alkylengruppen mit 2 oder 3 Kohlenstoffatomen, wie -CH=CH-, -CH=CH-CH₂-, -CH₂-CH=CH-. Im Falle einer Substitution können diese im Allgemeinen 1, 2 oder, 3 Substituenten R¹² gemäß obiger Definition tragen.

Im Rahmen der Erfindung entpricht der Ausdruck "Teil eines 1-kernigen, 2- kernigen oder 3-kernigen aromatischen oder heteroaromatischen C₆-C₁₄-Ringsystems" den entsprechen zweifach oder mehrfach verknüpften Analoga der Aryle oder Hetaryle.

Im Rahmen der Erfindung entpricht der Ausdruck "Brückengruppe, die 2 oder 3 Kohlenstoffatome zwischen den Stickstoffatomen aufweist", dass 2 oder 3 Kohlenstoffatome in direkter Linie zwischen den flankierenden Bindungen (Stickstoffatomen) liegen.

Unter Halogen wird im Rahmen der Erfindung Fluor, Chlor, Brom und Jod verstanden.

Der Ausdruck "Aryl" umfasst im Rahmen der vorliegenden Erfindung ein- oder mehrkernige aromatische Kohlenwasserstoffreste mit üblicherweise 6 bis 14, besonders bevorzugt 6 bis 10 Kohlenstoffatomen. Beispiele für Aryl sind insbesondere Phenyl, Naphthyl, Indenyl, Fluorenyl, Anthracenyl, Phenanthrenyl, Naphthacenyl, Chrysenyl, Pyrenyl, etc. und speziell Phenyl oder Naphthyl.

Der Ausdruck "Hetaryl" umfasst im Rahmen der vorliegenden Erfindung ein- oder mehrkernige aromatische Kohlenwasserstoffreste mit 4 bis 13 Kohlenstoffatomen, wobei 1, 2 oder 3 Kohlenstoffatome durch 1, 2 oder 3 identische oder verschiedene Heteroatome oder heteroatomhaltige Gruppen als Ringglieder ersetzt wurden, wobei die Heteroatome und heteroatomhaltigen Gruppen ausgewählt sind unter N, NR⁸, O, S, SO und SO₂. Die Hetaryl-Gruppe kann über einen Ringkohlenstoff oder über einen Ringstickstoff an den Rest des Moleküls gebunden sein. Beispiele für 5- oder 6-gliedrige aromatische heterocyclische Ringe (auch heteroaromatische Ringe oder Hetaryl genannt) sind: 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1 ,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl. Beispiele für 8-, 9- oder 10-gliedrige aromatische heterobicyclische Ringe sind Hetaryl, das einen der vorgenannten 5- oder 6-gliedrigen heteroaromatischen Ringe und einen weiteren aromatischen Carbocyclus oder 5- oder 6-gliedrigen Heterocyclus, der daran kondensiert ist, aufweist, beispielsweise ein kondensierter Benzol-, Thiophen-, Furan-, Pyrrol, Pyrazol-, Imidazol-, Pyridin- oder Pyrimidinring. Zu diesen bicyclischen Hetarylen gehören beispielsweise Chinolinyl, Isochinolinyl, Cinnolinyl, Indolyl, Indolizynyl, Isoindolyl, Indazolyl, Benzofuryl, insbesondere 2-Benzofuryl, Benzothienyl, insbesondere 2-Benzothienyl, Benzo[b]thiazolyl, insbesondere 2-Benzo[b]thiazolyl, Benzoxazolyl, insbesondere 2-Benzoxazolyl, Benzthiazolyl, insbesondere 2-Benzthiazolyl, Benzimidazolyl, insbesondere 2-Benzimidazol, Imidazo[1,2-a]pyridin-2-yl, Thieno[3,2-b]pyridin-5yl, Imidazo-[2,1-b]-thiazol-6-yl und 1,2,4-Triazolo[1,5-a]pyridin-2-yl.

Im Rahmen der Erfindung wird unter dem Ausdruck "CN" eine Cyanogruppe (-C=N) verstanden.

Wenn # in einer Formel erscheint, die eine bevorzugte Substruktur einer Verbindung der vorliegenden Erfindung zeigt, bezeichnet es die Bindung zum restlichen Molekül.

### Cer-Verbindungen der Formel (I)

Geeignete Cer(IV)-Verbindungen im Sinne der Erfindung sind die Verbindungen der allgemeinen Formel (I)

Ce⁴⁺[L₁L₂]⁴⁻ (I),

wobei L₁ und L₂, die zuvor und im Folgenden definieren Liganden umfassen, wobei
- L₁ und L₂ die selbe Bedeutung aufweisen,
- L₁ und L₂ jeweils unterschiedliche Bedeutung aufweisen und
- Mischungen aus zwei oder mehr Verbindungen der Formel (I).

Bevorzugt sind Verbindungen der Formel (I), wobei L₁ und L₂ die selbe Bedeutung aufweisen.

Die homoleptischen Verbindungen der Formel (I) werden durch Reaktion des Liganden der Formel (I.1) oder der Formel (II) mit einem Cersalz hergestellt. Normalerweise ist das Cersalz im Reaktionsmedium löslich. Geeignete Salze sind Cer-Ammoniumnitrat und Cer-Ammoniumsulfat. Die Liganden sind entweder kommerziell erhältlich oder sie können durch eine dem Fachmann bekannte Synthese hergestellt werden.

Die heteroleptischen Verbindungen der Formel (I) werden hergestellt durch
- Mischen von zwei verschiedenen homoleptischen Cerverbindungen in einem geeigneten Lösungsmittel,
- Mischen einer homoleptischen Cerverbindung mit einem Liganden oder seinem Alkali-/Erdalkalisalz, der sich von den Liganden der eingesetzten Cerverbindung unterscheidet,
- Gasphasenabscheidung von zwei verschiedenen homoleptischen Cer-Verbindungen,
- Dampfabscheidung (Dampf-Cokondensation) von homoleptischen Cer-Verbindungen mit einem Liganden, der sich von den Liganden der eingesetzten Cerverbindung unterscheidet.

In der Cer-Verbindung der Formel (I) sind L₁ und L₂ tetradentate Liganden, die unabhängig voneinander ausgewählt sind unter Liganden der Formel (I.1) oder der Formel (II) 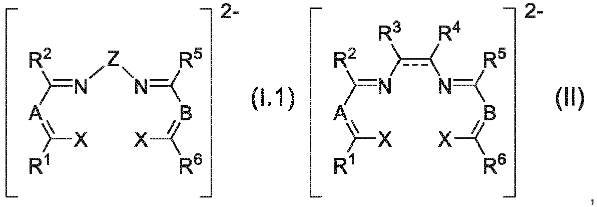 wobei
die Reste X, Z, A, B, R¹, R², R³, R⁴, R⁵ und R⁶ die zuvor und wie im Folgenden definierten Bedeutungen haben.

In einer bevorzugeten Ausführungsform steht Z für C₂-C₃ Alkylen, C₂-C₃ Alkenylen, wobei Alkylen oder Alkenylen unsubstituert oder mit 1, 2, 3, 4, 5, oder 6 identischen oder verschiedenen Resten R¹² substituiert sind; oder
2 oder 3 der Kohlenstoffatome der Brückengruppe Teil eines 1-kernigen oder 2- kernigen aromatischen oder heteroaromatischen C₆-C₁₀-Ringsystems sind, wobei das heteroaromatische Ringsystem 4 bis 13 Kohlenstoffatomen aufweist und 1, 2 oder 3 identische oder verschiedene Heteroatome oder heteroatomhaltige Gruppen als Ringglieder hat, die ausgewählt sind unter N, NR⁸, O, S, SO und SO₂ und wobei das aromatische oder heteroaromatische Ringsystem unsubstituiert oder mit 1, 2, 3, 4 oder 5 identischen oder verschiedenen Resten R⁹ substituiert ist, wobei R¹² und R⁹ die obigen und folgenden Definitionen aufweisen.

Bevorzugt sind Cer-Verbindung der Formel (I), wobei die Liganden L₁ und L₂ unabhängig voneinander ausgewählt sind Liganden der Formel (II).

Bevorzugt sind Liganden der Formel (!.1) oder (II), wobei R¹, R², R⁵ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 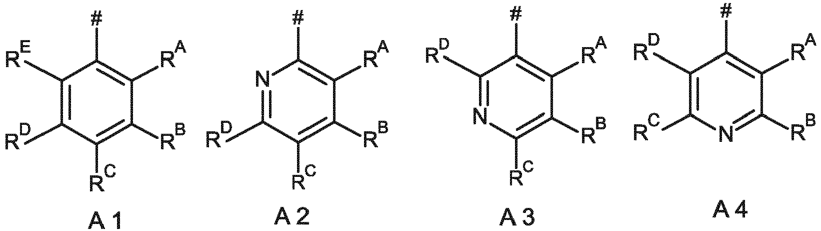 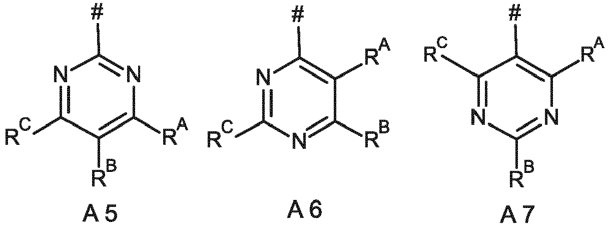 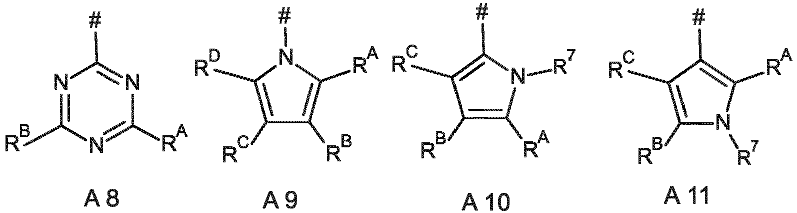 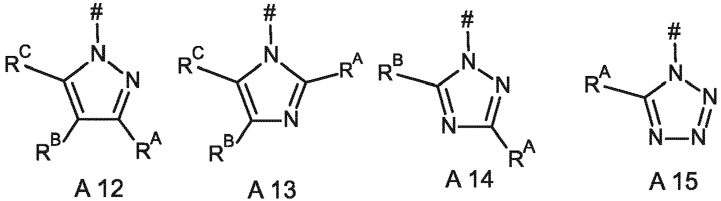 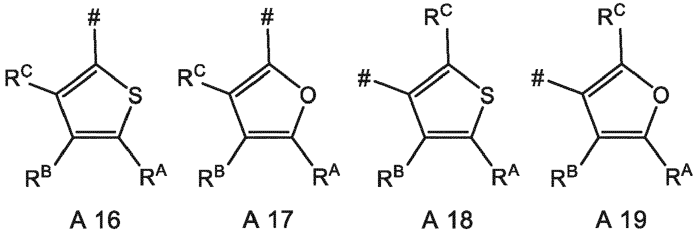
wobei # die Bindung an die Gruppe der Formel (!.1) oder (II) bezeichnet, wobei R^{A}, R^{B}, R^{C}, R^{D} und R^{E} unabhängig voneinander ausgewählt sind unter Wasserstoff, CN, Halogen, C₁-C₄-Alkyl und C₁-C₄-Haloalkyl; oder
R¹ und A gemeinsam mit dem Kohlenstoffatom, an das sie gebunden sind, eine C₆-C₁₄-Arylgruppe bilden, wobei Aryl unsubstituiert oder mit 1, 2, 3 oder 4 identischen oder verschiedenen Resten R⁹ substituiert ist; oder
R⁶ und B gemeinsam mit dem Kohlenstoffatom, an das sie gebunden sind, eine C₆-C₁₄-Arylgruppe bilden, wobei Aryl unsubstituiert oder mit 1, 2, 3 oder 4 identischen oder verschiedenen Resten R⁹ substituiert ist;
R^{4a}, R^{4b} unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
R⁹ ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten, ausgewählt unter C₁-C₄-Alkyl und C₁-C₄-Haloalkyl substituiert ist; und
R¹⁰ ausgewählt ist unter CN, Halogen, C₁-C₄-Alkyl und C₁-C₄-Haloalkyl.

Unabhängig von ihrem Vorkommen sind die Reste R^{A}, R^{B}, R^{C}, R^{D} und R^{E} vorzugsweise ausgewählt unter Wasserstoff, CN, Fluor, Chlor, C₁-C₄-Alkyl, C₁-C₄-Fluoralkyl und C₁-C₄-Chloralkyl.

Besonders bevorzugt sind die Reste R^{A}, R^{B}, R^{C}, R^{D} und R^{E} unabhängig voneinander ausgewählt unter Wasserstoff, CN, C₁-C₄-Fluoralkyl, Fluor und Chlor. Im speziellen stehen die Reste R^{A}, R^{B}, R^{C}, R^{D} und R^{E} unabhängig voneinander für Wasserstoff oder CF₃.

In einer Ausführungsform sind in der Formel (!.1) oder (II), die Reste R¹, R², R⁵ und R⁶ unabhängig voneinander ausgewählt unter Wasserstoff, C₁-C₄-Alkyl, C₁-C₄-Haloalkyl und A1 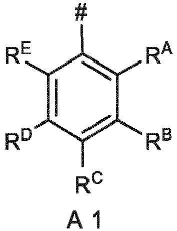
wobei # die Bindung zum restlichen Molekül der Formel (II) bezeichnet, und worin R^{A}, R^{B}, R^{C}, R^{D} und R^{E} unabhängig voneinander ausgewählt sind unter Wasserstoff, CN, Fluor, Chlor, C₁-C₄-Alkyl, C₁-C₄-Fluoralkyl und C₁-C₄-Chloralkyl, bevorzugt unter Wasserstoff, CN, Fluor und Chlor; oder
R¹ und A gemeinsam mit dem Kohlenstoffatom, an das sie gebunden sind, einen Phenylring bilden, wobei Phenyl unsubstituiert oder mit 1, 2, 3 oder 4 identischen oder verschiedenen Resten R⁹ substituiert ist; oder
R⁶ und B gemeinsam mit dem Kohlenstoffatom, an das sie gebunden, sind einen Phenylring bilden, wobei Phenyl unsubstituiert oder mit 1, 2, 3 oder 4 identischen oder verschiedenen Resten R⁹ substituiert ist;
R⁹ ausgewählt ist unter für CN, Halogen, C₁-C₄-Alkyl, C₁-C₄-Haloalkyl und Phenyl, wobei Phenyl unsubstituiert oder mit 1, 2, 3, 4 oder 5 identischen oder verschiedenen Resten, ausgewählt unter C₁-C₄-Alkyl oder C₁-C₄-Haloalkyl, substituiert ist.

In einer weiteren Ausführungsform sind in der Formel (I.1) oder (II) R¹ und R⁶ unabhängig voneinander ausgewählt unter CF₃ und A1, worin R^{A}, R^{B}, R^{C}, R^{D} und R^{E} unabhängig voneinander ausgewählt sind unter CN, CF₃, Fluor und Chlor;
R² und R⁵ unabhängig voneinander ausgewählt unter Wasserstoff, CF₃ und A1, worin R^{A}, R^{B}, R^{C}, R^{D} und R^{E} unabhängig voneinander ausgewählt sind unter Wasserstoff, CN, CF₃, Fluor und Chlor; oder
R¹ und A bilden gemeinsam mit dem Kohlenstoffatom, an das sie gebunden sind, einen Phenylring, wobei Phenyl unsubstituiert oder mit 1 oder 2 identischen oder verschiedenen Resten R⁹ substituiert ist; oder
R⁶ und B bilden gemeinsam mit dem Kohlenstoffatom, an das sie gebunden sind, einen Phenylring, wobei Phenyl unsubstituiert oder mit 1 oder 2 identischen oder verschiedenen Resten R⁹ substituiert ist;
R⁹ ausgewählt ist unter für CN, Halogen, C₁-C₂-Alkyl, C₁-C₂-Haloalkyl.

In einer speziellen Ausführungsform sind in der Formel (I.1) oder (II) R¹ und R⁶ unabhängig voneinander ausgewählt unter Wasserstoff, CF₃ und A1, worin R^{A}, R^{B}, R^{C}, R^{D} und R^{E} unabhängig voneinander ausgewählt sind unter Wasserstoff und CF₃;
R² und R⁵ sind unabhängig voneinander ausgewählt unter Wasserstoff, CF₃ und A1, worin R^{A}, R^{B}, R^{C}, R^{D} und R^{E} unabhängig voneinander ausgewählt sind unter Wasserstoff und CF₃;
R¹ und A bilden gemeinsam mit dem Kohlenstoffatom, an das sie gebunden sind, einen Phenylring, wobei Phenyl unsubstituiert oder mit 1 oder 2 identischen oder verschiedenen Resten R⁹ substituiert ist;
R⁶ und B bilden gemeinsam mit dem Kohlenstoffatom, an das sie gebunden, sind einen Phenylring, wobei Phenyl unsubstituiert oder mit 1 oder 2 identischen oder verschiedenen Resten R⁹ substituiert ist;
R⁹ ausgewählt ist unter für CN, F, CI und CF₃.

In einer anderen speziellen Ausführungsform sind in der Formel (I.1) oder (II) R² und R⁵ unabhängig voneinander ausgewählt unter Wasserstoff und CF₃ und R¹ und R⁶ sind unabhängig voneinander ausgewählt unter CF₃ und A1, worin R^{A}, R^{B}, R^{C}, R^{D} und R^{E} für Wasserstoff oder CF₃ steht, oder
R¹ und A bilden gemeinsam mit dem Kohlenstoffatom, an das sie gebunden sind, einen Phenylring, wobei Phenyl unsubstituiert oder mit 1 oder 2 identischen oder verschiedenen Resten R⁹ substituiert ist; oder
R⁶ und B bilden gemeinsam mit dem Kohlenstoffatom, an das sie gebunden sind, einen Phenylring, wobei Phenyl unsubstituiert oder mit 1 oder 2 identischen oder verschiedenen Resten R⁹ substituiert ist;
R⁹ ausgewählt ist unter für CN, F, CI und CF₃.

Bevorzugt sind Liganden der Formel (II), wobei in der Formel (II) R³ und R⁴ unabhängig voneinander ausgewählt sind unter Wasserstoff, CN, C₁-C₆-Alkyl, C₁-C₆-Haloalkyl, C₁-C₆-Alkoxy und C₁-C₆-Haloalkoxy, oder
R³ und R⁴ gemeinsam mit den Kohlenstoffatomen, an die sie gebunden sind, eine C₆-C₁₄-Arylgruppe bilden, wobei Aryl unsubstituiert oder mit 1, 2, 3 oder 4 identischen oder verschiedenen Resten R⁹ substituiert ist;
R⁹ ausgewählt ist unter CN, Halogen, C₁-C₆-Alkyl, C₁-C₆-Haloalkyl oder C₆-C₁₄-Aryl, wobei Aryl unsubstituiert oder mit 1, 2, 3, 4 oder 5 identischen oder verschiedenen Resten, ausgewählt unter C₁-C₄-Alkyl oder C₁-C₄-Haloalkyl, substituiert ist.

Besonders bevorzugt sind Liganden der Formel (II), wobei in der Formel (II) R³ und R⁴ unhabhängig voneinander ausgewählt sind unter Wasserstoff, CN, C₁-C₄-Alkyl, C₁-C₄-Haloalkyl und Phenyl, wobei Phenyl unsubstituiert oder mit 1, 2, 3 oder 4 identischen oder verschiedenen Resten R⁹ substituiert ist, oder
R³ und R⁴ bilden gemeinsam mit den Kohlenstoffatomen, an die sie gebunden sind, einen Phenylring, wobei Phenyl unsubstituiert oder mit 1, 2, 3, oder 4 identischen oder verschiedenen Resten R⁹ substituiert ist;
R⁹ ausgewählt ist unter CN, Halogen, C₁-C₄-Alkyl, C₁-C₄-Haloalkyl oder Phenyl, wobei Phenyl unsubstituiert oder mit 1, 2, 3 oder 4 identischen oder verschiedenen Resten, ausgewählt unter C₁-C₄-Alkyl oder C₁-C₄-Haloalkyl, substituiert ist.

Besonders bevorzugt sind Liganden der Formel (II), wobei in der Formel (II) R³ und R⁴ unabhängig voneinander ausgewählt sind unter Wasserstoff, CN, C₁-C₂-Alkyl und CF₃;
R³ und R⁴ gemeinsam mit den Kohlenstoffatomen, an die sie gebunden sind, eine Phenylgruppe bilden, wobei Phenyl unsubstituiert ist.

Bevorzugt sind Liganden der Formel (I.1) oder (II), wobei in der Formel (II) A und B unhabhängig voneinander für einen Rest CR⁷ stehen und R⁷ ausgewählt ist unter Wasserstoff, CN, Nitro, Halogen und der Gruppe B, bestehend aus B1, B2, B3, B4, B5, B6, B7, B8, B9, B10, B11, B12, B13, B14 und B15 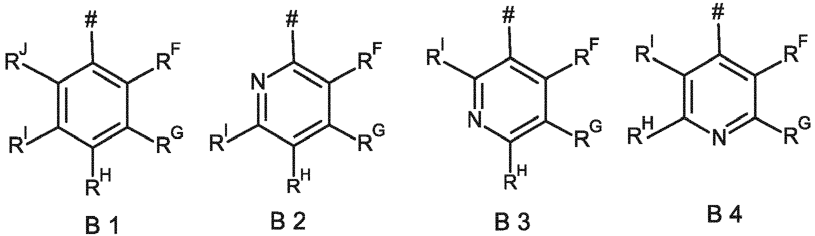 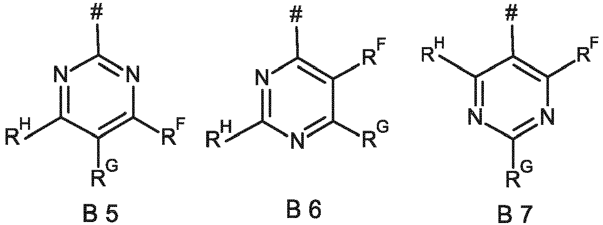 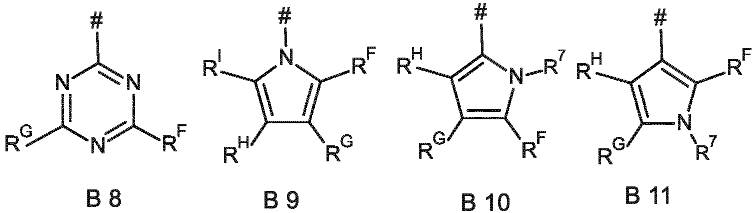 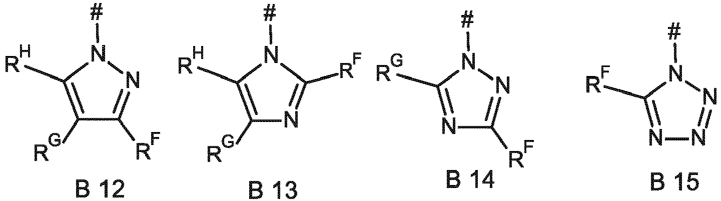
wobei # die Bindung zur Gruppe der Formel (II) bezeichnet, und wobei
R^{F}, R^{G}, R^{H}, R^{I} und R^{J} unabhängig voneinander ausgewählt sind unter Wasserstoff, CN, Halogen, C₁-C₄-Alkyl und C₁-C₄-Haloalkyl.

Unabhängig von ihrem Vorkommen sind die Reste R^{F}, R^{G}, R^{H}, R^{I} und R^{J} vorzugsweise ausgewählt unter Wasserstoff, CN, Fluor, Chlor, C₁-C₄-Alkyl, C₁-C₄-Fluoralkyl und C₁-C₄-Chloralkyl. In einer bevorzugten Ausführungsform stehen die Reste R^{F}, R^{G}, R^{H}, und R^{J} unabhängig voneinander für Wasserstoff, CN, Fluor oder Chlor.

Bevorzugt sind Liganden der Formel (I.1) oder (II), wobei in der Formel (II) A und B unhabhängig voneinander für einen Rest CR⁷ stehen und R⁷ ausgewählt ist unter Wasserstoff, CN, Nitro, Halogen, C₁-C₂-Alkyl, C₁-C₂-Haloalkyl und B1, worin R^{F}, R^{G}, R^{H}, R^{I} und R^{J} unabhängig voneinander ausgewählt sind unter Wasserstoff, CN, Fluor und Chlor.

Insbesondere bevorzugt sind Liganden der Formel (!.1) oder (II), wobei in der Formel (II) A und B unhabhängig voneinander für einen Rest CR⁷ stehen und R⁷ ausgewählt ist unter Wasserstoff, CN, Nitro, Halogen, C₁-C₂-Alkyl und C₁-C₂-Haloalkyl. Im speziellen ist R⁷ ausgewählt unter Wasserstoff, CN, Nitro, Fluor und Chlor, insbesondere Wasserstoff, Fluor und CN.

In einer Ausführungsform A sind Liganden der Formel (II) ausgewählt unter der Formel (I.A) 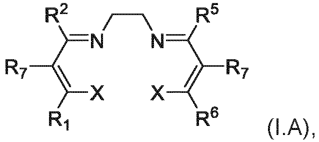 wobei
- X: für O steht,
- R¹: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert,
- R²: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert,
- R⁵: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert,
- R⁶: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert,
- R⁷: ist ausgewählt unter Wasserstoff, CN, Nitro, Halogen, C₁-C₂-Alkyl und C₁-C₂-Haloalkyl.

In einer Ausführungsform B sind Liganden der Formel (II) ausgewählt unter der Formel (I.B) 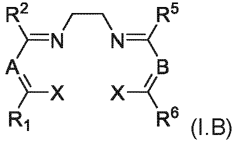 wobei
- X: für O steht;
- R¹ und A: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R²: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R⁵: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R⁶ und B: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind.

In einer Ausführungsform C sind Liganden der Formel (II) ausgewählt unter der Formel (I.C) 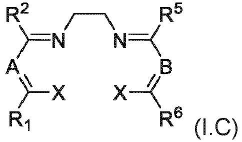 wobei
- X: für O steht;
- R¹: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R² und A: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R⁵ und B: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R⁶: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert.

In einer Ausführungsform D sind Liganden der Formel (II) ausgewählt unter der Formel (I.D) 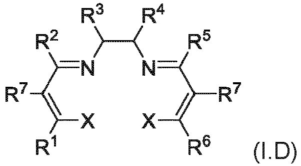
- R¹: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R²: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R³ und R⁴: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R⁵: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R⁶: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R⁷: ist ausgwählt unter Wasserstoff, CN, Nitro, Halogen, C₁-C₂-Alkyl und C₁-C₂-Haloalkyl.

In einer Ausführungsform E sind Liganden der Formel (II) ausgewählt unter der Formel (I.E) 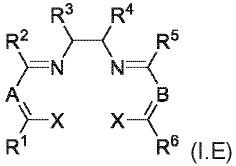 wobei
- R¹ und A: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R²: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R³ und R⁴: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R⁵: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R⁶ und B: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind.

In einer Ausführungsform F sind Liganden der Formel (II) ausgewählt unter der Formel (I.F) 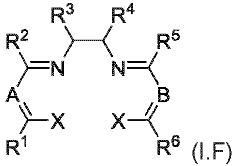 wobei
- R¹: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert;
- R² und A: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R³ und R⁴: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind;
- R⁵ und B: gemeinsam mit dem Kohlenstoffatom, an das sie gebunden sind, eine C₆-C₁₄-Arylgruppe oder Hetarylgruppe mit 3 bis 6 Kohlenstoffatomen bilden, wobei Hetaryl 1, 2 oder 3 Heteroatome oder heteroatomhaltige Gruppen als Ringglieder hat, die ausgewählt sind unter N und NR⁸, wobei Aryl und Hetaryl unsubstituiert oder mit 1, 2, 3 oder 4 identischen oder verschiedenen Resten R⁹ substituiert sind
- R⁶: ist ausgewählt unter Wasserstoff, CN, C₁-C₆-Alkyl, C₁-C₆-Haloalkyl, C₃-C₇-Cycloalkyl, wobei C₃-C₇-Cycloalkyl unsubstituiert oder mit 1, 2, 3, 4, 5 oder 6 identischen oder verschiedenen Resten ausgewählt unter CN, Halogen, C₁-C₄-Alkyl und C₁-C₄-Haloalkyl substituiert ist, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 und A19 wie oben definiert.

In einer bevorzugten Ausführungsfom handelt es sich um Verbindungen der Formel (I), wobei in den Liganden der Formel (II), 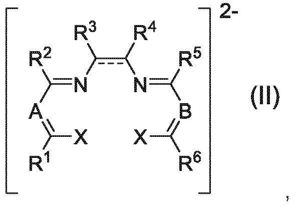 wobei
-̅-̅-̅-̅-̅-̅ zwischen der Gruppe CR³-CR⁴ für eine Einfach- oder Doppelbindung steht,
X für O steht;
A und B unabhängig voneinander für CR⁷ steht, wobei R⁷ ausgewählt ist unter H und CN; R¹ und R⁶ unabhängig voneinander ausgewählt sind unter Wasserstoff, CH₃, CF₃, C₂F₅ und phenyl, oder
R¹ und A gemeinsam mit dem Kohlenstoffatom, an das sie gebunden sind, [4-(trifluoromethyl)phenyl] bilden, oder
R⁶ und B gemeinsam mit dem Kohlenstoffatom, an das sie gebunden sind, [4-(trifluoromethyl)phenyl];
R³ und R⁴ für Wasserstoff steht, oder
R³ und R⁴ gemeinsam mit den Kohlenstoffen, an denen sie gebunden sind, phenyl oder (3,4-difluorophenyl) bilden;
R² und R⁵ unabhängig voneinander ausgewählt sind unter Wasserstoff, CH₃, CF₃, C₂F₅, phenyl und [3-chloro-4-(trifluoromethyl)phenyl].

Insbesonder sind die Verbindungen ausgewählt unter

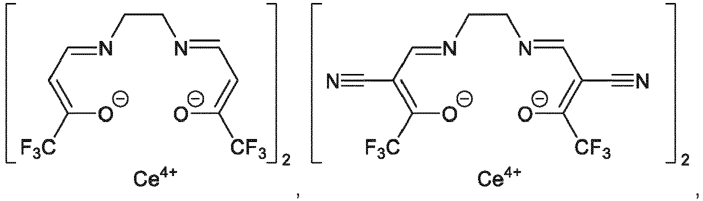

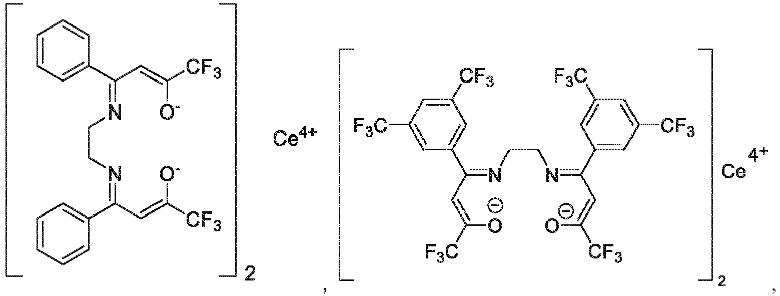

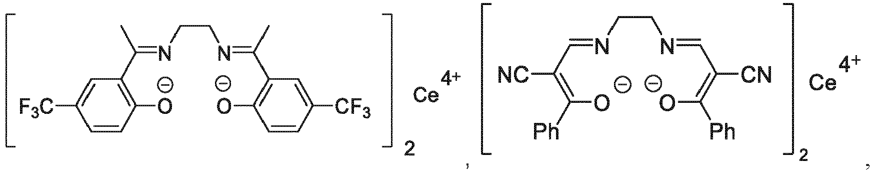

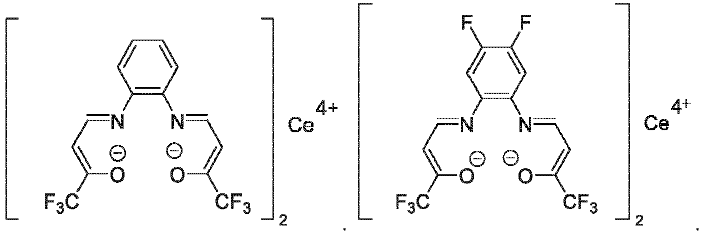

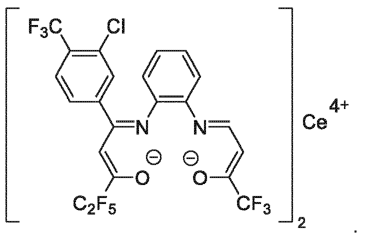

Unabhängig von ihrem Vorkommen sind die Reste R^{4a} und R^{4b} bevorzugt ausgewählt unter Wasserstoff und C₁-C₄-Alkyl.

Unabhängig von seinem Vorkommen ist der Rest R⁸ bevorzugt ausgewählt unter C₁-C₄-Alkyl und C₁-C₄-Haloalkyl.

Unabhängig von seinem Vorkommen ist der Rest R⁹ bevorzugt ausgewählt unter CN, Halogen, C₁-C₄-Alkyl, C₁-C₄-Haloalkyl und Phenyl, wobei Phenyl unsubstituiert oder mit 1, 2, 3, 4 oder 5 identischen oder verschiedenen Resten, ausgewählt unter C₁-C₄-Alkyl oder C₁-C₄-Haloalkyl, substituiert ist. Insbesondere ist R⁹ ausgewählt unter CN, Halogen, C₁-C₂-Alkyl und C₁-C₂-Haloalkyl, Im speziellen ist R⁹ ausgewählt unter CN, F, CI und CF₃.

Unabhängig von seinem Vorkommen ist der Rest R¹² bevorzugt ausgewählt unter Halogen, CN, C₁-C₄-Alkyl, C₁-C₄-Haloalkyl und Phenyl, wobei Phenyl unsubstituiert oder mit 1, 2, 3 oder 4 identischen oder verschiedenen Resten R⁹ substituiert ist

### Bauteil

Unter einem elektronischen Bauteil versteht man im Rahmen der Erfindung ein diskretes oder integriertes elektrisches Bauelement, das die Eigenschaften der Verbindungen der allgemeinen Formel (I) oder von Halbleitermatrixmaterialien, die eine Verbindungen der allgemeinen Formel (I) enthalten, nutzt. In einer speziellen Ausführung weist das elektronische Bauteil eine Schichtstruktur auf, die insbesondere 2, 3, 4, 5, 6, 7 oder mehr Schichten umfasst, wobei wenigstens eine der Schichten wenigstens eine Verbindung der allgemeinen Formel (I) enthält. Jede der Schichten kann aber auch anorganische Materialien enthalten, oder das Bauteil kann auch Schichten umfassen, die vollständig aus anorganischen Materialien aufgebaut sind.

Bevorzugt ist das elektronische Bauteil ausgewählt unter organischen Feldeffekttransistoren (OFETs), organischen Elektrolumineszenzvorrichtungen, organischen Solarzellen (OSCs), Vorrichtungen für die Elektrofotografie, organischen optischen Detektoren, organischen Fotorezeptoren, lichtemittierenden elektrochemischen Zellen (LECs) und organischen Laserdioden. Bevorzugte organische Feldeffekttransistoren (OFETs) sind organische Dünnschichttransistoren (OTFTs). Bevorzugte organische Elektrolumineszenzvorrichtungen sind organische lichtemittierende Dioden (OLEDs). Bevorzugte organische Solarzellen sind Exzitonsolarzellen, farbstoffsensibilisierte Solarzellen (DSSCs) oder Perowskitsolarzellen. Bevorzugte Vorrichtungen für die Elektrofotografie sind fotoleitfähige Materialien in organischen Fotoleitern (OPC).

Bevorzugt liegt das erfindungsgemäße elektronische Bauteil in Form einer organischen lichtemittierenden Diode, einer organischen Solarzelle, einer Photovoltaikzelle, eines organischen Sensors, einer organischen Diode oder eines organischen Transistors, bevorzugt eines Feldeffekttransistors oder Dünnschichtfilmtransistors oder Perowskitsolarzelle, vor.

Bei dem elektronischen Bauteil kann es sich speziell um eine organische Elektrolumineszenzvorrichtung, insbesondere in Form einer organischen lichtemittierenden Diode (OLED), handeln. Eine organische Elektrolumineszenzvorrichtung umfasst eine Kathode, eine Anode und mindestens eine emittierende Schicht. Sie kann neben diesen Schichten auch weitere Schichten umfassen, z. B. jeweils eine oder mehrere Lochinjektionsschichten, Lochtransportschichten, Lochblockierschichten, Elektronentransportschichten, Elektroneninjektionsschichten, Excitonblockierschichten, Elektronenblockierschichten und/oder Ladungserzeugungsschichten. Zwischenschichten, die beispielsweise eine Exciton-Blocking-Funktion haben, können ebenfalls zwischen zwei emittierenden Schichten eingebracht werden. Nicht jede dieser Schichten muss unbedingt vorhanden sein.

Eine bevorzugte Ausführungsform ist ein elektronisches Bauteil, insbesondere in Form einer OLED, wobei die Schicht, die die Verbindung der Formel (I) umfasst, eine Lochtransportschicht oder eine Lochinjektionsschicht ist. Eine Lochinjektionsschicht ist im Allgemeinen eine Schicht, die die Elektroneninjektion von der Anode in das organische Halbleitermatrixmaterial erleichtert. Die Lochinjektionsschicht kann direkt neben der Anode angeordnet werden. Eine Lochtransportschicht transportiert die Löcher von der Anode zur emittierenden Schicht und befindet sich zwischen einer Lochinjektionsschicht und einer emittierenden Schicht.

Eine bevorzugte Ausführungsform ist ein elektronisches Bauteil in Form einer organischen Solarzelle. Organische Solarzellen sind im Allgemeinen schichtförmig aufgebaut und umfassen in der Regel zumindest die folgenden Schichten: Anode, wenigstens eine photoaktive Schicht und Kathode. Diese Schichten werden im Allgemeinen auf ein für diesen Zweck übliches Substrat aufgetragen. Der photoaktive Bereich der Solarzelle kann zwei Schichten umfassen, die jeweils eine homogene Zusammensetzung haben und einen "flachen" Donor-Akzeptor-Heteroübergang (flat donor-acceptor heterojunction) ausbilden. Ein photoaktiver Bereich kann auch eine Mischschicht umfassen und einen Donor-Akzeptor Heteroübergang in Form einer donor-acceptor bulk heterojunction ausbilden. Die organische Solarzelle kann neben diesen Schichten auch weitere Schichten umfassen, z. B. ausgewählt unter
- Schichten mit elektronenleitenden Eigenschaften (electron transport layer, ETL),
- Schichten, die ein lochleitendes Material umfassen (hole transport layer, HTL), diese müssen keine Strahlung absorbieren,
- Excitonen und Löcher blockende Schichten (z. B. EBLs), diese dürfen nicht absorbieren, und
- Multiplikatorschichten.

Eine weitere bevorzugte Ausführungsform ist ein elektronisches Bauteil in Form einer organischen Solarzelle, wobei die Schicht, die die Verbindung der Formel (I) umfasst, eine Schicht mit elektronenleitenden Eigenschaften (electron transport layer, ETL) ist.

Eine spezielle Ausführungsform ist ein elektronisches Bauteil, speziell in Form einer organischen Solarzelle, wobei die Schicht, die mindestens eine der Verbindungen der Formel (I) umfasst, Teil eines pn-Übergangs ist, der eine lichtabsorbierende Einheit mit einer zusätzlichen lichtabsorbierenden Einheit in einer Tandemvorrichtung oder in einer mehrfach gestapelten Vorrichtung und/oder einem pn-Übergang verbindet, der eine Kathode oder eine Anode mit einer lichtabsorbierenden Einheit verbindet.

Eine weitere bevorzugte Ausführungsform ist ein elektronisches Bauteil, das eine Elektrontransportschicht umfassend, die wenigstens eine Verbindung der Formel (I) umfasst.

### Halbleitermatrixmaterialien

Die erfindungsgemäßen und erfindungsgemäß eingesetzten Verbindungen der Formel (I) sowie deren Ladungstransferkomplexe, deren Reduktionsprodukte können als Dotierstoffe in organischen Halbleitermatrixmaterialen, insbesondere als p-Dotand in Lochtransportschichten, eingesetzt werden. Das dotierte Halbleitermatrixmaterial umfasst vorzugsweise mindestens einen Elektronendonor und mindestens eine Verbindung der Formel (I), wie zuvor definiert.

Geeignete Diaminoterphenyle sind in der DE 102012007795 beschrieben. Diaminotrimethylphenylindane sind in der WO 2018/206769 beschrieben.

Insbesondere sind die Elektronendonoren ausgewählt unter 4,4',4"-Tris(N-(2-naphthyl)-N-phenyl-amino)triphenylamin, 4,4',4"-Tris(N-3-methylphenyl-N-phenyl-amino)triphenylamin, N,N,N',N'-Tetrakis(4-methoxy-phenyl)benzidin, (2,2',7,7'-Tetrakis-(N,N-diphenylamino)-9,9'-spirobifluoren, N,N'-Bis(naphthalen-1-yl)-N,N'-bis(phenyl)-benzidin, N,N'-Bis(naphthalen-1-yl)-N,N'-bis(phenyl)-9,9-spiro-bifluoren, 9,9-Bis[4-(N,N-bis-biphenyl-4-yl-amino)phenyl]-9H-fluoren, 2,2'-Bis[N,N-bis(biphenyl-4-yl)amino]-9,9-spiro-bifluoren, N,N'-((9H-fluoren-9,9-diyl)bis(4,1-phenylen))bis(N-([1,1'-biphenyl]-4-yl)-[1,1'-biphenyl]-4-amin),N,N'-Bis(phenanthren-9-yl)-N,N'-bis(phenyl)-benzidin, 1,3,5-Tris{4-[bis(9,9-dimethyl-fluoren-2-yl)amino]phenyl}benzol, Tri(terphenyl-4-yl)amin, N-(4-(6-((9,9-Dimethyl-9H-fluoren-2-yl)(6-methoxy-[1,1'-biphenyl]-3-yl)amino)-1,3,3-trimethyl-2,3-dihydro-1H-inden-1-yl)phenyl)-N-(6-methoxy-[1,1'-biphenyl]-3-yl)-9,9-dimethyl-9H-fluoren-2-amin, N-([1,1'-Biphenyl]-4-yl)-N-(4-(6-([1,1'-biphenyl]-4-yl(9,9-dimethyl-9H-fluoren-2-yl)amino)-1,3,3-trimethyl-2,3-dihydro-1H-inden-1-yl)phenyl)-9,9-dimethyl-9H-fluoren-2-amin, N,N-Di([1,1'-biphenyl]-4-yl)-3-(4-(di([1,1'-biphenyl]-4-yl)amino)phenyl)-1,1,3-trimethyl-2,3-dihydro-1H-inden-5-amin, N-(4-(6-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-(4-(6-(9,9'-Spirobi[fluoren]-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9'-spirobi[fluoren]-2-amin, N-(4-(6-(Dibenzo[b,d]furan-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)dibenzo[b,d]furan-2-amin, 9-(4-(6-(9H-Carbazol-9-yl)-1,3,3-trimethyl-2,3-dihydro-1H-inden-1-yl)phenyl)-9H-carbazol, N-([1,1'-Biphenyl]-4-yl)-3-(4-([1,1'-biphenyl]-4-yl(4-methoxyphenyl)amino)phenyl)-N-(4-methoxyphenyl)-1,1,3-trimethyl-2,3-dihydro-1H-inden-5-amin, 3-(4-(Bis(6-methoxy-[1,1'-biphenyl]-3-yl)amino)phenyl)-N,N-bis(6-methoxy-[1,1'-biphenyl]-3-yl)-1,1,3-trimethyl-2,3-dihydro-1H-inden-5-amin, N1-([1,1'-Biphenyl]-4-yl)-N1-(4-(6-([1,1'-biphenyl]-4-yl(4-(diphenylamino)phenyl)amino)-1,3,3-trimethyl-2,3-dihydro-1H-inden-1-yl)phenyl)-N4,N4-diphenylbenzene-1,4-diamin, N,N-Di([1,1'-biphenyl]-4-yl)-4'-(6-(4-(di([1,1'-biphenyl]-4-yl)amino)phenyl)-1,3,3-trimethyl-2,3-dihydro-1H-inden-1-yl)-[1,1'-biphenyl]-4-amin, N-(4-(5-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-(4-(6-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N,N'-bis(9,9-dimethyl-fluoren-2-yl)-N,N'-diphenyl-benzidin (BF-DPB), N,N'-((9H-fluoren-9,9-diyl)bis(4,1-phenylen))bis(N-([1,1'-biphenyl]-4-yl)-[1,1'-biphenyl]-4-amin) (BPAPF), N4,N4,N4',N4'-tetrakis(9,9-dimethyl-9H-fluoren-2-yl)-[1,1'-biphenyl]-4,4'-diamin (TDMFB), N-([1,1'-biphenyl]-2-yl)-N-(9,9-dimethyl-9H-fluoren-2-yl)-9,9'-spirobi[fluoren]-2-amin, (2,7-bis[N,N-bis(4-methoxyphenyl)amino]-9,9-spirobi[9H-fluoren] (spiro-MeO-TPD), einem Gemisch aus N-(4-(5-(bis(9,9-Dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin und N-(4-(6-(bis(9,9-Dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-([1,1'-biphenyl]-4-yl)-9,9-dimethyl-N-(4-(9-phenyl-9H-carbazol-3-yl)phenyl)-9H-fluoren-2-amin und Mischungen davon.

Es versteht sich, dass auch andere geeignete organische Halbleitermatrixmaterialien, insbesondere lochleitende Materialien mit halbleitenden Eigenschaften, verwendet werden können.

### Dotierung

Die Dotierung kann insbesondere so erfolgen, dass das Molverhältnis von Matrixmolekülen zur Verbindung der allgemeinen Formel (I), 10000 : 1 bis 1 : 1, vorzugsweise 1000 : 1 bis 2 : 1, besonders bevorzugt 5 : 1 bis 100 : 1 beträgt.

### Herstellung des dotierten Halbleitermatrixmaterials

Die Dotierung des jeweiligen Matrixmaterials (im Folgenden auch als Lochleitermatrix HT bezeichnet) mit den erfindungsgemäßen und erfindungsgemäß verwendeten Verbindungen der allgemeinen Formel (I) kann durch eines oder eine Kombination aus zwei oder mehr als zwei der folgenden Verfahren erfolgen:
a) Gemischte Verdampfung im Vakuum mit einer Quelle für HT und einer für mindestens eine Verbindung der allgemeinen Formel (I).
b) Sequentielle Abscheidung von HT und mindestens einer Verbindung der allgemeinen Formel (I) mit anschließender Diffusion des Dotierstoffs nach innen durch thermische Behandlung.
c) Dotierung einer HT-Schicht durch eine Lösung von mindestens einer Verbindung der allgemeinen Formel (I) mit anschließender Verdampfung des Lösungsmittels durch thermische Behandlung.
d) Oberflächendotierung einer HT-Schicht durch eine Schicht aus mindestens einer Verbindung der allgemeinen Formel (I), die auf eine oder beide Oberflächen der HT-Schicht aufgebracht ist.
e) Herstellen einer Lösung aus Wirt und mindestens einer Verbindung der allgemeinen Formel (I) und Bilden eines Films aus der Lösung, z. B. durch Beschichtungs-, Gieß- oder Drucktechniken oder andere Filmherstellungstechniken, die einem Fachmann bekannt sind.

Ein weiterer Gegenstand der Erfindung ist die Verwendung einer Verbindung (I), wie zuvor definiert
- als organischer Halbleiter,
- als Dotierstoff in organischen Halbleitermatrixmaterialien, insbesondere als p-Dotand in Lochtransportschichten,
- als Elektronentransportschicht,
- als Ladungsinjektor in einer Ladungsinjektionsschicht,
- als Kathodenmaterial in organischen Batterien,
- als elektrochromes Material.

Ein weiterer Gegenstand der Erfindung ist die Verwendung von durch Reduktion einer Verbindung (I), wie zuvor definiert, erhaltenen Ce(III)-Komplexanionen oder von Ladungstransferkomplexen einer Verbindung (I), wie zuvor definiert, mit Elektronendonoren Leiter, insbesondere als organische Halbleiter als Charge-Transfer-Komplex oder als elektrochromes Material.

Ein weiterer Gegenstand der Erfindung sind Verbindungen der allgemeinen Formel (I)

Ce⁴⁺[L₁L₂]⁴⁻ (I),

deren Ladungstransferkomplexe, deren Reduktionsprodukte, und Mischungen davon, wobei L₁ und L₂ jeweils ein tetradentater Ligand ist, die unabhängig voneinander ausgewählt sind unter Liganden der Formel (I.1) 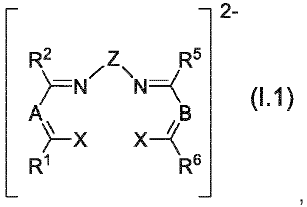 wobei
- X: unabhängig voneinander für O oder S stehen;
- Z: für eine Brückengruppe steht, die 2 oder 3 Kohlenstoffatome zwischen den Stickstoffatomen aufweist, wobei die Kohlenstoffatome jeweils unsubstituiert oder mit 1, 2, 3, 4, 5 oder 6 identischen oder verschiedenen Resten R¹² substituiert sind, wobei zwei benachbarte Kohlenstoffatome auch durch eine Doppelbindung miteinander verbunden sein können, oder 2 oder 3 der Kohlenstoffatome Teil eines 1-kernigen, 2-kernigen oder 3- kernigen aromatischen oder heteroaromatischen C₆-C₁₄-Ringsystems sind, wobei das heteroaromatische Ringsystem 4 bis 13 Kohlenstoffatomen aufweist und 1, 2 oder 3 identische oder verschiedene Heteroatome oder heteroatomhaltige Gruppen als Ringglieder hat, die ausgewählt sind unter N, NR⁸, O, S, SO und SO₂ und wobei das aromatische oder heteroaromatische Ringsystem unsubstituiert oder mit 1, 2, 3, 4 oder 5 identischen oder verschiedenen Resten R⁹ substituiert ist;
- A und B: unabhängig voneinander für N oder CR⁷ stehen;
- R¹ und R⁶: unabhängig voneinander ausgewählt sind unter Wasserstoff, CN, C1-C6-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
- R¹ und A: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind, oder
- R⁶ und B: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R² und R⁵: unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
- R² und A: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
- R⁵ und B: gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
- R^{4a}R^{4b}: unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
- R⁷: ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
- R⁸: ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert sind;
- R⁹: ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
- R¹⁰: ausgewählt ist unter CN, Halogen, C₁-C₄-Alkyl und C₁-C₄-Haloalkyl;
- R¹¹: ausgewählt ist unter CN, Halogen, C₁-C₄-Alkyl und C₁-C₄-Haloalkyl;
- R¹²: unabhängig voneinander ausgewählt sind unter Halogen, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
wobei folgende Verbindungen und deren Reduktionsprodukte ausgenommen sind:

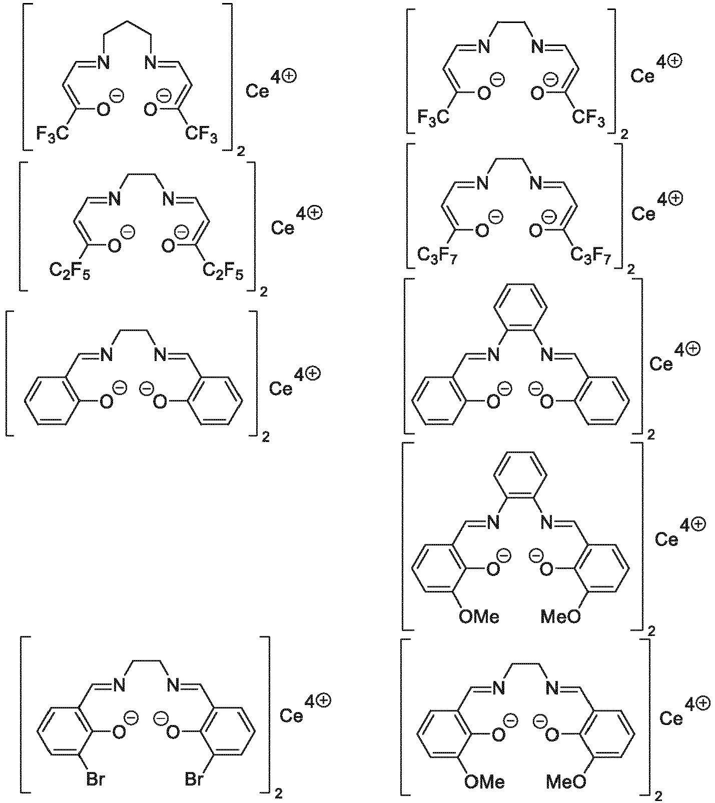

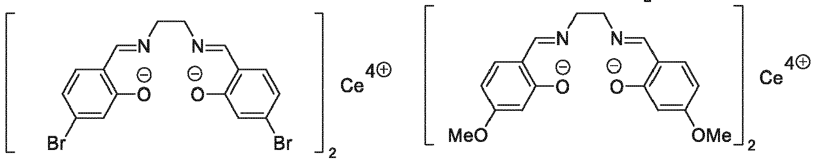

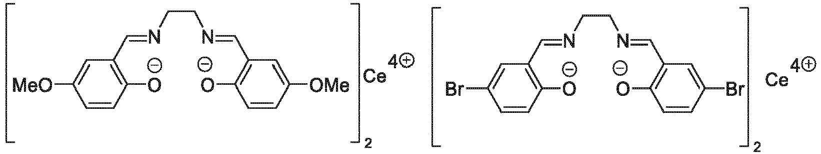

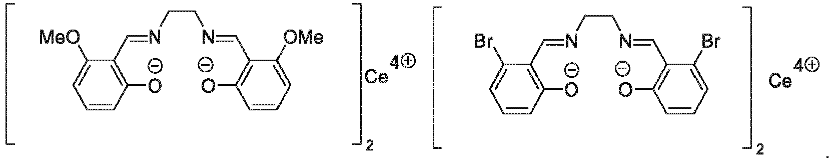

Ein weiterer Gegenstand der Erfindung sind Verbindungen der allgemeinen Formel (I)

Ce⁴⁺[L₁L₂]⁴⁻ (I),

deren Ladungstransferkomplexe, deren Reduktionsprodukte, und Mischungen davon, wobei L₁ und L₂ jeweils ein tetradentater Ligand ist, die unabhängig voneinander ausgewählt sind unter Liganden der Formel (II) 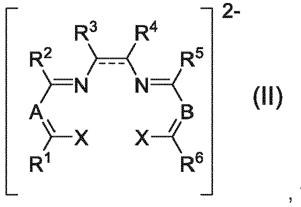 wobei
-̅-̅-̅-̅-̅-̅ zwischen der Gruppe CR³-CR⁴ für eine Einfach- oder Doppelbindung steht,
X unabhängig voneinander für O oder S stehen;
A und B unabhängig voneinander für N oder CR⁷ stehen;
R¹ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R¹ und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
R⁶ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R³ und R⁴ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl oder Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R³ und R⁴ gemeinsam mit den Kohlenstoffen, an denen sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R² und R⁵ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl oder Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
R^{4a}R^{4b} unabhängig voneinander ausgewählt sind unter Wasserstoff, C₁-C₆-Alkyl oder C₆-C₁₄-Aryl, wobei Aryl unsubstituiert oder mit 1, 2, 3, 4 oder 5 identischen oder verschiedenen Resten R¹⁰ substituiert ist;
R⁷ ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
R⁸ ausgewählt ist unter Wasserstoff, C₁-C₆-Alkyl, C₁-C₆-Haloalkyl oder C₆-C₁₄-Aryl, wobei Aryl unsubstituiert oder mit 1, 2, 3, 4 oder 5 identischen oder verschiedenen Resten R¹⁰ substituiert ist;
R⁹ ausgewählt ist unter CN, Halogen, C₁-C₆-Alkyl, C₁-C₆-Haloalkyl oder C₆-C₁₄-Aryl, wobei Aryl unsubstituiert oder mit 1, 2, 3, 4 oder 5 identischen oder verschiedenen Resten ausgewählt unter C₁-C₄-Alkyl und C₁-C₄-Haloalkyl substituiert ist;
R¹⁰ ausgewählt ist unter CN, Halogen, C₁-C₄-Alkyl und C₁-C₄-Haloalkyl, wobei folgende Verbindungen und deren Reduktionsprodukte der Formel (I) ausgenommen sind, wobei
L₁ = L₂ = N,N'-bis(4,4,4,-trifluorobut-1-en-3-on)-ethylendiamin,
L₁ = L₂ = N,N'-bis(4,4,5,5,5-pentafluoropent-1-en-3-on)-ethylendiamin,
L₁ = L₂ = N,N'-bis(4,4,5,5,6,6,6-heptafluorohex-1-en-3-on)-ethylendiamin, und folgende Verbindungen und deren Reduktionsprodukte:

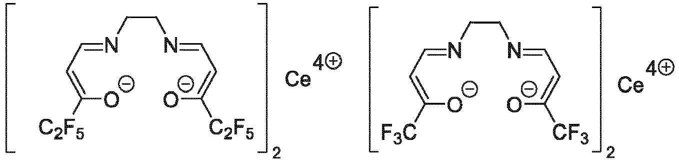

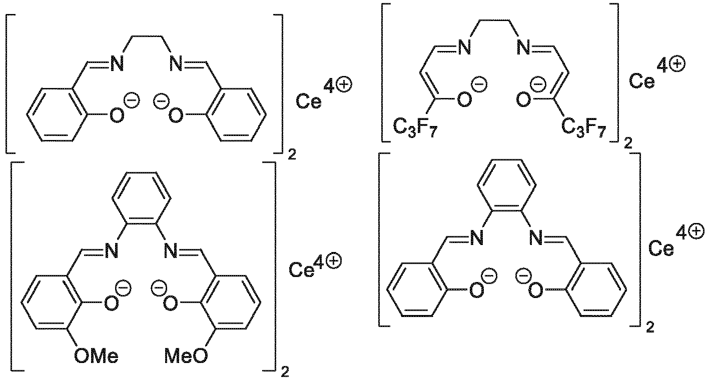

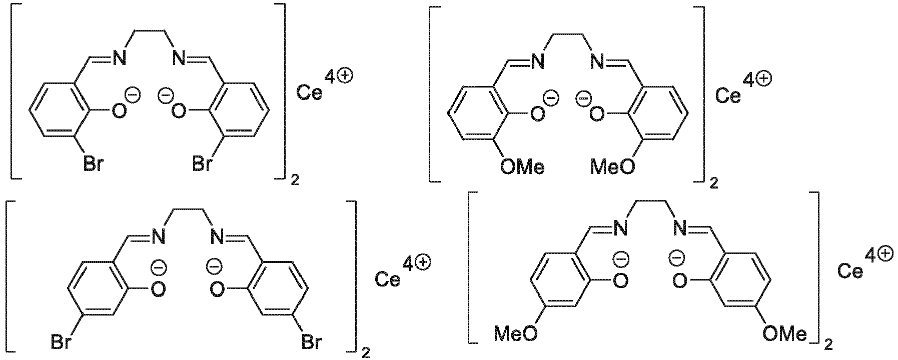

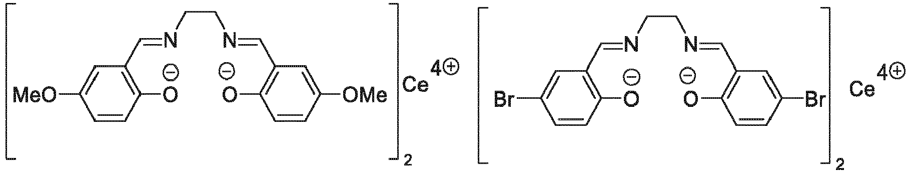

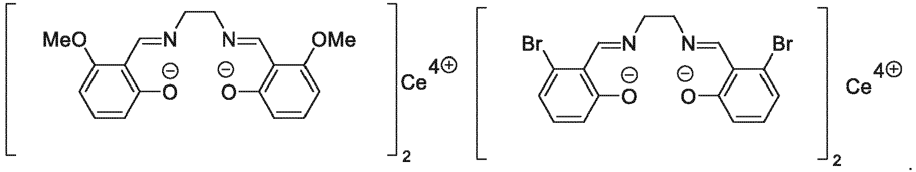

Die folgenden Beispiele dienen der Verdeutlichung der Erfindung, ohne sie in irgendeiner Weise zu beschränken.

### BEISPIELE

### Abkürzungen:

- APCI-MS: atmospheric pressure chemical ionization-Massenspektrometer
- DCM: Dichlormethan
- DEM: Ethylenglycoldimethylether
- HTM: Löchertransportmaterial
- MeCN: Acetonitril
- iPrOH: Iso-Propanol
- EDTA: Ethylendiamintetraessigsäure

Cer-isopropoxid wurde gemäß *"*Gradeff, P.S. et al., Journal of the less common metals, Vol. 126, 1986, 335-338." hergestellt.

Probenherstellung zur Leitfähigkeitsmessung:
Zur Herstellung der dotierten Dünnschichtproben wurden Glassubstrate mit 50nm dicken, strukturierten Au-Elektroden mit Kanallänge 100 µm genutzt. Die Schichten wurden entweder mittels thermischer Verdampfung oder durch Abscheidung aus der Flüssigphase, z.B. mit Rotationsbeschichtung ("spin-coating"), aufgebracht. Die thermische Verdampfung fand bei Raumtemperatur im Hochvakuum (Basisdruck < 5x10-7 mbar) statt. Löchertransportmaterial (HTM) und Dotand wurden entsprechend einer Dotandenkonzentration von 20wt% co-verdampft, wobei die Verdampfungsraten mit zwei unabhängigen Quarzkristall-Mikrowaagen kontrolliert wurden.

Die flüssigprozessierten Schichten wurden aus einer Chlorobenzollösung von HTM und Dotand bei 3000 rpm aufgeschleudert. Die Konzentration von Dotand im HTM war nominell 10wt%. Anschließend wurden die Schichten bei 80°C für 5 min ausgeheizt. Die Schichtdicke wurde mittels Profilometrie überprüft und betrug zwischen 50 und 110 nm.

### Charakterisierung:

Die laterale Leitfähigkeit der Schichten wurde aus dem Anstieg der Strom-Spannungskennlinien zwischen -10 und 10V ermittelt. Die Messung fand direkt nach Probenherstellung für thermische verdampfte Schichten in-situ im Hochvakuum statt, für flüssigprozessierte Schichten in Luft.

### Synthese von 1:

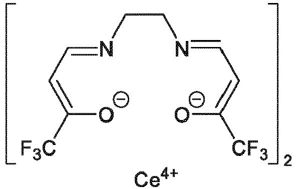

Die Synthese wurde entsprechend der Literatur J. Schläfer, D. Graf, G. Fornalczyk, A. Mettenbörger, S. Mathur, Inorg. Chem. 2016, 55, 5422-5429 durchgeführt.

Die Cyclovoltametrie von 1 in Dichlormethan (DCM, 200 mV/s, 0.1M NBu₄PF₆) ergab folgendes: E_{1/2} (vs. Fc/Fc⁺) = -0.51V.

Verbindung 1 sublimiert ohne Rückstand bei einer Außentemperatur von 140°C und einem Druck von 10⁻² mbar.

### Synthese von 2:

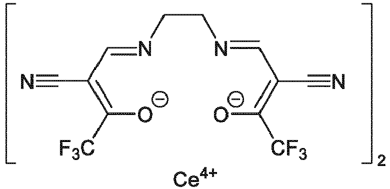

Die Herstellung von 4,4,4-Trifluoro-3-oxobutannitril wurde nach WO2005/49033 durchgeführt.

4,4,4-Trifluoro-3-oxobutannitril (1.9 g, 13.9 mmol) wurde in Essigsäureanhydrid (Ac₂O, 5 ml) und (EtO)₃CH (2.05 g, 13.9 mmol) gelöst. Das Reaktionsgemisch wurde für eine Stunde refluxiert. Im Anschluss wurden alle volatilen Bestandteile im Vakuum entfernt. Der braune Rückstand wurde in Dichlormethan (DCM 20 ml) gelöst und 1,2-Diaminoethan (0.2 ml) zugegeben. Der weiße Niederschlag wurde abfiltriert (0.079 g, 0.22 mmol, 1.6%). Das Massenspektrum des Liganden zeigt [M-H]⁺; 353.

Zu einer 0.3M Lösung von Ce₂(OiPr)₈(HOiPr)₂ (0.17 ml, 0.055 mmol) in Ethylenglycoldimethylether/iPropanol (DME/iOrOH, 1:1 v/v) wurde der Ligand (76 mg, 0.21 mmol) unter N₂ zugeben. Nach 30 Minuten wurde das Lösungsmittel entfernt. Es wurden 0.078 mg roter Feststoff der Verbindung 2 erhalten. Das Massenspektrum der Verbindung 2 zeigt [M-H]⁻; 843.

Die Cyclovoltametrie von 2 in Acetonitril (ACN, 200 mV/s, 0.1M NBu₄PF₆) ergab folgendes: E_{1/2} (vs. Fc/Fc⁺) = +0.40V.

### Synthese von 3:

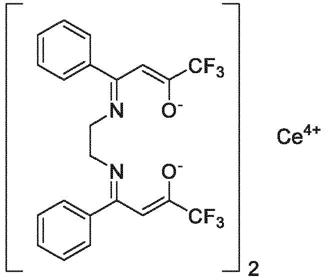

Die Synthese des Liganden wurde entsprechend der Literaur Gurley, L.; Beloukhina, N.; Boudreau, K.; Klegeris, A.; McNeil, W. S. Journal of Inorganic Biochemistry 2011, 105, 6, 858 - 866 durchgeführt.

Zu einer 0.4M Lösung von Ce₂(OiPr)₈(HOiPr)₂ (0.33 ml, 0.131 mmol) in Ethylenglycoldimethylether/iPropanol (DME/iPrOH) (1:1 v/v) wurde 1 (120 mg, 0.26 mmol) unter N₂ zugeben. Nach 30 Minuten wurde das Lösungsmittel entfernt und der Feststoff mit Diethylether (Et₂O) gewaschen. Es wurden 56 mg roter Feststoff der Verbindung 3 erhalten. Das Massenspektrum der Verbindung 3 zeigt [M-H]-; 1047.

Die Cyclovoltammetrie von 3 in Acetonitril (ACN, 200 mV/s, 0.1M NBu₄PF₆) ergab folgendes: E_{1/2} (vs. Fc/Fc⁺) = -0,36V.

### Synthese von 4:

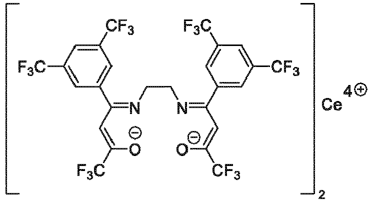

3,5-Bistrifluoromethylacetophenon (8 g, 31,3 mmol) wurde in Ethanol (40 ml) gelöst, und Essigsäure (0,1 ml) wurde zugegeben. Dann wurde 1,2-Diaminoethan (0,94 g, 15,6 mmol) zugegeben und die Lösung im Druckröhrchen für 12 h bei 90°C erhitzt. Beim Abkühlen auf -20°C bilden sich farblose Kristalle, welche abfiltriert wurden. Nach Trocknung im Vakuum erhält man 2.0 g (0,37 mmol, 24%) eines farblosen Feststoffs, N1,N2-Bis(1-(3,5-bis(trifluoromethyl)phenyl)vinyl)ethan-1,2-diamin, APCI-MS (positiv): 537.3 [M+H]⁺.

N1,N2-Bis(1-(3,5-bis(trifluoromethyl)phenyl)vinyl)ethan-1,2-diamin (9.72 g, 18.1 mmol) wurde in DCM (50 ml) suspendiert und Trifluoressigsäureanhydrid (8.37 g, 39.8 mmol) wurden zugegeben. Nach 12h Rühren bei Raumtemperatur wurde die Reaktionslösung auf -20°C abgekühlt und der weiße Feststoff abfiltriert. Der Feststoff wurde aus Acetonitril (50 ml) umkristallisiert und abfiltriert. Weißer Feststoff, 4.16 g (5.71 mmol, 31%): 4,4'-(ethane-1,2-diylbis(azanediyl))bis(4-(3,5-bis(trifluoromethyl)phenyl)-1,1,1-trifluorobut-3-en-2-one), APCI-MS (positive): 729.4 [M+H]⁺.

4,4'-(ethan-1,2-diylbis(azanediyl))bis(4-(3,5-bis(trifluormethyl)phenyl)-1,1,1-trifluorbut-3-en-2-on) (2 g, 2.74 mmol) wurden zu einer Lösung von Cerium(IV)isopropoxid (0.32M in DME/iPrOH, 4,3 ml, 1.37 mmol) gegeben. Nach Rühren für 12 h wurde die Suspension filtriert und mit DME gewaschen. Roter Feststoff, 1.9 g (1,19 mmol, 87%),Cer-bis((2Z,2'Z,4Z,4'Z)-4,4'-(ethan-1,2-diylbis(azanylylide))bis(4-(3,5-bis(trifluormethyl)phenyl)-1,1,1-trifluorbut-2-en-2-olat)) (4), APCI-MS (positive): 1593 [M+H]⁺.

Der Metall-Komplex (4) (538 mg) wurde bei 200°C bei 4·10⁻⁶ mbar sublimiert. Die Ausbeute betrug 335 mg (62%). Der Metall-Komplex (4) zersetzt sich bei 260 °C.

Die Cyclovoltammetrie von 4 in Acetonitril (ACN, 200 mV/s, 0.1M NBu₄PF₆) ergab folgendes: E_{1/2} (vs. Fc/Fc⁺) = -0,12V.

Bei Dotierung von Spiro-MeO-TAD (CAS-Nr: 207739-72-8) mit 20 Gew.% des Dotanden (4) wurden eine Erhöhung der Leitfähigkeit bis zu 1,5·10⁻⁶ S/cm nach Lösungsmittelprozessierung (Spin-Coating) gemessen. Als intrinsische Leitfähigkeit des spiro-MeO-TAD ohne Dotierung wurden 2·10⁻⁸ S/cm gemessen.

### Synthese von 5:

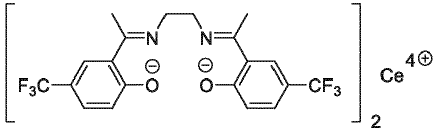

1-(2-hydroxy-5-(trifluoromethyl)phenyl)ethan-1-one (1.19 g, 0.583 mmol) und 1,2-Diaminoethan (0.18 g, 0.29 mmol) wurden in DCM gelöst und 12h gerührt. Danach wurde der Feststoff abfiltriert. Es wurde ein weißer Feststoff, 0.63 g (1.45 mmol, 50%), 2,2'-(-(Ethan-1,2-diylbis(azaneylylidene))bis(ethan-1-yl-1-yliden))bis(4-(trifluoromethyl)phenol) erhalten, APCI-MS (positiv): 433.3 [M+H]⁺.

2,2'-(-(Ethan-1,2-diylbis(azaneylylidene))bis(ethan-1-yl-1-yliden))bis(4-(trifluoromethyl)phenol) (0.59 g, 1.36 mmol) wurden zu einer Lösung von Cerium(IV)isopropoxid (0.32M in DME/iPrOH, 0,68 mmol, 2,12 ml) gegeben. Das Lösungsmittel wurde entfernt und der Rückstand mit Hexan gewaschen. Der Rückstand wurde in CHCl₃ gelöst, filtriert, und mit Hexan gefällt und abfiltriert. Es wurde ein orangeroter Feststoff, 0.42 g (0,42 mmol, 62%), Cer-bis((2,2'-((1E,1'E)-(ethan-1,2-diylbis(azanylyliden))bis(ethan-1-yl-1-yliden))bis(4-(trifluormethyl)phenolat)) (5) erhalten. APCI-MS (positive): 1001 [M+H]⁺.

Der Metall-Komplex (5) (350mg) wurde bei 180-200°C bei 4·10⁻⁶ mbar sublimiert. Die Ausbeute betrug 12 mg (0,3%).

Die Cyclovoltammetrie von 5 in Acetonitril (ACN, 200 mV/s, 0.1M NBu₄PF₆) ergab folgendes: E_{1/2} (vs. Fc/Fc⁺) = +0,3V.

### Synthese von 6:

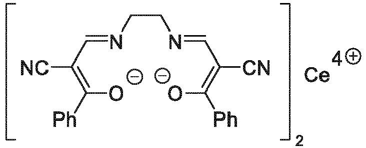

Ethylenediamine (830 mg, 13.6 mmol) wurde zu einer Lösung von 2-Benzoyl-3-ethoxyacrylonitril (5.54 g, 27.6 mmol) in 50 ml Dichlormethan gegeben und bei Raumtemperatur rühren gelassen. Nach 2 h hatte sich ein Niederschlag gebildet, welcher abfiltriert und mit Dichlormethan gewaschen wurde. Nach Trocknen im Vakuum wurden 2.75 g (7.43 mmol, 55%) von (2Z,2'E)-2,2'-((ethan-1,2-diylbis(azandiyl))bis(methanylylidene))bis(3-oxo-3-phenylpropannitril) erhalten.

Zu einer Lösung Cer-isopropoxid (11.25 ml, 3.6 mmol) wurden 2.67g (, 7.2 mmol) (2Z,2'E)-2,2'-((ethan-1,2-diylbis(azandiyl))bis(methanylylidene))bis(3-oxo-3-phenylpropannitril). Der braune Feststoff wurde abfiltriert und mit DME (15ml) und Hexan (20ml) gewaschen. Der Feststoff wurde dann dreimal mit insgesamt 200 ml Dichlormethan gewaschen und die Waschlösung filtriert. Die Waschlösung wurde auf die Hälfte eingeengt und mit Hexan (100ml) versetzt. Der sich gebildete mikrokristalline dunkle Feststoff wurde filtriert und im Vakuum getrocknet. Es wurden 1.80g (2.05 mmol, 57%) erhalten von (6). (6) zersetzt sich bei 170 °C.

Die Cyclovoltammetrie von 6 in Acetonitril (ACN, 200 mV/s, 0.1M NBu₄PF₆) ergab folgendes: E_{1/2} (vs. Fc/Fc⁺) = -0,1V.

### Synthese von 7:

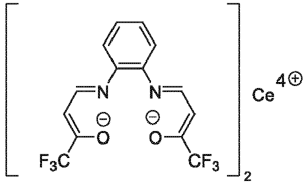

Phenylendiamin (1.62g, 15 mmol) und (E)-4-ethoxy-1,1,1-trifluorobut-3-en-2-one (5g, 30 mmol) wurden zusammen gegeben und 2 d bei Raumtemperatur in 100 ml Dichlormethan gerührt. Die Lösung wurde zur Hälfte eingeengt danach wurden 100 ml Pentan zugegeben. Der mikrokristalline Feststoff, 4,4'-(1,2-Phenylenbis(azanylyliden))bis(1,1,1-trifluorbut-2-en-2-ol) (2,04 g, 5,80 mmol), wurde filtriert und im Vakuum getrocknet. Es wurden 4.12 g (11.7 mmol, 78%) isoliert.

4,4'-(1,2-Phenylenbis(azanylyliden))bis(1,1,1-trifluorbut-2-en-2-ol) (2,04 g, 5,80 mmol) wurde zu einer Lösung Cerium(IV)isopropoxid (0,32M in DME/iPrOH, 9 ml, 2,89 mmol) gegen. Der direkt entstehende rote Feststoff wurde abfiltriert und mit DME gewaschen. Dunkelroter Feststoff (7) wurde erhalten, 1,08 g (1,39 mmol, 48%). APCI-MS (positiv): 841,1 [M+H]⁺.

Der Metall-Komplex (7) (1,00 g) wurde bei 180-210 °C bei 3·10⁻⁶ mbar sublimiert. Die Ausbeute betrug 205 mg (20%). Der Metall-Komplex (7) zersetzt sich bei 237 °C.

Die Cyclovoltammetrie von 7 in Acetonitril (ACN, 200 mV/s, 0.1M NBu₄PF₆) ergab folgendes: E_{1/2} (vs. Fc/Fc⁺) = -0,09V.

### Synthese von 8:

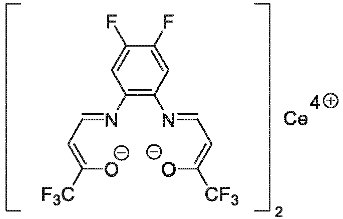

4,5-difluorbenzen-1,2-diamin (2.14g, 15 mmol) und (E)-4-ethoxy-1,1,1-trifluorbut-3-en-2-one (5g, 30 mmol) wurden zusammengegeben und 2 d bei Raumtemperatur in 500 ml Dichlormethan gerührt. 100 ml des Dichlormethans wurden im Vakuum entfernt und 200 ml Pentan zugegeben. Der mikrokristalline Feststoff, (2Z,2'Z,4E,4'E)-4,4'-((4,5-difluor-1,2-phenylen)bis(azanylyliden))bis(1,1,1-trifluorbut-2-en-2-ol), wurde filtriert und im Vakuum getrocknet. Es wurden 3.60 g (9.27 mmol, 62%) isoliert.

4,4'-((4,5-Difluor-1,2-phenylen)bis(azanylyliden))bis(1,1,1-trifluorbut-2-en-2-ol) (2,50 g, 6,44 mmol) wurde zu einer Lösung von Cerium(IV)isopropoxid (0.32M in DME/iPrOH, 4,3 ml, 1.37 mmol) gegeben. Die Suspension wurde filtriert und der rote Feststoff mit wenig DME und Hexan gewaschen. Es wurde ein dunkelroter Feststoff, (8), 0,70 g (0,77 mmol, 12%), Cer-bis((2Z,2'Z,4E,4'E)-4,4'-((4,5-difluor-1,2-phenylen)bis(azanylyliden))bis(1,1,1-trifluorbut-2-en-2-olat)) erhalten, APCI-MS (positiv): 913 [M+H]⁺.

Der Metall-Komplex (8) (700 mg) wurde bei 180°C bei 3·10⁻⁶ mbar sublimiert. Die Ausbeute betrug 150 mg (22%). Der Metall-Komplex (8) zersetzt sich bei 244 °C.

Die Cyclovoltammetrie von 8 in Acetonitril (ACN, 200 mV/s, 0.1M NBu₄PF₆) ergab folgendes: E_{1/2} (vs. Fc/Fc⁺) = +0,08V.

Bei Dotierung von ZnPc (Zink-Phthalocyanin) mit 20 Gew.% des Dotanden (8) wurde eine Erhöhung der Leitfähigkeit bis zu 7,6·10⁻⁵ S/cm nach Co-Verdampfung im Vakuum gemessen. Die intrinsische Leitfähigkeit des ZnPc ohne Dotierung beträgt <1·10⁻⁸ S/cm. *(*Tietze, M. L., Pahner, P., Schmidt, K., Leo, K., & Lüssem, B. (2015). Advanced Functional Materials, 25(18), 2701-2707*).*

Bei Dotierung von MeO-TPD (CAS-Nr: 122738-21-0) mit 20 Gew.% des Dotanden (8) wurde eine Erhöhung der Leitfähigkeit bis zu 1·10⁻⁵ S/cm nach Co-Verdampfung im Vakuum gemessen. Die intrinsische Leitfähigkeit des ZnPc ohne Dotierung beträgt 7·10⁻⁸ S/cm. (Sakai, N., Warren, R., Zhang, F., Nayak, S., Liu, J., Kesava, S. V., Lin, Y.-H., Biswal, H. S., Lin, X., Grovenor, C., Malinauskas, T., Basu, A., Anthopoulos, T. D., Getautis, V., Kahn, A., Riede, M., Nayak, P. K., & Snaith, H. J. (2021). Adduct-based p-doping of organic semiconductors. Nature Materials 2021 20:9, 20(9), 1248-1254.*)*

Bei Dotierung von Spiro-MeO-TAD (CAS-Nr: 207739-72-8) mit 20 Gew.% des Dotanden (8) wurde eine Erhöhung der Leitfähigkeit bis zu 2,8·10⁻⁶ S/cm nach Lösungsmittelprozessierung (Spin-Coating) gemessen. Als intrinsische Leitfähigkeit des spiro-MeO-TAD ohne Dotierung wurden 2·10⁻⁸ S/cm gemessen.

### Synthese von 9:

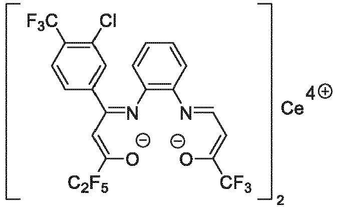

1-(3-Chlor-4-(trifluormethyl)phenyl)-4,4,5,5,5-pentafluorpentan-1,3-dion (3,00 g, 8,13 mmol) wurde in B(nBuO)₃ (9,35 g, 40,7 mmol) suspendiert und 1,2-Diaminophenylen (0,88 g, 8,13 mmol) wurde zugegeben. Nach 15 Minuten wurde der weiße Feststoff abfiltriert und mit wenig Hexan gewaschen. Es wurde ein weißer Feststoff, 2,2 g (4,79 mmol, 59%), 1-((2-Aminophenyl)amino)-1-(3-chloro-4-(trifluoromethyl)phenyl)-4,4,5,5,5-pentafluoropent-1-en-3-on. APCI-MS (positiv): 459 [M+H]⁺.

4-Ethoxy-1,1,1-trifluorobut-3-en-2-on (0,73 g, 4,37 mmol) wurde in DCM gelöst und 1-((2-Aminophenyl)amino)-1-(3-chlor-4-(trifluormethyl)phenyl)-4,4,5,5,5-pentafluorpent-1-en-3-on (2,0 g, 4,37 mmol) zugegeben. Die Reaktionsmischung wurde für 12h gerührt und dann das Lösungsmittel entfernt. Der Rückstand wurde in Acetonitril gelöst und anschießend Kupfer(II)acetat (1,5 g) zugegeben. Zu dieser dunkelgrünen Lösung wurde Wasser zugegeben und der Feststoff abfiltriert. Der Feststoff wurde säulenchromatografisch aufgereinigt (Chloroform als Eluent). Der Rückstand wurde mit Oxalsäure und EDTA, dann konz. HCl und Chlroform behandelt und die Phasen getrennt. Die organische Phase wurde mit gesättigter NaHCOs und gesättigter NaCl-Lösung gewaschen und getrocknet (Na₂SO₄). Das Lösungsmittel wurde entfernt. Es wurde ein weißer Feststoff, 2,0 g (3,44 mmol, 79%), 1-(3-Chloro-4-(trifluormethyl)phenyl)-4,4,5,5,5-pentafluor-1-((2-((-4,4,4-trifluor-3-oxobut-1-en-1-yl)amino)phenyl)amino)pent-1-en-3-on erhalten. APCI-MS (positiv): 581,8 [M+H]⁺.

1-(3-Chlor-4-(trifluormethyl)phenyl)-4,4,5,5,5-pentafluor-1-((2-((-4,4,4-trifluor-3-oxobut-1-en-1-yl)amino)phenyl)amino)pent-1-en-3-on (1,9 g, 3,2 mmol) wurde zu einer Lösung Cerium(IV)isopropoxid (0,32M in DME/iPrOH, 1,63 mmol,5,1 ml) gegeben. Hexan wurde zugegeben und der entstehende Feststoff abfiltriert und mit Hexan gewaschen. Der Rückstand wurde in Chloroform gelöst, filtriert, und aus Chloroform/Hexan umkristallisiert. Es wurde ein roter Feststoff (9), 0,41 g (0,316 mmol, 20%). APCI-MS (positiv): 1296,8 [M+H]⁺.

Der Metall-Komplex (9) (313mg) wurde bei 180-190°C bei 3.10-6 mbar sublimiert. Die Ausbeute betrug 167mg (53%). Der Metall-Komplex (9) zersetzt sich bei 244 °C.

Die Cyclovoltammetrie von 9 in Acetonitril (ACN, 200 mV/s, 0.1M NBu₄PF₆) ergab folgendes: E_{1/2} (vs. Fc/Fc⁺) = +0,0V.

Bei Dotierung von ZnPc (Zink-Phthalocyanin) mit 20 Gew.% des Dotanden (9) wurde eine Erhöhung der Leitfähigkeit bis zu 1,1·10⁻⁵ S/cm nach Co-Verdampfung im Vakuum gemessen. Die intrinsische Leitfähigkeit des ZnPc ohne Dotierung beträgt <1·10⁻⁸ S/cm. *(*Tietze, M. L., Pahner, P., Schmidt, K., Leo, K., & Lüssem, B. (2015). Advanced Functional Materials, 25(18), 2701-2707*).*

Bei Dotierung von Spiro-MeO-TAD (CAS-Nr: 207739-72-8) mit 20 Gew.% des Dotanden (9) wurde eine Erhöhung der Leitfähigkeit bis zu 5,1·10⁻⁶ S/cm nach Lösungsmittelprozessierung (Spin-Coating) gemessen. Als intrinsische Leitfähigkeit des spiro-MeO-TAD ohne Dotierung wurden 2·10⁻⁸ S/cm gemessen.

## Patentansprüche

1. Elektronisches Bauteil, umfassend mindestens eine Verbindung der allgemeinen Formel (I)
Ce⁴⁺[L₁L₂]⁴⁻ (I),
wobei L₁ und L₂ jeweils ein tetradentater Ligand ist, die unabhängig voneinander ausgewählt sind unter Liganden der Formel (I.1) 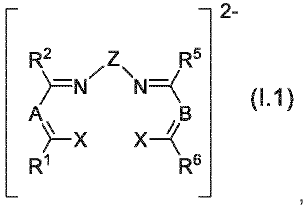 wobei
X unabhängig voneinander für O oder S stehen;
Z für eine Brückengruppe steht, die 2 oder 3 Kohlenstoffatome zwischen den Stickstoffatomen aufweist, wobei die Kohlenstoffatome jeweils unsubstituiert oder mit 1, 2, 3, 4, 5 oder 6 identischen oder verschiedenen Resten R¹² substituiert sind, wobei zwei benachbarte Kohlenstoffatome auch durch eine Doppelbindung miteinander verbunden sein können, oder 2 oder 3 der Kohlenstoffatome Teil eines 1-kernigen, 2- kernigen oder 3-kernigen aromatischen oder heteroaromatischen C₆-C₁₄-Ringsystems sind, wobei das heteroaromatische Ringsystem 4 bis 13 Kohlenstoffatomen aufweist und 1, 2 oder 3 identische oder verschiedene Heteroatome oder heteroatomhaltige Gruppen als Ringglieder hat, die ausgewählt sind unter N, NR⁸, O, S, SO und SO₂ und wobei das aromatische oder heteroaromatische Ringsystem unsubstituiert oder mit 1, 2, 3, 4 oder 5 identischen oder verschiedenen Resten R⁹ substituiert ist;
A und B unabhängig voneinander für N oder CR⁷ stehen;
R¹ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind; oder
R¹ und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
R⁶ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind;
R² und R⁵ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
R² und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
R⁵ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R^{4a}R^{4b} unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
R⁷ ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
R⁸ ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert ist;
R⁹ ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
R¹⁰ ausgewählt ist unter CN, Halogen, C₁-C₄-Alkyl und C₁-C₄-Haloalkyl;
R¹¹ ausgewählt ist unter CN, Halogen, C₁-C₄-Alkyl und C₁-C₄-Haloalkyl;
R¹² unabhängig voneinander ausgewählt sind unter Halogen, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind.

2. Elektronisches Bauteil nach Anspruch 1, umfassend mindestens eine Verbindung der allgemeinen Formel (I)
Ce⁴⁺[L₁L₂]⁴⁻ (I),
wobei L₁ und L₂ jeweils ein tetradentater Ligand ist, die unabhängig voneinander ausgewählt sind unter Liganden der Formel (II) 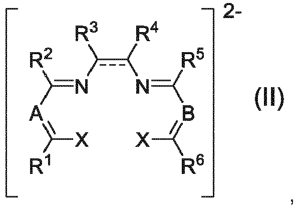 wobei
-̅-̅-̅-̅-̅-̅ zwischen der Gruppe CR³-CR⁴ für eine Einfach- oder Doppelbindung steht,
X unabhängig voneinander für O oder S stehen;
A und B unabhängig voneinander für N oder CR⁷ stehen;
R¹ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind; oder
R¹ und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
R⁶ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind;
R³ und R⁴ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder R³ und R⁴ gemeinsam mit den Kohlenstoffen, an denen sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind;
R² und R⁵ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
R^{4a}R^{4b} unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
R⁷ ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
R⁸ ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert ist;
R⁹ ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
R¹⁰ ausgewählt ist unter CN, Halogen, C₁-C₄-Alkyl und C₁-C₄-Haloalkyl.

3. Elektronisches Bauteil nach Anspruch 1 oder 2, wobei in der Formel (I.1) oder Formel (II) R¹, R², R⁵ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b} und der Gruppe A, bestehend aus A1, A2, A3, A4, A5, A6, A7, A8, A9, A10, A11, A12, A13, A14, A15, A16, A17, A18 and A19 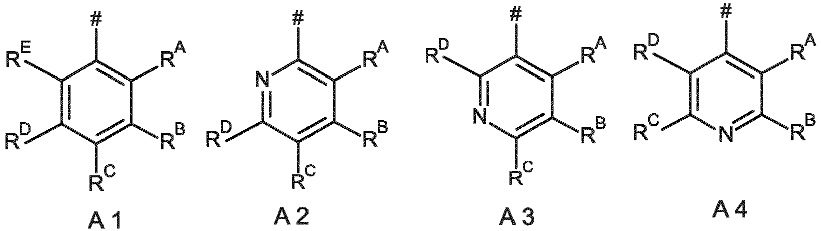 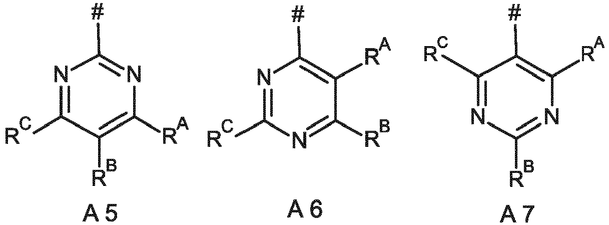 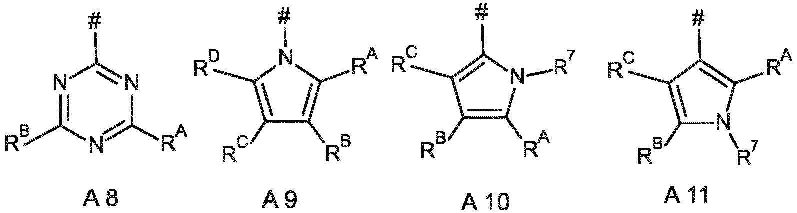 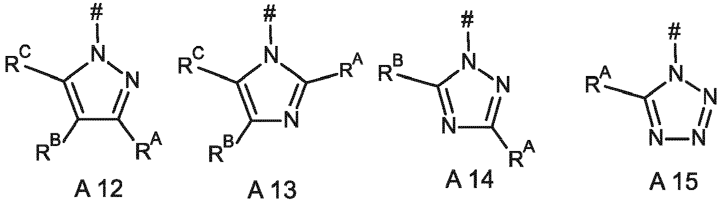 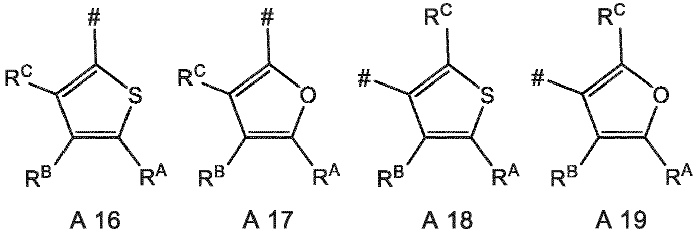
wobei # die Bindung an die Gruppe der Formel (I) bezeichnet, wobei R^{A}, R^{B}, R^{C}, R^{D} und R^{E} unabhängig voneinander ausgewählt sind unter Wasserstoff, CN, Halogen, C₁-C₄-Alkyl und C₁-C₄-Haloalkyl; oder
R¹ und A gemeinsam mit dem Kohlenstoffatom an das sie gebunden sind eine C₆-C₁₄-Arylgruppe bilden, wobei Aryl unsubstituiert oder mit 1, 2, 3, oder 4 identischen oder verschiedenen Resten R⁹ substituiert ist; oder
R⁶ und B gemeinsam mit dem Kohlenstoffatom an das sie gebunden sind eine C₆-C₁₄-Arylgruppe bilden, wobei Aryl unsubstituiert oder mit 1, 2, 3 oder 4 identischen oder verschiedenen Resten R⁹ substituiert ist;
R^{4a}, R^{4b} unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
R⁹ ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten, ausgewählt unter C₁-C₄-Alkyl oder C₁-C₄-Haloalkyl substituiert ist; und
R¹⁰ ausgewählt ist unter CN, Halogen, C₁-C₄-Alkyl und C₁-C₄-Haloalkyl.

4. Elektronisches Bauteil nach einem der vorherigen Ansprüchen, wobei in der Formel (II) R³ und R⁴ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy und Phenyl, wobei Phenyl unsubstituiert oder mit 1, 2, 3 oder 4 identischen oder verschiedenen Resten R⁹ substituiert ist, oder
R³ und R⁴ gemeinsam mit den Kohlenstoffatomen, an denen sie gebunden sind, eine C₆-C₁₄-Arylgruppe bilden, wobei Aryl unsubstituiert oder mit 1, 2, 3, oder 4 identischen oder verschiedenen Resten R⁹ substituiert ist;
R⁹ ausgewählt ist unter CN, Halogen, C₁-C₆-Alkyl, C₁-C₆-Haloalkyl oder C₆-C₁₄-Aryl, wobei Aryl unsubstituiert oder mit 1, 2, 3, 4 oder 5 identischen oder verschiedenen Resten, ausgewählt unter C₁-C₄-Alkyl oder C₁-C₄-Haloalkyl, substituiert ist.

5. Elektronisches Bauteil nach einem der vorherigen Ansprüchen, wobei in der Formel (1.1) oder Formel (II) A und B unhabhängig voneinander für einen Rest CR⁷ stehen und R⁷ ausgewählt ist unter Wasserstoff, CN, Nitro, Halogen und der Gruppe B, bestehend aus B1, B2, B3, B4, B5, B6, B7, B8, B9, B10, B11, B12, B13, B14 und B15 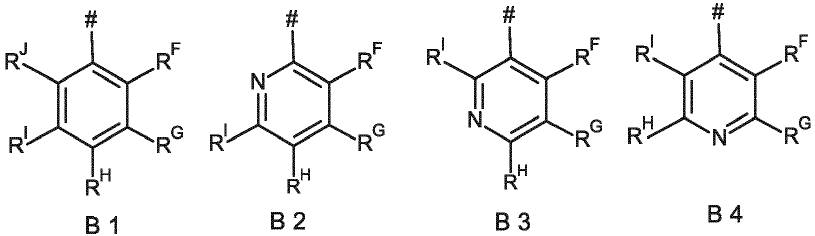 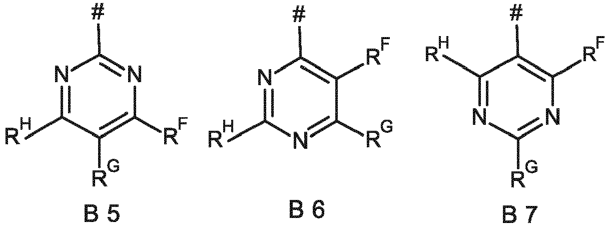 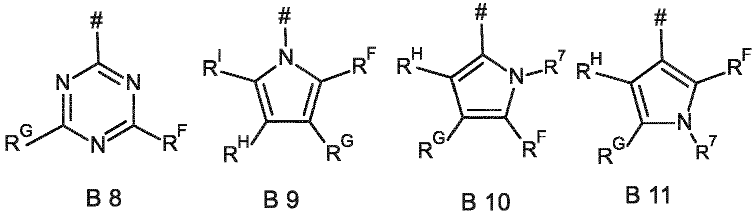 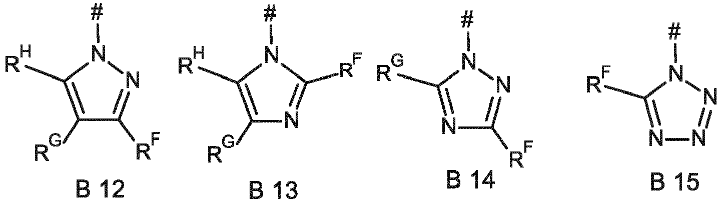
wobei # die Bindung zur Gruppe der Formel (II) bezeichnet, und wobei
R^{F}, R^{G}, R^{H}, R^{I} und R^{J} unabhängig voneinander ausgewählt sind unter Wasserstoff, CN, Halogen, C₁-C₄-Alkyl und C₁-C₄-Haloalkyl.

6. Elektronisches Bauteil nach einem der vorhergehenden Ansprüche, wobei in der Formel (I.1) oder Formel (II) R¹ R², R⁵ und R⁶ unabhängig voneinander ausgewählt sind unter Wasserstoff, C₁-C₄-Alkyl, C₁-C₄-Haloalkyl, A1 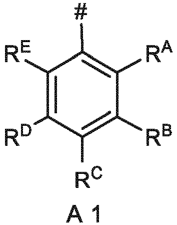
wobei # die Bindung zur Gruppe der Formel (I.1) oder Formel (II) bezeichnet, und wobei R^{A}, R^{B}, R^{C}, R^{D} und R^{E} unabhängig voneinander ausgewählt sind unter Wasserstoff, CN, Fluor, Chlor, C₁-C₄-Alkyl, C₁-C₄-Fluoralkyl und C₁-C₄-Chloralkyl, bevorzugt unter Wasserstoff, CN, Fluor und Chlor; oder
R¹ und A gemeinsam mit dem Kohlenstoffatom, an das sie gebunden sind, einen Phenylring bilden, wobei Phenyl unsubstituiert oder mit 1, 2, 3 oder 4 identischen oder verschiedenen Resten R⁹ substituiert ist;
R⁶ und B gemeinsam mit dem Kohlenstoffatom, an das sie gebunden, sind einen Phenylring bilden, wobei Phenyl unsubstituiert oder mit 1, 2, 3 oder 4 identischen oder verschiedenen Resten R⁹ substituiert ist;
R⁹ ausgewählt ist unter für CN, Halogen, C₁-C₄-Alkyl, C₁-C₄-Haloalkyl oder Phenyl, wobei Phenyl unsubstituiert oder mit 1, 2, 3, 4 oder 5 identischen oder verschiedenen Resten, ausgewählt unter C₁-C₄-Alkyl oder C₁-C₄-Haloalkyl, substituiert ist.

7. Elektronisches Bauteil nach einem der vorhergehenden Ansprüche, wobei in der Formel (I.1) oder Formel (II) A und B unhabhängig voneinander für einen Rest CR⁷ stehen und R⁷ ausgewählt ist unter Wasserstoff, CN, Nitro, Halogen, C₁-C₂-Alkyl und C₁-C₂-Haloalkyl.

8. Elektronisches Bauteil nach einem der vorhergehenden Ansprüche, wobei R³ und R⁴ unhabhängig voneinander ausgewählt sind unter Wasserstoff, CN, C₁-C₄-Alkyl und C₁-C₄-Haloalkyl, oder
R³ und R⁴ gemeinsam mit den Kohlenstoffatomen, an die sie gebunden sind, einen Phenylring bilden, wobei Phenyl unsubstituiert oder mit 1, 2, 3 oder 4 identischen oder verschiedenen Resten R⁹ substituiert ist;
R⁹ ausgewählt ist unter CN, Halogen, C₁-C₄-Alkyl, C₁-C₄-Haloalkyl und Phenyl, wobei Phenyl unsubstituiert oder mit 1, 2, 3 oder 4 identischen oder verschiedenen Resten, ausgewählt unter C₁-C₄-Alkyl oder C₁-C₄-Haloalkyl, substituiert ist.

9. Elektronisches Bauteil nach einem der vorhergehenden Ansprüche, wobei in der Formel (I) L₁ und L₂ die gleiche Bedeutung haben.

10. Elektronisches Bauteil nach einem der vorhergehenden Ansprüche, in Form einer organischen lichtemittierenden Diode, einer organischen Solarzelle, einer Photovoltaikzelle, eines organischen Sensors, einer organischen Diode oder eines organischen Feldeffekttransistors, bevorzugt in Form eines Dünnschichttransistors.

11. Elektronisches Bauteil nach einem der vorhergehenden Ansprüche mit einer Schichtstruktur, die 2, 3, 4, 5, 6, 7 oder mehr Schichten umfasst.

12. Elektronisches Bauteil nach einem der vorhergehenden Ansprüche, umfassend eine Lochtransportschicht und/oder eine Lochinjektionsschicht, die wenigstens eine Verbindung der Formel (I) umfasst.

13. Elektronisches Bauteil nach einem der vorhergehenden Ansprüche, umfassend eine Elektrontransportschicht, die wenigstens eine Verbindung der Formel (I) umfasst.

14. Dotiertes Halbleitermatrixmaterial, umfassend mindestens einen Elektronendonor und mindestens eine Verbindung der Formel (I), wie in einem der Ansprüche 1 bis 9 definiert, wobei der Elektronendonor insbesondere ausgewählt ist unter 4,4',4"-Tris(N-(2-naphthyl)-N-phenyl-amino)triphenylamin, 4,4',4"-Tris(N-3-methylphenyl-N-phenyl-amino)triphenylamin, N,N,N',N'-Tetrakis(4-methoxy-phenyl)benzidin, (2,2',7,7'-Tetrakis-(N,N-diphenylamino)-9,9'-spirobifluoren, N,N'-Bis(naphthalen-1-yl)-N,N'-bis(phenyl)-benzidin, N,N'-Bis(naphthalen-1-yl)-N,N'-bis(phenyl)-9,9-spiro-bifluoren, 9,9-Bis[4-(N,N-bis-biphenyl-4-yl-amino)phenyl]-9H-fluoren, 2,2'-Bis[N,N-bis(biphenyl-4-yl)amino]-9,9-spiro-bifluoren, N,N'-((9H-fluoren-9,9-diyl)bis(4,1-phenylen))bis(N-([1,1'-biphenyl]-4-yl)-[1,1'-biphenyl]-4-amin),N,N'-Bis(phenanthren-9-yl)-N,N'-bis(phenyl)-benzidin, 1,3,5-Tris{4-[bis(9,9-dimethyl-fluoren-2-yl)amino]phenyl}benzol, Tri(terphenyl-4-yl)amin, N-(4-(6-((9,9-Dimethyl-9H-fluoren-2-yl)(6-methoxy-[1,1'-biphenyl]-3-yl)amino)-1,3,3-trimethyl-2,3-dihydro-1H-inden-1-yl)phenyl)-N-(6-methoxy-[1,1'-biphenyl]-3-yl)-9,9-dimethyl-9H-fluoren-2-amin, N-([1,1Biphenyl]-4-yl)-N-(4-(6-([1,1'-biphenyl]-4-yl(9,9-dimethyl-9H-fluoren-2-yl)amino)-1,3,3-trimethyl-2,3-dihydro-1H-inden-1-yl)phenyl)-9,9-dimethyl-9H-fluoren-2-amin, N,N-Di([1,1'-biphenyl]-4-yl)-3-(4-(di([1,1'-biphenyl]-4-yl)amino)phenyl)-1,1,3-trimethyl-2,3-dihydro-1H-inden-5-amin, N-(4-(6-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-(4-(6-(9,9'-Spirobi[fluoren]-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9'-spirobi[fluoren]-2-amin, N-(4-(6-(Dibenzo[b,d]furan-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)dibenzo[b,d]furan-2-amin, 9-(4-(6-(9H-Carbazol-9-yl)-1,3,3-trimethyl-2,3-dihydro-1H-inden-1-yl)phenyl)-9H-carbazol, N-([1,1'-Biphenyl]-4-yl)-3-(4-([1,1'-biphenyl]-4-yl(4-methoxyphenyl)amino)phenyl)-N-(4-methoxyphenyl)-1,1,3-trimethyl-2,3-dihydro-1H-inden-5-amin, 3-(4-(Bis(6-methoxy-[1,1'-biphenyl]-3-yl)amino)phenyl)-N,N-bis(6-methoxy-[1,1'-biphenyl]-3-yl)-1,1,3-trimethyl-2,3-dihydro-1H-inden-5-amin, N1-([1,1'-Biphenyl]-4-yl)-N1-(4-(6-([1,1'-biphenyl]-4-yl(4-(diphenylamino)phenyl)amino)-1,3,3-trimethyl-2,3-dihydro-1H-inden-1-yl)phenyl)-N4,N4-diphenylbenzene-1,4-diamin, N,N-Di([1,1'-biphenyl]-4-yl)-4'-(6-(4-(di([1,1'-biphenyl]-4-yl)amino)phenyl)-1,3,3-trimethyl-2,3-dihydro-1H-inden-1-yl)-[1,1'-biphenyl]-4-amin, N-(4-(5-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-(4-(6-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N,N'-bis(9,9-dimethyl-fluoren-2-yl)-N,N'-diphenyl-benzidin (BF-DPB), N,N'-((9H-fluoren-9,9-diyl)bis(4,1-phenylen))bis(N-([1,1'-biphenyl]-4-yl)-[1,1'-biphenyl]-4-amin) (BPAPF), N4,N4,N4',N4'-tetrakis(9,9-dimethyl-9H-fluoren-2-yl)-[1,1'-biphenyl]-4,4'-diamin (TDMFB), N-([1,1'-biphenyl]-2-yl)-N-(9,9-dimethyl-9H-fluoren-2-yl)-9,9'-spirobi[fluoren]-2-amin, (2,7-bis[N,N-bis(4-methoxyphenyl)amino]-9,9-spirobi[9H-fluoren] (spiro-MeO-TPD), einem Gemisch aus N-(4-(5-(bis(9,9-Dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin und N-(4-(6-(bis(9,9-Dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-([1,1'-biphenyl]-4-yl)-9,9-dimethyl-N-(4-(9-phenyl-9H-carbazol-3-yl)phenyl)-9H-fluoren-2-amin und Mischungen davon.

15. Verwendung einer Verbindung (I) oder Mischungen davon, wie in einem der Ansprüche 1 bis 9 definiert,
- als organischer Halbleiter,
- als Dotierstoff in organischen Halbleitermatrixmaterialien, insbesondere als p-Dotand in Lochtransportschichten,
- als Ladungsinjektor in einer Ladungsinjektionsschicht,
- als Elektronentransportschicht,
- als Kathodenmaterial in organischen Batterien,
- als elektrochromes Material,
- als Elektronenakzeptor.

16. Verwendung von durch Reduktion einer Verbindung (I), wie in einem der Ansprüche 1 bis 9 definiert, erhaltenen Ce(III)-Komplexanionen oder von Ladungstransferkomplexen einer Verbindung (I), wie in einem der Ansprüche 1 bis 9 definiert, mit Elektronendonoren als organischer Halbleiter, als Charge-Transferkomplex, als elektrochromes Material oder als Ferromagneten.

17. Verbindung der allgemeinen Formel (I)
Ce⁴⁺[L₁L₂]⁴⁻ (I),
deren Ladungstransferkomplexe, deren Reduktionsprodukte, und Mischungen davon, wobei L₁ und L₂ jeweils ein tetradentater Ligand ist, die unabhängig voneinander ausgewählt sind unter Liganden der Formel (I.1) 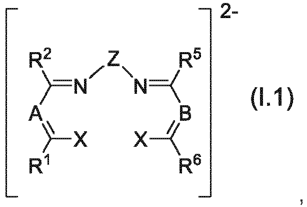 wobei
X unabhängig voneinander für O oder S stehen;
Z für eine Brückengruppe steht, die 2 oder 3 Kohlenstoffatome zwischen den Stickstoffatomen aufweist, wobei die Kohlenstoffatome jeweils unsubstituiert oder mit 1, 2, 3, 4, 5 oder 6 identischen oder verschiedenen Resten R¹² substituiert sind, wobei zwei benachbarte Kohlenstoffatome auch durch eine Doppelbindung miteinander verbunden sein können, oder 2 oder 3 der Kohlenstoffatome Teil eines 1-kernigen, 2- kernigen oder 3-kernigen aromatischen oder heteroaromatischen C₆-C₁₄-Ringsystems sind, wobei das heteroaromatische Ringsystem 4 bis 13 Kohlenstoffatomen aufweist und 1, 2 oder 3 identische oder verschiedene Heteroatome oder heteroatomhaltige Gruppen als Ringglieder hat, die ausgewählt sind unter N, NR⁸, O, S, SO und SO₂ und wobei das aromatische oder heteroaromatische Ringsystem unsubstituiert oder mit 1, 2, 3, 4 oder 5 identischen oder verschiedenen Resten R⁹ substituiert ist;
A und B unabhängig voneinander für N oder CR⁷ stehen;
R¹ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl,
NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R¹ und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind, oder
R⁶ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R² und R⁵ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
R² und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind, oder
R⁵ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R^{4a}R^{4b} unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
R⁷ ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkyl-sulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
R⁸ ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert sind;
R⁹ ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
R¹⁰ ausgewählt ist unter CN, Halogen, C₁-C₄-Alkyl und C₁-C₄-Haloalkyl;
R¹¹ ausgewählt ist unter CN, Halogen, C₁-C₄-Alkyl und C₁-C₄-Haloalkyl;
R¹² unabhängig voneinander ausgewählt sind unter Halogen, CN, C₁-C₆-Alkyl, C₁-C₆-Haloalkyl, C₁-C₆-Alkoxy, C₁-C₆-Haloalkoxy, C₃-C₇-Cycloalkyl, wobei C₃-C₇-Cycloalkyl unsubstituiert oder mit 1, 2, 3, 4, 5 oder 6 identischen oder verschiedenen Resten R¹¹ substituiert ist, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
wobei folgende Verbindungen und deren Reduktionsprodukte ausgenommen sind:

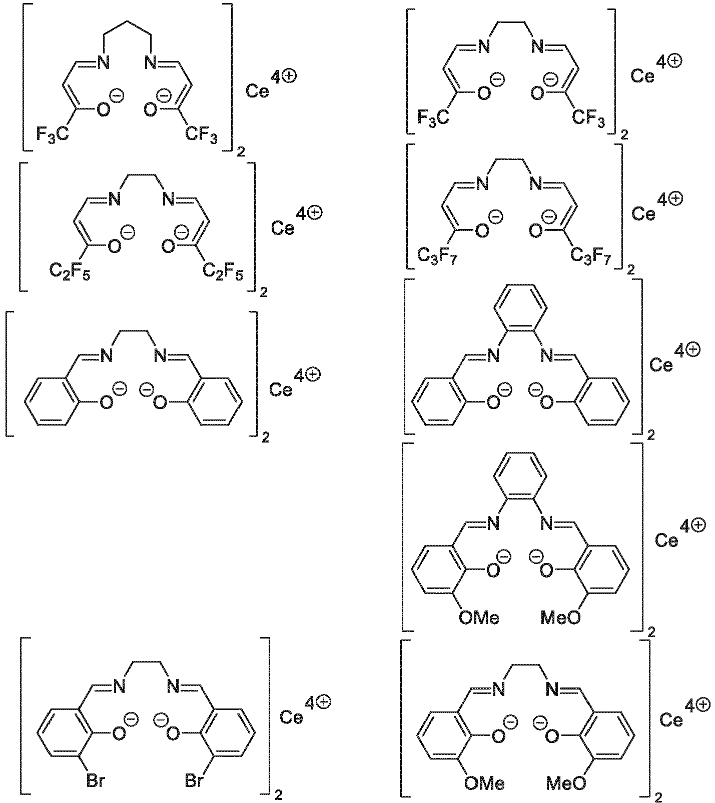

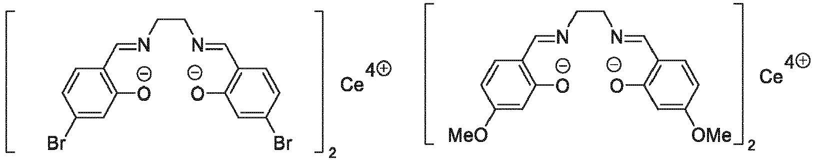

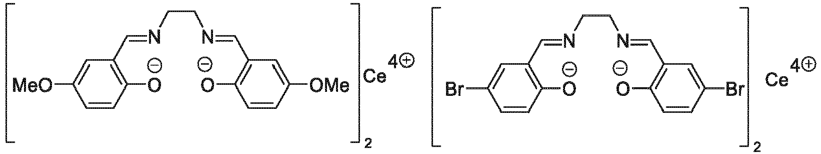

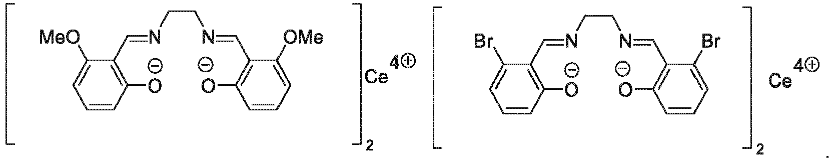

18. Verbindung nach Anspruch 17, mit der allgemeinen Formel (I)
Ce⁴⁺[L₁L₂]⁴⁻ (I),
deren Ladungstransferkomplexe, deren Reduktionsprodukte, und Mischungen davon, wobei L₁ und L₂ jeweils ein tetradentater Liganden ist, die unabhängig voneinander ausgewählt sind unter Ligand der Formel (II) 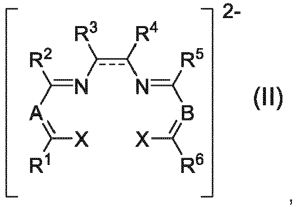 wobei
-̅-̅-̅-̅-̅-̅ zwischen der Gruppe CR³-CR⁴ für eine Einfach- oder Doppelbindung steht,
X unabhängig voneinander für O oder S stehen;
A und B unabhängig voneinander für N oder CR⁷ stehen;
R¹ und R⁶ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R¹ und A gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, oder 4 identischen oder verschiedenen Resten R⁹ substituiert sind, oder
R⁶ und B gemeinsam mit dem Kohlenstoffatom, an das sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R³ und R⁴ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind, oder
R³ und R⁴ gemeinsam mit den Kohlenstoffen, an denen sie gebunden sind, eine C₆-C₁₄-Arylgruppe oder Hetarylgruppe mit 4 bis 13 Kohlenstoffatomen bilden, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3 oder 4 identischen oder verschiedenen Resten R⁹ substituiert sind;
R² und R⁵ unabhängig voneinander ausgewählt sind unter Wasserstoff, CN, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkylsulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R⁹ substituiert sind;
R^{4a}R^{4b} unabhängig voneinander ausgewählt sind unter Wasserstoff, C₁-C₆-Alkyl und C₆-C₁₄-Aryl, wobei Aryl unsubstituiert oder mit 1, 2, 3, 4 oder 5 identischen oder verschiedenen Resten R¹⁰ substituiert ist;
R⁷ ausgewählt ist unter Wasserstoff, CN, Nitro, Halogen, C₁-C₆-Alkyl, C₁-C₆-Haloalkyl, C₁-C₆-Alkoxy, C₁-C₆-Haloalkoxy, C₁-C₆-Alkylsulfanyl, C₁-C₆-Haloalkyl-sulfanyl, NR^{4a}R^{4b}, C₆-C₁₄-Aryl und Hetaryl mit 4 bis 13 Kohlenstoffatomen, wobei Hetaryl 1, 2 oder 3 identische oder verschiedene Heteroatome oder heteroatomhaltige Gruppen als Ringglieder hat, die ausgewählt sind unter N, NR⁸, O, S, SO und SO₂, wobei Aryl und Hetaryl unsubstituiert oder mit 1, 2, 3, 4 oder 5 identischen oder verschiedenen Resten R¹⁰ substituiert sind;
R⁸ ausgewählt ist unter Wasserstoff, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten R¹⁰ substituiert sind;
R⁹ ausgewählt ist unter CN, Halogen, C₁-C₆-Alkyl, C₁-C₆-Haloalkyl und C₆-C₁₄-Aryl, wobei Aryl unsubstituiert oder mit 1, 2, 3, 4 oder 5 identischen oder verschiedenen Resten ausgewählt unter C₁-C₄-Alkyl oder C₁-C₄-Haloalkyl substituiert ist;
R¹⁰ ausgewählt ist unter CN, Halogen, C₁-C₄-Alkyl und C₁-C₄-Haloalkyl,
wobei folgende Verbindungen und deren Reduktionsprodukte ausgenommen sind:

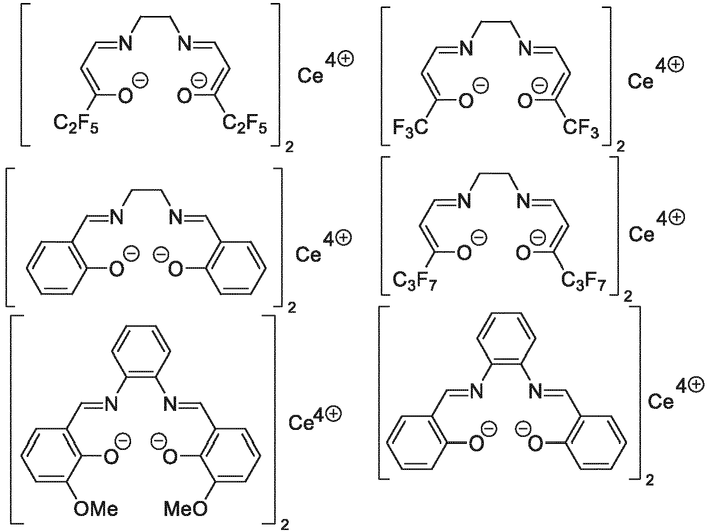

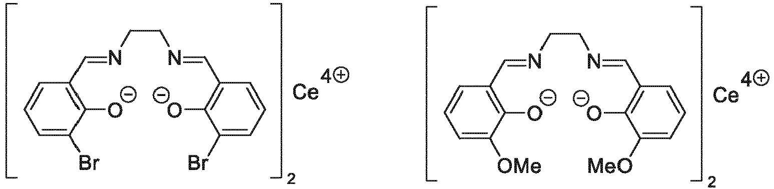

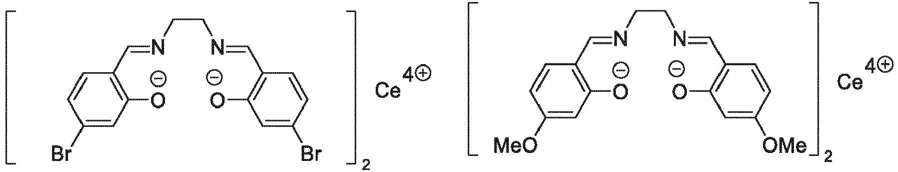

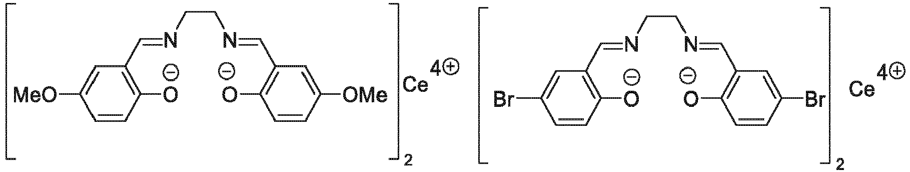

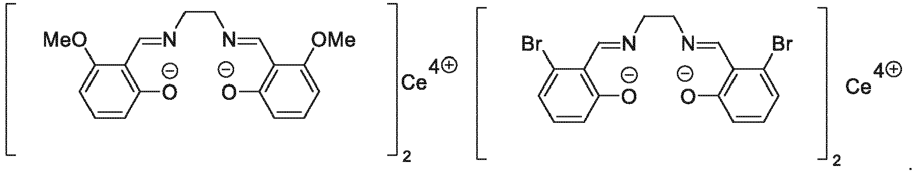

## Claims

1. A electronic component comprising at least one compound of general formula (I)
Ce⁴⁺[L₁L₂]⁴⁻ (I),
wherein L₁ and L₂ are each a tetradentate ligand independently selected from ligands of formula (1.1) 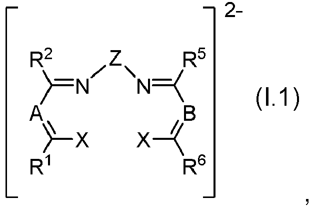 wherein
X independently from each other represents O or S;
Z is a bridging group, which has 2 or 3 carbon atoms between the nitrogen atoms, wherein the carbon atoms each being unsubstituted or substituted by 1, 2, 3, 4, 5 or 6 identical or different radicals R¹², wherein two adjacent carbon atoms may be linked to one another by a double bond, or 2 or 3 of the carbon atoms are part of a mononuclear, 2-nuclear or 3-nuclear C₆-C₁₄ aromatic or heteroaromatic ring system, wherein the heteroaromatic ring system has 4 to 13 carbon atoms and has 1, 2 or 3 identical or different heteroatoms or heteroatom-containing groups as ring members selected from N, NR⁸, O, S, SO and SO₂ and wherein the aromatic or heteroaromatic ring system is unsubstituted or substituted by 1, 2, 3, 4 or 5 identical or different radicals R⁹;
A and B independently from each other represent N or CR⁷;
R¹ and R⁶ are independently selected from hydrogen, CN, C₁-C₆ alkyl, C₁-C₆ haloalkyl, C₁-C₆ alkoxy, C₁-C₆ haloalkoxy, C₃-C₇ cycloalkyl, wherein C₃-C₇ cycloalkyl is unsubstituted or substituted by 1, 2, 3, 4, 5 or 6 identical or different radicals R¹¹,
C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹; or
R¹ and A together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹, or
R⁶ und B together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹;
R² und R⁵ are independently selected from hydrogen, CN, C₁-C₆ alkyl, C₁-C₆ haloalkyl, C₁-C₆ alkoxy, C₁-C₆ haloalkoxy, C₃-C₇ cycloalkyl, wherein C₃-C₇ cycloalkyl is unsubstituted or substituted by 1, 2, 3, 4, 5 or 6 identical or different radicals R¹¹,
C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹;
R² and A together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹, or
R⁵ and B together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹;
R^{4a}R^{4b} are independently from each other selected from hydrogen, C₁-C₆ alkyl and C₆-C₁₄ aryl, wherein aryl is unsubstituted or substituted with 1, 2, 3, 4 or 5 identical or different radicals R¹⁰;
R⁷ is selected from hydrogen, CN, nitro, halogen, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl has 1, 2 or 3 identical or different heteroatoms or heteroatom-containing groups as ring members selected from N, NR⁸, O, S, SO and SO₂, wherein aryl and hetaryl are unsubstituted or substituted with 1, 2, 3, 4 or 5 identical or different radicals R¹⁰;
R⁸ is selected from hydrogen, C₁-C₆ alkyl, C₁-C₆ haloalkyl and C₆-C₁₄ aryl, wherein aryl is unsubstituted or substituted with 1, 2, 3, 4 or 5 identical or different radicals R¹⁰;
R⁹ is selected from CN, halogen, C₁-C₆ alkyl, C₁-C₆ haloalkyl and C₆-C₁₄ aryl, wherein aryl is unsubstituted or substituted with 1, 2, 3, 4 or 5 identical or different radicals selected from C₁-C₄ alkyl and C₁-C₄ haloalkyl;
R¹⁰ is selected from CN, halogen, C₁-C₄ alkyl, C₁-C₄ haloalkyl;
R¹¹ is selected from CN, halogen, C₁-C₄ alkyl, C₁-C₄ haloalkyl;
R¹² independently selected from halogen, CN, C₁-C₆ alkyl, C₁-C₆ haloalkyl, C₁-C₆ alkoxy, C₁-C₆ haloalkoxy, C₃-C₇ cycloalkyl, where C₃-C₇ cycloalkyl is unsubstituted or substituted by 1, 2, 3, 4, 5 or 6 identical or different radicals R¹¹, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹.

2. The electronic component according to claim 1 comprising at least one compound of general formula (I)
Ce⁴⁺[L₁L₂]⁴⁻ (I),
wherein L₁ and L₂ are each a tetradentate ligand independently selected from ligands of formula (II) 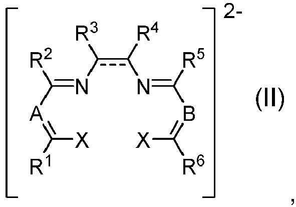 wherein
-̅ -̅ -̅ -̅ -̅ -̅ between the group CR³-CR⁴ represents a single or double bond;
X independently from each other represents O or S;
A and B independently from each other represent N or CR⁷;
R¹ and R⁶ are independently selected from hydrogen, CN, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹; or
R¹ and A together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹, or
R⁶ und B together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹;
R³ and R⁴ are independently selected from hydrogen, CN, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹, or
R³ and R⁴ together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different R⁹ radicals;
R² and R⁵ are independently selected from hydrogen, CN, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹;
R^{4a}R^{4b} are independently from each other selected from hydrogen, C₁-C₆ alkyl and C₆-C₁₄ aryl, wherein aryl is unsubstituted or substituted with 1, 2, 3, 4 or 5 identical or different radicals R¹⁰;
R⁷ is selected from hydrogen, CN, nitro, halogen, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b},
C₆-C₁₄ aryl and hetaryl having 4 to 13 carbon atoms, wherein hetaryl has 1, 2 or 3 identical or different heteroatoms or heteroatom-containing groups as ring members selected from N, NR⁸, O, S, SO and SO₂, wherein aryl and hetaryl are unsubstituted or substituted with 1, 2, 3, 4 or 5 identical or different radicals R¹⁰;
R⁸ is selected from hydrogen, C₁-C₆ alkyl, C₁-C₆ haloalkyl and C₆-C₁₄ aryl, wherein aryl is unsubstituted or substituted with 1, 2, 3, 4 or 5 identical or different radicals R¹⁰;
R⁹ is selected from CN, halogen, C₁-C₆ alkyl, C₁-C₆ haloalkyl and C₆-C₁₄ aryl, wherein aryl is unsubstituted or substituted with 1, 2, 3, 4 or 5 identical or different radicals selected from C₁-C₄ alkyl and C₁-C₄ haloalkyl;
R¹⁰ is selected from CN, halogen, C₁-C₄ alkyl, C₁-C₄ haloalkyl.

3. The electronic component according to claim 1 or 2, wherein in formula (1.1) or formula (II) wherein R¹, R², R⁵ and R⁶ are independently selected from hydrogen, CN, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b} and group A consisting of A1, A2, A3, A4, A5, A6, A7, A8, A9, A10, A11, A12, A13, A14, A15, A16, A17, A18 and A19 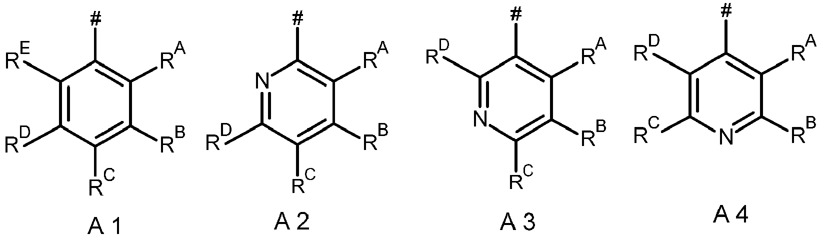 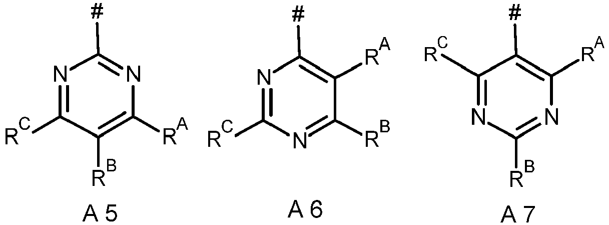 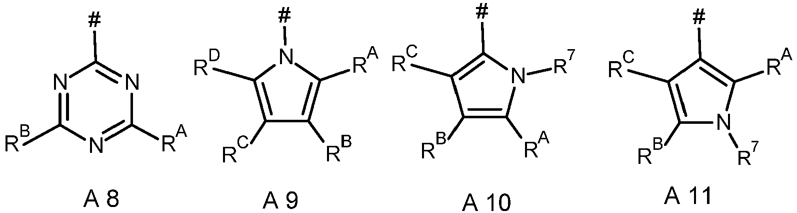 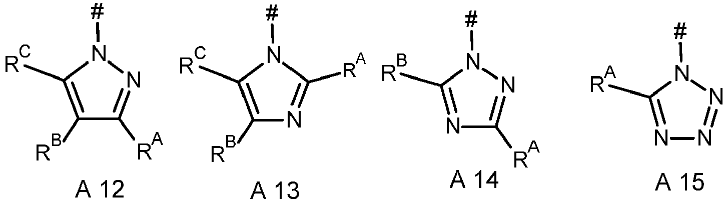 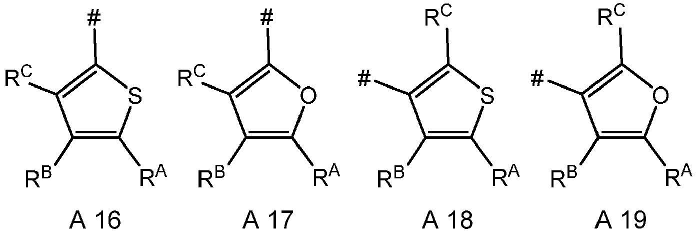
wherein # denotes the bond to the group of formula (I) wherein R^{A}, R^{B}, R^{C}, R^{D} and R^{E} are independently selected from hydrogen, CN, halogen, C₁-C₄ alkyl and C₁-C₄ haloalkyl; or
R¹ and A together with the carbon atom to which they are attached form a C₆-C₁₄ aryl group, wherein aryl is unsubstituted or substituted with 1, 2, 3 or 4 identical or different radicals R⁹; or
R⁶ and B together with the carbon atom to which they are attached form a C₆-C₁₄ aryl group, wherein aryl is unsubstituted or substituted with 1, 2, 3 or 4 identical or different radicals R⁹;
R^{4a}, R^{4b} are independently selected from hydrogen, C₁-C₆ alkyl and C₆-C₁₄ aryl, wherein aryl is unsubstituted or substituted with 1, 2, 3, 4 or 5 identical or different radicals R¹⁰;
R⁹ is selected from CN, halogen, C₁-C₆ alkyl, C₁-C₆ haloalkyl and C₆-C₁₄ aryl, wherein aryl is unsubstituted or substituted with 1, 2, 3, 4 or 5 identical or different radicals selected from C₁-C₄ alkyl and C₁-C₄ haloalkyl; and
R¹⁰ is selected from CN, halogen, C₁-C₄ alkyl and C₁-C₄ haloalkyl.

4. The electronic component according to any one of the preceding claims, wherein in formula (II) R³ and R⁴ are independently selected from hydrogen, CN, C₁-C₆ alkyl, C₁-C₆ haloalkyl, C₁-C₆ alkoxy and C₁-C₆ haloalkoxy and phenyl, wherein phenyl is unsubstituted or substituted by 1, 2, 3 or 4 identical or different radicals R⁹, or
R³ and R⁴ together with the carbon atoms to which they are attached form a C₆-C₁₄ aryl group wherein aryl is unsubstituted or substituted with 1, 2, 3 or 4 identical or different radicals R⁹;
R⁹ is selected from CN, halogen, C₁-C₆ alkyl, C₁-C₆ haloalkyl or C₆-C₁₄ aryl, wherein aryl is unsubstituted or substituted with 1, 2, 3, 4 or 5 identical or different substituents selected from C₁-C₄ alkyl or C₁-C₄ haloalkyl.

5. The electronic component according to any one of the preceding claims, wherein in formula (1.1) or formula (II) A and B are independently a radical CR⁷ and R⁷ is selected from hydrogen, CN, nitro, halogen and group B consisting of B1, B2, B3, B4, B5, B6, B7, B8, B9, B10, B11, B12, B13, B14 and B15 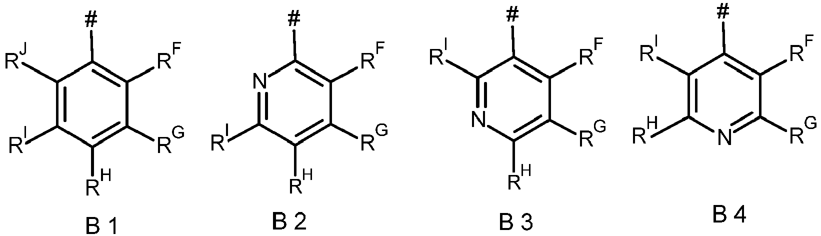 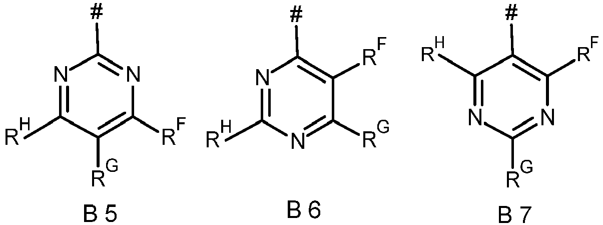 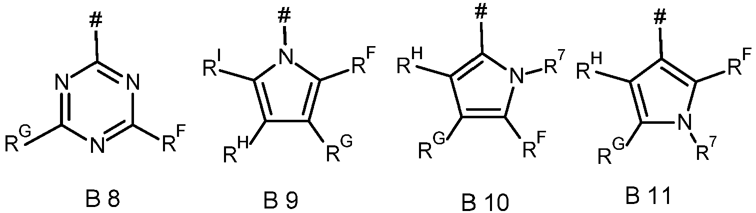 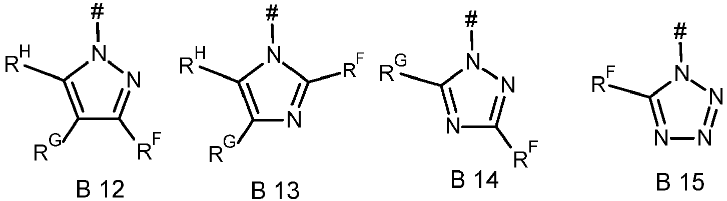 wherein # denotes the bond to the group of formula (II), and wherein R^{F}, R^{G}, R^{H}, R^{I} and R^{J} are independently selected from hydrogen, CN, halogen, C₁-C₄ alkyl and C₁-C₄ haloalkyl.

6. The electronic component according to any one of the preceding claims, wherein in R¹, R², R⁵ and R⁶, in formula (1.1) or formula (II), are independently selected from hydrogen, C₁-C₄ alkyl, C₁-C₄ haloalkyl, and A1 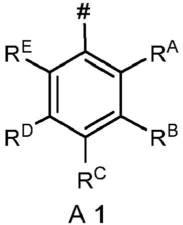
# denotes the bond to the remaining molecule of formula (1.1) or formula (II), and wherein R^{A}, R^{B}, R^{C}, R^{D} and R^{E} are independently selected from hydrogen, CN, fluorine, chlorine, C₁-C₄ alkyl, C₁-C₄ fluoroalkyl and C₁-C₄ chloroalkyl, preferably selected from hydrogen, CN, fluorine and chlorine; or
R¹ and A together with the carbon atom to which they are attached form a phenyl ring, wherein phenyl is unsubstituted or substituted with 1, 2, 3 or 4 identical or different radicals R⁹; or
R⁶ and B together with the carbon atom to which they are attached form a phenyl ring, wherein phenyl is unsubstituted or substituted with 1, 2, 3 or 4 identical or different radicals R⁹;
R⁹ is selected from CN, halogen, C₁-C₄ alkyl, C₁-C₄ haloalkyl and phenyl, wherein phenyl is unsubstituted or substituted with 1, 2, 3, 4 or 5 identical or different substituents selected from C₁-C₄ alkyl or C₁-C₄ haloalkyl.

7. The electronic component according to any one of the preceding claims, wherein in formula (1.1) or formula (II) A and B are independently a radical CR⁷ and R⁷ is selected from hydrogen, CN, nitro, halogen, C₁-C₂ alkyl and C₁-C₂ haloalkyl.

8. The electronic component according to any one of the preceding claims, wherein R³ and R⁴ are selected independently of one another from hydrogen, CN, C₁-C₄-alkyl, C₁-C₄-haloalkyl, or
R³ and R⁴ together with the carbon atoms to which they are attached form a phenyl ring, wherein phenyl is unsubstituted or substituted by 1, 2, 3 or 4 identical or different radicals R⁹;
R⁹ is selected from CN, halogen, C₁-C₄-alkyl, C₁-C₄-haloalkyl and phenyl, where phenyl is unsubstituted or substituted by 1, 2, 3 or 4 identical or different substituents selected from C₁-C₄-alkyl or C₁-C₄-haloalky.

9. The electronic component according to any one of the preceding claims, wherein L₁ and L₂ have the same meanings.

10. The electronic component according to any one of the preceding claims, in the form of an organic light-emitting diode, an organic solar cell, a photovoltaic cell, an organic sensor, an organic diode or an organic field-effect transistor, preferably in form of a thin-film transistor.

11. The electronic component according to any one of the preceding claims, having a layer structure, which comprises 2, 3, 4, 5, 6, 7 or more layers.

12. The electronic component according to any one of the preceding claims, comprising an hole transport layer and/or a hole injection layer, which comprises at least one of the compound of formula (I).

13. The electronic component according to any one of the preceding claims, comprising an electron transport layer, which comprises at least one of the compound of formula (I).

14. Doped semiconductor matrix material comprising at least one electron donor and at least one compound of the formula (I) as defined in any one of claims 1 to 9, wherein the electron donor is preferably selected from 4,4',4"-tris(N-(2-naphthyl)-N-phenyl-amino)triphenylamine (2-TNATA), 4,4',4"-tris(N-3-methylphenyl-N-phenyl-amino)triphenylamine (m-MTDATA), N,N,N',N'-tetrakis(4-methoxy-phenyl)benzidine (MeO-TPD), (2,2',7,7'-tetrakis-(N,N-diphenylamino)-9,9'-spirobifluorene (spiro-TTB), N,N'-bis(naphthalene-1-yl)-N,N'-bis(phenyl)-benzidine, N,N'-bis(naphthalene-1-yl)-N,N'-bis(phenyl)-9,9-spiro-bifluorene, 9,9-bis[4-(N,N-bis-biphenyl-4-yl-amino)phenyl]-9H-fluorene, 2,2'-bis[N,N-bis(biphenyl-4-yl)amino]-9,9-spiro-bifluorene, N,N'-((9H-fluorene-9,9-diyl)bis(4,1-phenylene))bis(N-([1,1'-biphenyl]-4-yl)-[1,1'-biphenyl]-4-amine) (BPAPF), N,N'-bis(phenanthrene-9-yl)-N,N'-bis(phenyl)-benzidine, 1,3,5-tris{4-[bis(9,9-dimethyl-fluorene-2-yl)amino]phenyl}benzene, tri(terphenyl-4-yl)amine, N-(4-(6-((9,9-dimethyl-9H-fluorene-2-yl)(6-methoxy-[1,1'-biphenyl]-3-yl)amino)-1,3,3-trimethyl-2,3-dihydro-1H-indene-1-yl)phenyl)-N-(6-methoxy-[1,1'-biphenyl]-3-yl)-9,9-dimethyl-9H-fluorene-2-amine, N-([1,1'-biphenyl]-4-yl)-N-(4-(6-([1,1'-biphenyl]-4-yl(9,9-dimethyl-9H-fluorene-2-yl)amino)-1,3,3-trimethyl-2,3-dihydro-1H-indene-1-yl)phenyl)-9,9-dimethyl-9H-fluorene-2-amine, N,N-di([1,1'-biphenyl]-4-yl)-3-(4-(di([1,1'-biphenyl]-4-yl)amino)phenyl)-1,1,3-trimethyl-2,3-dihydro-1H-indene-5-amine, N-(4-(6-(bis(9,9-dimethyl-9H-fluorene-2-yl)amino)-1,3,3-trimethyl-2,3-dihydro-1H-inden-1-yl)phenyl)-N-(9,9-dimethyl-9H-fluorene-2-yl)-9,9-dimethyl-9H-fluorene-2-amine, N-(4-(6-(9,9'-spirobi[fluorene]-2-yl(9,9-dimethyl-9H-fluorene-2-yl)amino)-1,3,3-trimethyl-2,3-dihydro-1H-inden-1-yl)phenyl)-N-(9,9-dimethyl-9H-fluoren-2-yl)-9,9'-spirobi[fluorene]-2-amine, N-(4-(6-(dibenzo[b,d]furane-2-yl(9,9-dimethyl-9H-fluorene-2-yl)amino)-1,3,3-trimethyl-2,3-dihydro-1H-indene-1-yl)phenyl)-N-(9,9-dimethyl-9H-fluorene-2-yl)dibenzo[b,d]furan-2-amine, 9-(4-(6-(9H-carbazol-9-yl)-1,3,3-trimethyl-2,3-dihydro-1H-inden-1-yl)phenyl)-9H-carbazole, N-([1,1'-biphenyl]-4-yl)-3-(4-([1,1'-biphenyl]-4-yl(4-methoxyphenyl)amino)phenyl)-N-(4-methoxyphenyl)-1,1,3-trimethyl-2,3-dihydro-1H-inden-5-amine, 3-(4-(bis(6-methoxy-[1,1'-biphenyl]-3-yl)amino)phenyl)-N,N-bis(6-methoxy-[1,1'-biphenyl]-3-yl)-1,1,3-trimethyl-2,3-dihydro-1H-indene-5-amine, N1-([1,1'-biphenyl]-4-yl)-N1-(4-(6-([1,1'-biphenyl]-4-yl(4-(diphenylamino)phenyl)amino)-1,3,3-trimethyl-2,3-dihydro-1H-inden-1-yl)phenyl)-N4,N4-diphenylbenzene-1,4-diamine, N,N-di([1,1'-biphenyl]-4-yl)-4'-(6-(4-(di([1,1'-biphenyl]-4-yl)amino)phenyl)-1,3,3-trimethyl-2,3-dihydro-1H-indene-1-yl)-[1,1'-biphenyl]-4-amine, N-(4-(5-(bis(9,9-dimethyl-9H-fluorene-2-yl)amino)-1,3,3-trimethyl-2,3-dihydro-1H-indene-1-yl)phenyl)-N-(9,9-dimethyl-9H-fluorene-2-yl)-9,9-dimethyl-9H-fluorene-2-amine, N-(4-(6-(bis(9,9-dimethyl-9H-fluorene-2-yl)amino)-1,3,3-trimethyl-2,3-dihydro-1H-indene-1-yl)phenyl)-N-(9,9-dimethyl-9H-fluorene-2-yl)-9,9-dimethyl-9H-fluorene-2-amine, N,N'-bis(9,9-dimethyl-fluorene-2-yl)-N,N'-diphenyl-benzidine (BF-DPB), N,N'-((9H-fluorene-9,9-diyl)bis(4,1-phenylene))bis(N-([1,1'-biphenyl]-4-yl)-[1,1'-biphenyl]-4-amine) (BPAPF), N4,N4,N4',N4'-tetrakis(9,9-dimethyl-9H-fluorene-2-yl)-[1,1'-biphenyl]-4,4'-diamine (TDMFB), N-([1,1'-biphenyl]-2-yl)-N-(9,9-dimethyl-9H-fluorene-2-yl)-9,9'-spirobi[fluorene]-2-amine, (2,7-bis[N,N-bis(4-methoxyphenyl)amino]-9,9-spirobi[9H-fluorene] (spiro-MeO-TPD), a mixture of N-(4-(5-(bis(9,9-dimethyl-9H-fluorene-2-yl)amino)-1,3,3-trimethyl-2,3-dihydro-1H-inden-1-yl)phenyl)-N-(9,9-dimethyl-9H-fluorene-2-yl)-9,9-dimethyl-9H-fluorene-2-amine and N-(4-(6-(bis(9,9-dimethyl-9H-fluorene-2-yl)amino)-1,3,3-trimethyl-2,3-dihydro-1H-inden-1-yl)phenyl)-N-(9,9-dimethyl-9H-fluorene-2-yl)-9,9-dimethyl-9H-fluorene-2-amine, N-([1,1'-biphenyl]-4-yl)-9,9-dimethyl-N-(4-(9-phenyl-9H-carbazol-3-yl)phenyl)-9H-fluorene-2-amine and mixtures thereof.

15. Use of a compound of formula (I) or mixtures thereof, as defined in any one of claims 1 to 9
- as organic semiconductor,
- as a doping agent in organic semiconductor matrix materials, especially as p-dopant in hole transport layers,
- as charge injector in a charge injection layer,
- as electron transport layer,
- as cathode material in organic batteries,
- as electrochromic material
- as electron acceptor.

16. Use of Ce(lll) complex anions obtained by reduction of a compound of formula (I) as defined in any one of claims 1 to 9 or of charge transfer complexes of a compound of formula (I) as defined in any one of claims 1 to 9 with electron donors as organic semiconductor, as electrochromic material or as ferromagnets.

17. A compound of general formula (I)
Ce⁴⁺[L₁L₂]⁴⁻ (I)
, their charge transfer complexes, their reduction products and mixtures thereof, wherein L₁ and L₂ are each a tetradentate ligand independently selected from ligands of formula (1.1) 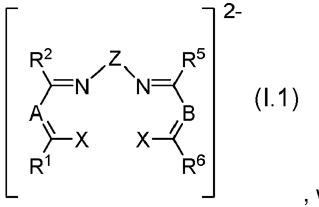 wherein
X independently from each other represents O or S;
Z is a bridging group, which has 2 or 3 carbon atoms between the nitrogen atoms, wherein the carbon atoms each being unsubstituted or substituted by 1, 2, 3, 4, 5 or 6 identical or different radicals R¹², wherein two adjacent carbon atoms may be linked to one another by a double bond, or 2 or 3 of the carbon atoms are part of a mononuclear, 2-nuclear or 3-nuclear C₆-C₁₄ aromatic or heteroaromatic ring system, wherein the heteroaromatic ring system has 4 to 13 carbon atoms and has 1, 2 or 3 identical or different heteroatoms or heteroatom-containing groups as ring members selected from N, NR⁸, O, S, SO and SO₂ and wherein the aromatic or heteroaromatic ring system is unsubstituted or substituted by 1, 2, 3, 4 or 5 identical or different radicals R⁹;
A and B independently from each other represent N or CR⁷;
R¹ and R⁶ are independently selected from hydrogen, CN, C₁-C₆ alkyl, C₁-C₆ haloalkyl, C₁-C₆ alkoxy, C₁-C₆ haloalkoxy, C₃-C₇ cycloalkyl, wherein C₃-C₇-cycloalkyl is unsubstituted or substituted by 1, 2, 3, 4, 5 or 6 identical or different radicals R¹¹,
C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹; or
R¹ and A together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹, or
R⁶ and B together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹;
R² and R⁵ are independently selected from hydrogen, CN, C₁-C₆ alkyl, C₁-C₆ haloalkyl, C₁-C₆ alkoxy, C₁-C₆ haloalkoxy, C₃-C₇ cycloalkyl, wherein C₃-C₇ cycloalkyl is unsubstituted or substituted by 1, 2, 3, 4, 5 or 6 identical or different radicals R¹¹,
C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R9;
R² and A together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹, or
R⁵ and B together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹;
R^{4a}R^{4b} are independently from each other selected from hydrogen, C₁-C₆ alkyl and C₆-C₁₄ aryl, wherein aryl is unsubstituted or substituted with 1, 2, 3, 4 or 5 identical or different radicals R¹⁰;
R⁷ is selected from hydrogen, CN, nitro, halogen, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl has 1, 2 or 3 identical or different heteroatoms or heteroatom-containing groups as ring members selected from N, NR⁸, O, S, SO and SO₂, wherein aryl and hetaryl are unsubstituted or substituted with 1, 2, 3, 4 or 5 identical or different radicals R¹⁰;
R⁸ is selected from hydrogen, C₁-C₆ alkyl, C₁-C₆ haloalkyl and C₆-C₁₄ aryl, wherein aryl is unsubstituted or substituted with 1, 2, 3, 4 or 5 identical or different radicals R¹⁰;
R⁹ is selected from CN, halogen, C₁-C₆ alkyl, C₁-C₆ haloalkyl and C₆-C₁₄ aryl, wherein aryl is unsubstituted or substituted with 1, 2, 3, 4 or 5 identical or different radicals selected from C₁-C₄ alkyl and C₁-C₄ haloalkyl;
R¹⁰ is selected from CN, halogen, C₁-C₄ alkyl, C₁-C₄ haloalkyl;
R¹¹ is selected from CN, halogen, C₁-C₄ alkyl, C₁-C₄ haloalkyl;
R¹² independently selected from halogen, CN, C₁-C₆ alkyl, C₁-C₆ haloalkyl, C₁-C₆ alkoxy, C₁-C₆ haloalkoxy, C₃-C₇ cycloalkyl, where C₃-C₇ cycloalkyl is unsubstituted or substituted by 1, 2, 3, 4, 5 or 6 identical or different radicals R¹¹, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹;
with the proviso that the following compounds are excluded:

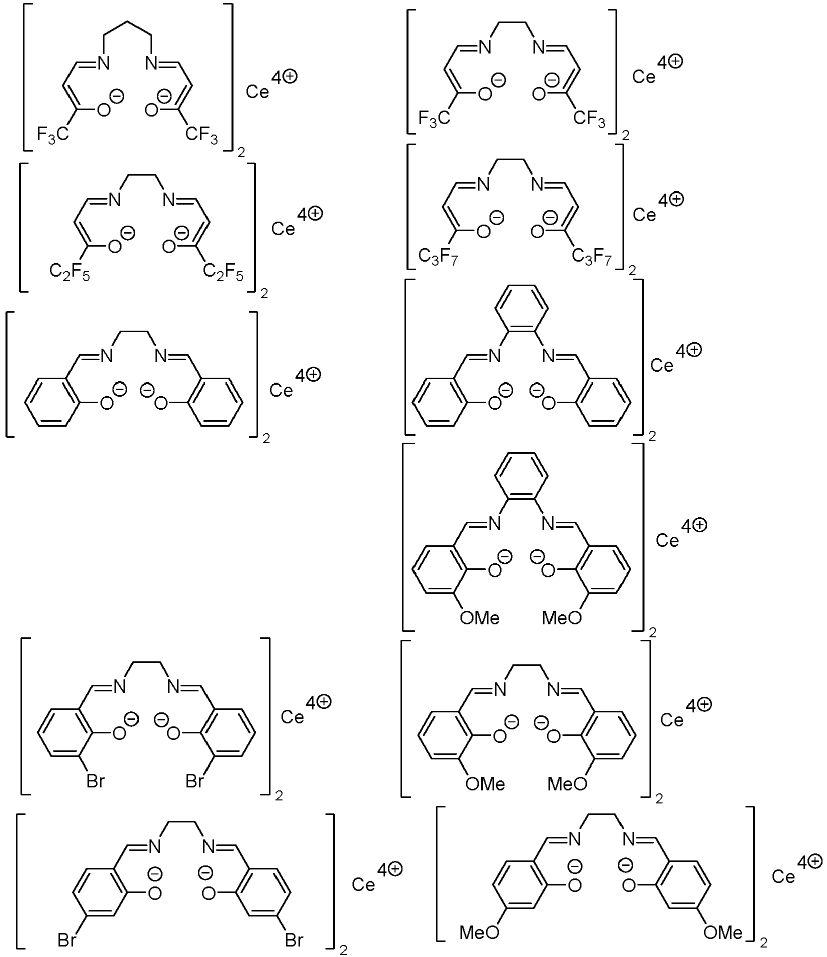

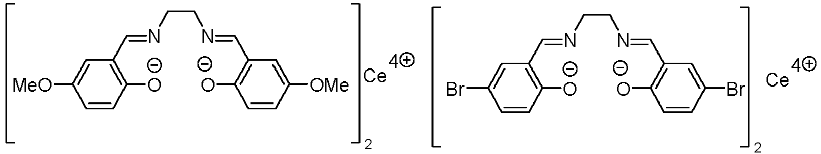

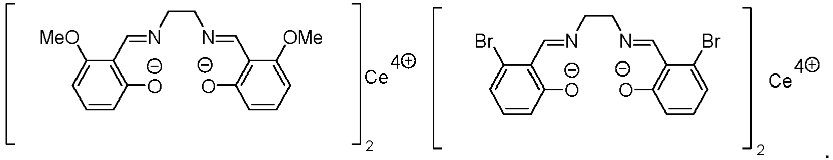

18. The compounds of general formula (I) according to claim 17
Ce⁴⁺[L₁L₂]⁴⁻ (I),
their charge transfer complexes, their reduction products and mixtures thereof, wherein L₁ and L₂ are each a tetradentate ligand independently selected from ligands of formula (II) 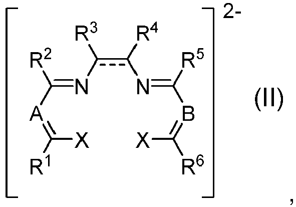 wherein
-̅ -̅ -̅ -̅ -̅ -̅ between the group CR³-CR⁴ represents a single or double bond;
X independently from each other represents O or S;
A and B independently from each other represent N or CR⁷;
R¹ and R⁶ are independently selected from hydrogen, CN, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹; or
R¹ and A together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹, or
R⁶ and B together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different radicals R⁹;
R³ and R⁴ are independently selected from hydrogen, CN, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹, or
R³ and R⁴ together with the carbon atom to which they are attached form a C₆-C₁₄ aryl group or hetaryl group having 4 to 13 carbon atoms, wherein hetaryl having 1, 2 or 3 identical or different heteroatoms or heteroatom-containing groups as ring members selected from N, NR⁸, O, S, SO and SO₂, aryl and hetaryl being unsubstituted or substituted with 1, 2, 3 or 4 identical or different R⁹ radicals;
R² and R⁵ are independently selected from hydrogen, CN, C₁-C₆-alkyl, C₁-C₆-haloalkyl, C₁-C₆-alkoxy, C₁-C₆-haloalkoxy, C₁-C₆-alkylsulfanyl, C₁-C₆-haloalkylsulfanyl, NR^{4a}R^{4b}, C₆-C₁₄ aryl and hetaryl having 4 to 13 carbon atoms, wherein hetaryl having 1, 2 or 3 identical or different heteroatoms or heteroatom-containing groups as ring members selected from N, NR⁸, O, S, SO and SO₂, wherein aryl and hetaryl being unsubstituted or substituted with 1, 2, 3, 4 or 5 identical or different radicals R⁹;
R^{4a}R^{4b} are independently from each other selected from hydrogen, C₁-C₆ alkyl and C₆ C₁₄ aryl, wherein aryl is unsubstituted or substituted with 1, 2, 3, 4 or 5 identical or different radicals R¹⁰;
R⁷ is selected from hydrogen, CN, nitro, halogen, C₁-C₆ alkyl, C₁-C₆ haloalkyl, C₁-C₆ alkoxy, C₁-C₆ haloalkoxy, C₁-C₆ alkylsulfanyl, C₁-C₆ haloalkylsulfanyl, NR^{4a}R^{4b}, C₆-C₁₄ aryl and hetaryl having 4 to 13 carbon atoms, wherein hetaryl has 1, 2 or 3 identical or different heteroatoms or heteroatom-containing groups as ring members selected from N, NR⁸, O, S, SO and SO₂, wherein aryl and hetaryl are unsubstituted or substituted with 1, 2, 3, 4 or 5 identical or different radicals R¹⁰;
R⁸ is selected from hydrogen, C₁-C₆ alkyl, C₁-C₆ haloalkyl and C₆-C₁₄ aryl, wherein aryl is unsubstituted or substituted with 1, 2, 3, 4 or 5 identical or different radicals R¹⁰;
R⁹ is selected from CN, halogen, C₁-C₆ alkyl, C₁-C₆ haloalkyl and C₆-C₁₄ aryl, wherein aryl is unsubstituted or substituted with 1, 2, 3, 4 or 5 identical or different radicals selected from C₁-C₄ alkyl and C₁-C₄ haloalkyl;
R¹⁰ is selected from CN, halogen, C₁-C₄ alkyl, C₁-C₄ haloalkyl,
with the proviso that the following compounds and their reaction products are excluded:

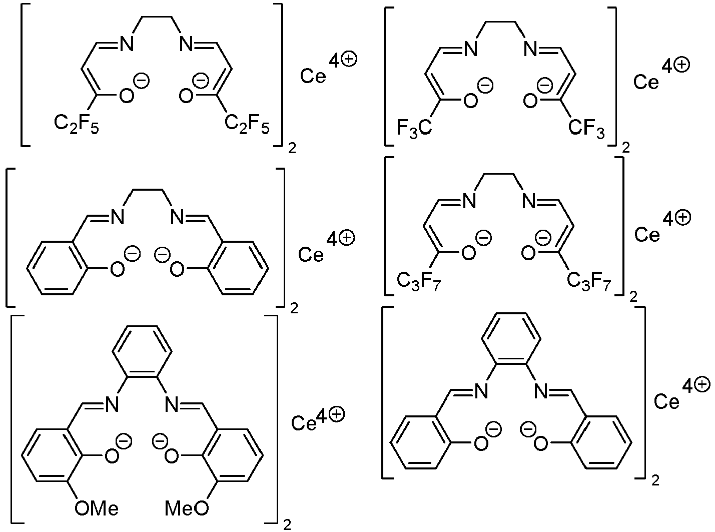

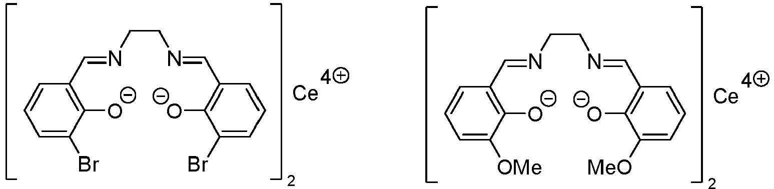

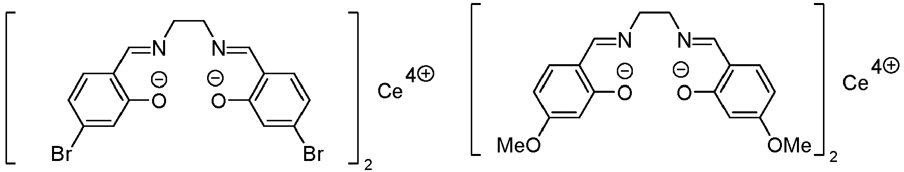

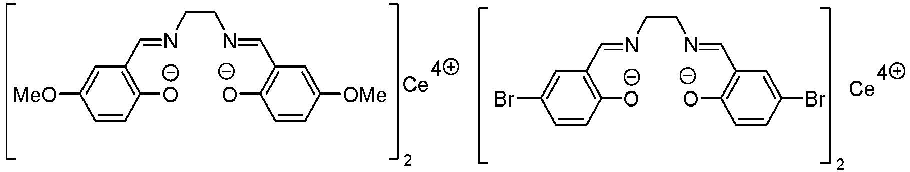

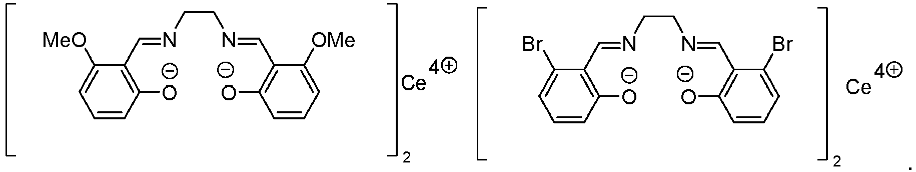

## Revendications

1. Composant électronique comprenant au moins un composé de formule générale (I)
Ce⁴⁺[L₁L₂]⁴⁻ (I),
dans laquelle L₁ et L₂ sont chacun un ligand tétradenté choisi indépendamment l'un de l'autre parmi les ligands de formule (1.1) 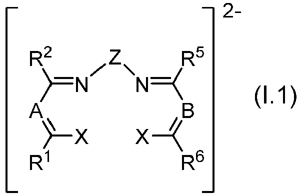 où
X représentent O ou S indépendamment l'un de l'autre;
Z représente un groupe pontant comportant 2 ou 3 atomes de carbone entre les atomes d'azote, les atomes de carbone étant à chaque fois non substitués ou substitués par 1, 2, 3, 4, 5 ou 6 radicaux R¹² identiques ou différents, deux atomes de carbone voisins pouvant également être reliés entre eux par une double liaison, ou 2 ou 3 des atomes de carbone font partie d'un groupe mononucléaire, 2-nucléaire ou 3-nucléaire aromatique ou hétéroaromatique en Ce -C₁₄, le système cyclique hétéroaromatique comportant 4 à 13 atomes de carbone et ayant 1, 2 ou 3 hétéroatomes identiques ou différents ou groupes contenant des hétéroatomes comme éléments du cycle, qui sont choisis parmi N, NR⁸, O, S, SO et SO₂ et où le système cyclique aromatique ou hétéroaromatique est non substitué ou substitué par 1, 2, 3, 4 ou 5 radicaux R⁹ identiques ou différents ;
A et B représentent N ou CR⁷indépendamment l'un de l'autre ;
R¹ et R⁶ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, un cycloalkyle en C₃ -C₇, où le cycloalkyle en C₃ -C₇ est non substitué ou substitué par 1, 2, 3, 4, 5 ou 6 radicaux R¹¹ identiques ou différents, un l'alkylsulfanyle en C₁ -C₆, un haloalkylsulfanyle en C₁ -C₆, NR^{4a} R^{4b} , un aryle en C₆ -C₁₄ et un hétéroaryle ayant de 4 à 13 atomes de carbone, ledit hétéroaryle ayant 1, 2 ou 3 hétéroatomes identiques ou différents ou groupes contenant des hétéroatomes comme éléments du cycle, choisis parmi N, NR⁸, O, S, SO et SO₂, ledit aryle et ledit hétéroaryle étant non substitués ou substitués par 1, 2, 3, 4 ou 5 radicaux R⁹ identiques ou différents ; où
R¹ et A forment ensemble, avec l'atome de carbone auquel ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, l'aryle et l'hétéroaryle étant non substitués ou substitués par 1, 2, 3 ou 4 radicaux R⁹ identiques ou différents, ou
R⁶ et B forment ensemble, avec l'atome de carbone auquel ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, où aryle et hétéroaryle sont non substitués ou substitués par 1, 2, 3 ou 4 radicaux R⁹ identiques ou différents ;
R² et R⁵ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, un cycloalkyle en C₃ -C₇, ledit cycloalkyle en C₃ -C₇ est non substitué ou substitué par 1, 2, 3, 4, 5 ou 6 radicaux R¹¹ identiques ou différents, l'alkylsulfanyle en C₁ -C₆ , l'haloalkylsulfanyle en C₁ -C₆, NR^{4a} R^{4b} , aryle en C₆ -C₁₄ et hétéroaryle ayant de 4 à 13 atomes de carbone, ledit hétéroaryle ayant 1, 2 ou 3 hétéroatomes ou groupes contenant des hétéroatomes identiques ou différents comme éléments du cycle, choisis parmi N, NR⁸, O, S, SO et SO₂, ledit aryle et ledit hétéroaryle étant non substitués ou substitués par 1, 2, 3, 4 ou 5 radicaux R⁹ identiques ou différents ;
R² et A forment ensemble, avec l'atome de carbone auquel ils sont liés, un groupe aryle en C₁ -C₆ où un groupe hétéroaryle ayant de 4 à 13 atomes de carbone, ledit hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, l'aryle et l'hétéroaryle étant non substitués ou substitués par 1, 2, 3 ou 4 radicaux R⁹ identiques ou différents, ou
R⁵ et B forment ensemble avec l'atome de carbone auquel ils sont liés un groupe aryle en C₆ -C₁₄ ou un groupe hétéroaryle ayant de 4 à 13 atomes de carbone, le groupe hétéroaryle ayant 1, 2 ou 3 hétéroatomes identiques ou différents ou groupes contenant des hétéroatomes comme éléments du cycle, choisis parmi N, NR⁸, O, S, SO et SO₂, le groupe aryle et le groupe hétéroaryle étant non substitués ou substitués par 1, 2, 3 ou 4 radicaux R⁹ identiques ou différents ;
R^{4a} R^{4b} sont choisis indépendamment l'un de l'autre parmi l'hydrogène, un groupe alkyle en C₁ -C₆ et un groupe aryle en C₆ -C₁₄ , le groupe aryle étant non substitué ou substitué par 1, 2, 3, 4 ou 5 radicaux R¹⁰ identiques ou différents ;
R⁷ est choisi parmi l'hydrogène, CN, nitro, halogène, alkyle en C₁ -C₆, haloalkyle en C₁ -C₆, alcoxy en C₁ -C₆, haloalcoxy en C₁ -C₆, alkylsulfanyle en C₁ -C₆, haloalkylsulfanyle en C₁ -C₆, NR^{4a} R^{4b} , aryle en C₆ -C₁₄ et hétéroaryle ayant de 4 à 13 atomes de carbone, ledit hétéroaryle a 1, 2 ou 3 hétéroatomes identiques ou différents ou des groupes comme éléments du cycle, choisis parmi N, NR⁸, O, S, SO et SO₂, ledit aryle et ledit hétéroaryle sont non substitués ou substitués par 1, 2, 3, 4 ou 5 radicaux R¹⁰ identiques ou différents ;
R⁸ est choisi parmi l'hydrogène, les groupes alkyles en C₁ -C₆, halogénoalkyles en C₁ -C₆ et aryles en C₆ -C₁₄, le groupe aryle étant non substitué ou substitué par 1, 2, 3, 4 ou 5 radicaux R¹⁰ identiques ou différents ;
R⁹ est choisi parmi CN, halogène, alkyle en C₁ -C₆, haloalkyle en C₁ - C₆ et aryle en C₆ -C₁₄, où l'aryle est non substitué ou substitué par 1, 2, 3, 4 ou 5 radicaux identiques ou différents choisis parmi alkyle en C₁ -C₄ ou haloalkyle en C₁ -C₄ ;
R¹⁰ est choisi parmi CN, un halogène, un alkyle en C₁ -C₄ et un haloalkyle en C₁ -C₄ ;
R¹¹ est choisi parmi CN, un halogène, un alkyle en C₁ -C₄ et un haloalkyle en C₁ -C₄ ;
R¹² sont choisis indépendamment les uns des autres parmi un halogène, CN, un alkyle en C₁ -C₆, un haloalkyle en C₁ -C₆, un alcoxy en C₁ -C₆, un haloalcoxy en C₁ -C₆, un cycloalkyle en C₃ - C₇, ledit cycloalkyle en C₃ -C₇ est non substitué ou substitué par 1, 2, 3, 4, 5 ou 6 radicaux R¹¹ identiques ou différents, l'alkylsulfanyle en C₁ -C , l'haloalkylsulfanyle en C₁ -C₆, NR^{4a} R^{4b} , aryle en C₆ - C₁₄ et hétéroaryle ayant de 4 à 13 atomes de carbone, ledit hétéroaryle ayant 1, 2 ou 3 hétéroatomes identiques ou différents ou groupes contenant des hétéroatomes comme éléments du cycle, choisis parmi N, NR⁸, O, S, SO et SO₂, ledit aryle et ledit hétéroaryle étant non substitués ou substitués par 1, 2, 3, 4 ou 5 radicaux R⁹ identiques ou différents.

2. Composant électronique selon la revendication 1, comprenant au moins un composé de formule générale (I)
Ce⁴⁺[L₁L₂]⁴⁻ (I),
dans laquelle L₁ et L₂ sont chacun un ligand tétradenté choisi indépendamment l'un de l'autre parmi les ligands de formule (II) 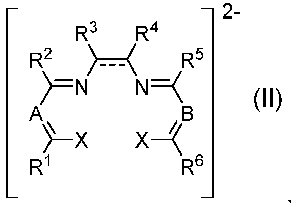 dans laquelle
-̅ -̅ -̅ -̅ -̅ -̅ représente une liaison simple ou double entre le groupe CR³ -CR⁴ ,
X représentent O ou S indépendamment l'un de l'autre ;
A et B représentent N ou CR⁷ indépendamment l'un de l'autre;
R¹ et R⁶ sont choisis indépendamment l'un de l'autre parmi l'hydrogène, CN, un alkyle en C₁-C₆ , un haloalkyle en C₁-C₆, un alcoxy en C₁-C₆, un haloalcoxy en C₁-C₆ , alkylsulfanyle en C₁-C₆, haloalkylsulfanyle en C₁-C₆, NR^{4a} R^{4b} , aryle en C₆ -C₁₄ et hétéroaryle ayant de 4 à 13 atomes de carbone, ledit hétéroaryle ayant de 1, 2 ou 3 hétéroatomes identiques ou différents ou des groupes contenant des hétéroatomes comme éléments du cycle, qui sont choisis parmi N, NR⁸, O, S, SO et SO₂, où aryle et hétéroaryle sont non substitués ou substitués par 1, 2, 3, 4 ou 5 radicaux R⁹ identiques ou différents ; ou
R¹ et A forment ensemble, avec l'atome de carbone auquel ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, l'aryle et l'hétéroaryle étant non substitués ou substitués par 1, 2, 3 ou 4 radicaux R⁹ identiques ou différents, ou
R⁶ et B forment ensemble, avec l'atome de carbone auquel ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, où les groupes aryle et hétéroaryle sont non substitués ou substitués par 1, 2, 3 ou 4 radicaux R⁹ identiques ou différents ;
R³ et R⁴ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, alkylsulfanyle en C₁ -C₆, haloalkylsulfanyle en C₁ -C₆, NR^{4a} R^{4b} , aryle en C₆ -C₁₄ et hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, où les groupes aryles et hétéroaryles sont non substitués ou substitués par 1, 2, 3, 4 ou 5 radicaux R⁹ identiques ou différents, ou
R³ et R⁴ forment ensemble, avec les carbones auxquels ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, où les groupes aryle et hétéroaryle sont non substitués ou substitués par 1, 2, 3 ou 4 radicaux R⁹ identiques ou différents ;
R² et R⁵ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, alkylsulfanyle en C₁ -C₆, haloalkylsulfanyle en C₁ -C₆, NR^{4a} R^{4b} , aryle en C₆ -C₁₄ et hétéroaryle ayant de 4 à 13 atomes de carbone, l'hétéroaryle ayant 1, 2 ou 3 hétéroatomes identiques ou différents ou des groupes contenant des hétéroatomes comme éléments du cycle, qui sont choisis parmi N, NR⁸, O, S, SO et SO₂, où aryle et hétéroaryle sont non substitués ou substitués par 1, 2, 3, 4 ou 5 radicaux R⁹ identiques ou différents ;
R^{4a} R^{4b} sont choisis indépendamment l'un de l'autre parmi l'hydrogène, un groupe alkyle en C₁ -C₆ et un groupe aryle en C₆ -C₁₄ , le groupe aryle étant non substitué ou substitué par 1, 2, 3, 4 ou 5 groupes R¹⁰ identiques ou différents;
R⁷ est choisi parmi l'hydrogène, CN, nitro, halogène, alkyle en C₁ -C₆, haloalkyle en C₁ -C₆, alcoxy en C₁ -C₆, haloalcoxy en C₁ -C₆, alkylsulfanyle en C₁ -C₆, haloalkylsulfanyle en C₁ -C₆, NR^{4a} R^{4b} , aryle en C₆ -C₁₄ et hétéroaryle ayant de 4 à 13 atomes de carbone, où l'hétéroaryle a 1, 2 ou 3 hétéroatomes identiques ou différents ou des groupes contenant des hétéroatomes comme éléments du cycle, choisis parmi N, NR⁸, O, S, SO et SO₂, où les groupes aryles et hétéroaryles sont non substitués ou substitués par 1, 2, 3, 4 ou 5 radicaux R¹⁰ identiques ou différents;
R⁸ est choisi parmi l'hydrogène, les groupes alkyles en C₁ -C₆, halogénoalkyle en C₁ -C₆ et aryle en C₆ -C₁₄, le groupe aryle étant non substitué ou substitué par 1, 2, 3, 4 ou 5 groupes R¹⁰ identiques ou différents ;
R⁹ est choisi parmi CN, halogène, alkyle en C₁ -C₆, haloalkyle en C₁ - C₆ et aryle en C₆ -C₁₄, où l'aryle est non substitué ou substitué par 1, 2, 3, 4 ou 5 radicaux identiques ou différents choisis parmi alkyle en C₁ -C₄ ou haloalkyle en C₁ -C₄ ;
R¹⁰ est choisi parmi CN, un halogène, un alkyle en C₁ -C₄ et un haloalkyle en C₁ -C₄ .

3. Composant électronique selon la revendication 1 ou 2, dans lequel, dans la formule (I.1) ou dans la formule (II), R¹ , R², R⁵ et R⁶ sont choisis indépendamment les un des autres parmi l'hydrogène, CN, un alkyle en C₁ -C₆, un haloalkyle en C₁ -C₆, un alcoxy en C₁ -C₆, un haloalcoxy en C₁ -C₆, alkylsulfanyle en C₁ -C₆, haloalkylsulfanyle en C₁ -C₆, NR^{4a} R^{4b} et le groupe A, constitué de A1, A2, A3, A4, A5, A6, A7, A8, A9, A10, A11, A12, A13, A14, A15, A16, A17, A18 et A19 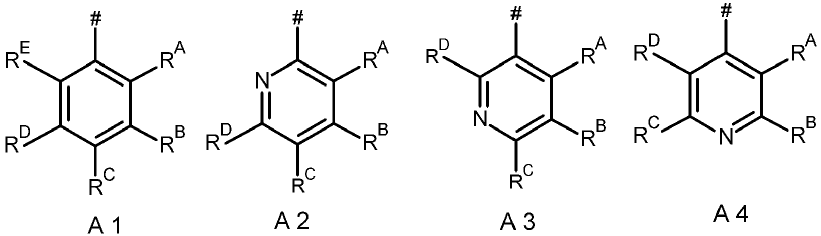 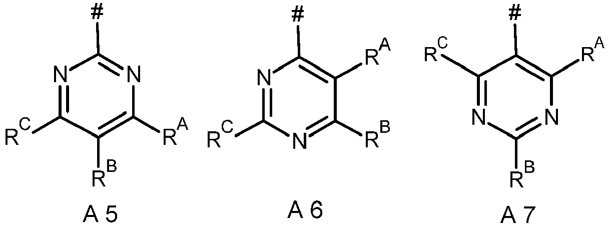 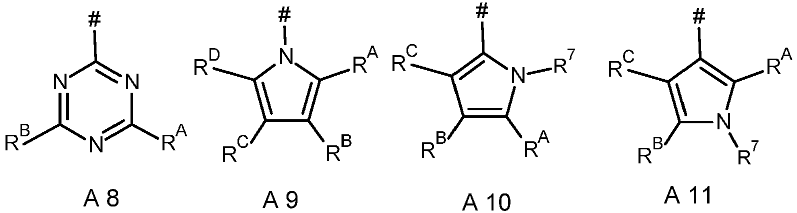 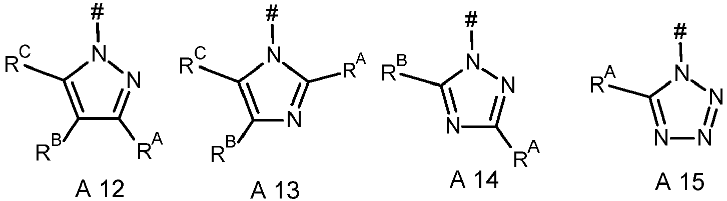 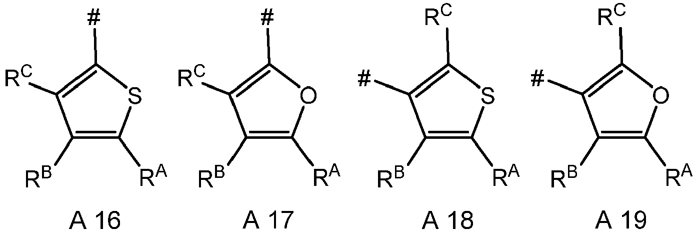
dans lesquelles # désigne la liaison au groupe de formule (I), où R^{A}, R^{B}, R^{C}, R^{D} et R^{E} sont choisis indépendamment les uns des autres parmi l'hydrogène, CN, un halogène, un alkyle en C₁ -C₄ et un haloalkyle en C₁ -C₄ ; ou
R¹ et A forment ensemble avec l'atome de carbone auquel ils sont liés un groupe aryle en C₆ -C₁₄ , le groupe aryle étant non substitué ou substitué par 1, 2, 3 ou 4 radicaux R⁹ identiques ou différents ; ou
R⁶ et B forment ensemble avec l'atome de carbone auquel ils sont liés un groupe aryle en C₆ -C₁₄ , le groupe aryle étant non substitué ou substitué par 1, 2, 3 ou 4 radicaux R⁹ identiques ou différents ;
R^{4a}, R^{4b} sont choisis indépendamment l'un de l'autre parmi l'hydrogène, un groupe alkyle en C₁ -C₆ et un groupe aryle en C₆ -C₁₄, le groupe aryle étant non substitué ou substitué par 1, 2, 3, 4 ou 5 groupes R¹⁰ identiques ou différents ;
R⁹ est choisi parmi CN, halogène, alkyle en C₁ -C₆, haloalkyle en C₁ -C₆ et aryle en C₆ -C₁₄, où l'aryle est non substitué ou substitué par 1, 2, 3, 4 ou 5 radicaux identiques ou différents choisis parmi alkyle en C₁ -C₄ ou haloalkyle en C₁ -C₄ ; et
R¹⁰ est choisi parmi CN, un halogène, un alkyle en C₁ -C₄ et un haloalkyle en C₁ -C₄.

4. Composant électronique selon l'une des revendications précédentes, dans lequel, dans la formule (II), R³ et R⁴ sont choisis indépendamment l'un de l'autre parmi un atome d'hydrogène, CN, un groupe alkyle en C₁ -C₆, un groupe haloalkyle en C₁ -C₆, un groupe alcoxy en C₁ -C₆, un groupe haloalcoxy en C₁ -C₆ et un groupe phényle, le groupe phényle étant non substitué ou substitué par 1, 2, 3 ou 4 radicaux R⁹ identiques ou différents, ou
R³ et R⁴ forment ensemble, avec les atomes de carbone auxquels ils sont liés, un groupe aryle en C₆ -C₁₄, le groupe aryle étant non substitué ou substitué par 1, 2, 3 ou 4 radicaux R⁹ identiques ou différents ;
R⁹ est choisi parmi CN, halogène, alkyle en C₁ -C₆, haloalkyle en C₁ -C₆ ou aryle en C₆ -C₁₄, où l'aryle est non substitué ou substitué par 1, 2, 3, 4 ou 5 radicaux identiques ou différents choisis parmi alkyle en C₁ -C₄ ou haloalkyle en C₁-C₄.

5. Composant électronique selon l'une quelconque des revendications précédentes, dans lequel, dans la formule (1.1) ou la formule (II), A et B représentent indépendamment l'un de l'autre un radical CR⁷ et R⁷ est choisi parmi l'hydrogène, CN, nitro, un halogène et le groupe B constitué de B1, B2, B3, B4, B5, B6, B7, B8, B9, B10, B11, B12, B13, B14 et B15. 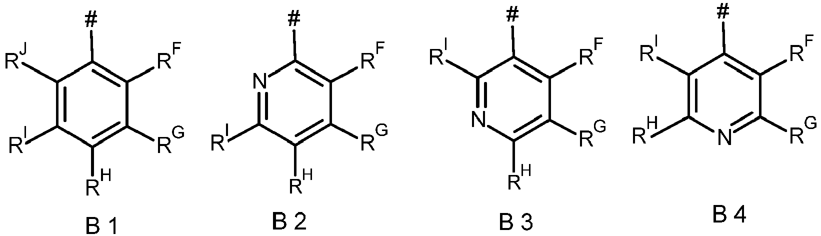 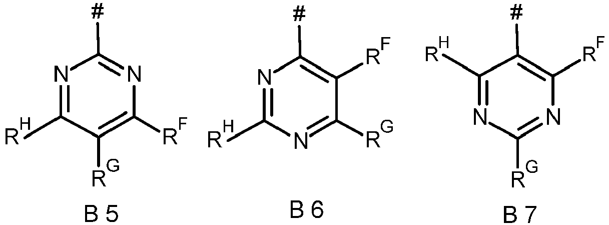 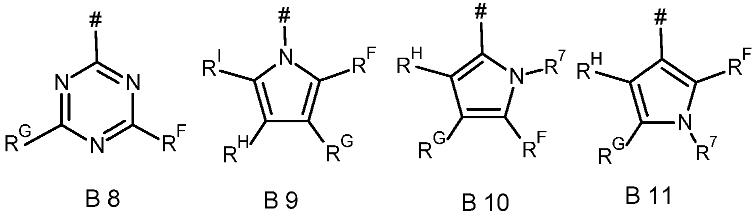 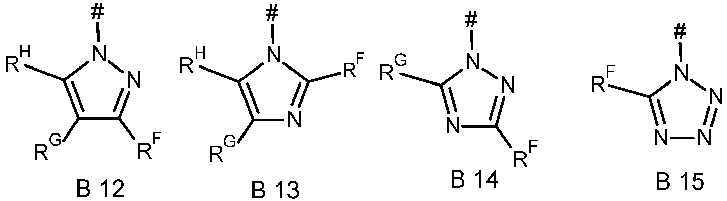
dans lesquelles # désigne la liaison au groupe de formule (II) , et dans lesquelles
R^{F}, R^{G}, R^{H}, R^{I} et R^{J} sont choisis indépendamment les uns des autres parmi l'hydrogène, CN, un halogène, un alkyle en C₁ -C₄ et un haloalkyle en C₁ -C₄ .

6. Composant électronique selon l'une quelconque des revendications précédentes, dans lequel, dans la formule (1.1) ou la formule (II), R¹ R², R⁵ et R⁶ sont choisis indépendamment les uns des autres parmi l'hydrogène, un groupe alkyle en C₁ -C₄, halogénoalkyle en C₁ -C₄, A1 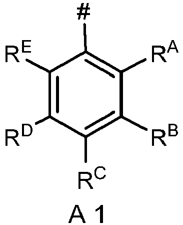
où # désigne la liaison au groupe de formule (1.1) ou de formule (II), et où R^{A}, R^{B}, R^{C}, R^{D} et R^{E} sont choisis indépendamment les uns des autres parmi l'hydrogène, CN, le fluor, le chlore, un alkyle en C₁ -C₄, un fluoroalkyle en C₁ -C₄ et un chloroalkyle en C₁ -C₄, de préférence parmi l'hydrogène, CN, le fluor et le chlore ; ou
R¹ et A forment ensemble avec l'atome de carbone auquel ils sont liés un cycle phényle, le phényle étant non substitué ou substitué par 1, 2, 3 ou 4 radicaux R⁹ identiques ou différents ;
R⁶ et B forment ensemble avec l'atome de carbone auquel ils sont liés un cycle phényle, le phényle étant non substitué ou substitué par 1, 2, 3 ou 4 radicaux R⁹ identiques ou différents ;
R⁹ est choisi parmi CN, halogène, alkyle en C₁ -C₄, haloalkyle en C₁ -C₄ ou phényle, le phényle étant non substitué ou substitué par 1, 2, 3, 4 ou 5 radicaux identiques ou différents choisis parmi alkyle en C₁ -C₄ ou haloalkyle en C₁ -C₄.

7. Composant électronique selon l'une quelconque des revendications précédentes, dans lequel, dans la formule (1.1) ou la formule (II), A et B représentent indépendamment l'un de l'autre un radical CR⁷ et R⁷ est choisi parmi l'hydrogène, CN, nitro, halogène, alkyle en C₁ -C₂ et haloalkyle en C₁ -C₂.

8. Composant électronique selon l'une quelconque des revendications précédentes, dans lequel R³ et R⁴ sont choisis indépendamment l'un de l'autre parmi l'hydrogène, CN, un alkyle en C₁ -C₄ et un haloalkyle en C₁ -C₄, ou
R³ et R⁴ forment ensemble avec les atomes de carbone auxquels ils sont liés un cycle phényle, le phényle étant non substitué ou substitué par 1, 2, 3 ou 4 radicaux R⁹ identiques ou différents ;
R⁹ est choisi parmi CN, halogène, alkyle en C₁ -C₄, haloalkyle en C₁ -C₄ et phényle, le phényle étant non substitué ou substitué par 1, 2, 3 ou 4 radicaux identiques ou différents choisis parmi alkyle en C₁ -C₄ ou haloalkyle en C₁ -C₄.

9. Composant électronique selon l'une quelconque des revendications précédentes, dans lequel, dans la formule (I), L₁ et L₂ ont la même signification.

10. Composant électronique selon l'une quelconque des revendications précédentes, sous la forme d'une diode électroluminescente organique, d'une cellule solaire organique, d'une cellule photovoltaïque, d'un capteur organique, d'une diode organique ou d'un transistor à effet de champ organique, de préférence sous la forme d'un transistor à couche mince.

11. Composant électronique selon l'une des revendications précédentes avec une structure en couches comprenant 2, 3, 4, 5, 6, 7 ou plus de couches.

12. Composant électronique selon l'une quelconque des revendications précédentes, comprenant une couche de transport de trous et/ou une couche d'injection de trous comprenant au moins un composé de formule (I).

13. Composant électronique selon l'une quelconque des revendications précédentes, comprenant une couche de transport d'électrons comprenant au moins un composé de formule (I).

14. Matériau de matrice semi-conducteur dopé comprenant au moins un donneur d'électrons et au moins un composé de formule (I) tel que défini dans l'une quelconque des revendications 1 à 9, ledit donneur d'électrons étant notamment choisi parmi 4,4',4"-tris(N-(2-naphtyl)-N-phényl-amino)triphénylamine, 4,4',4"-tris(N-3-méthylphényl-N-phényl-amino)triphénylamine, N,N,N',N'-tétrakis(4-méthoxy-phényl)benzidine, (2,2',7,7'-tétrakis-(N,N-diphénylamino)-9,9'-spirobifluorène, N,N'-bis(naphtalène-1-yl)-N,N'-bis(phényl)-benzidine, N,N'-bis(naphtalène-1-yl)-N,N'-bis(phényl)-9,9-spiro-bifluorène, 9,9-bis[4-(N,N-bis-biphényl-4-yl-amino) phényl]-9H-fluorène, 2,2'-bis[N,N-bis(biphényl-4-yl)amino]-9,9-spiro-bifluorène, N,N'-((9H-fluorène-9,9-diyl)bis(4,1-phénylène))bis(N-([1,1'-biphényl]-4-yl)-[1,1'-biphényl]-4-amine),N,N'-bis(phénanthrène-9-yl)-N,-N'-bis(phényl)-benzidine, 1,3,5 tris{4-[bis(9,9-diméthyl-fluorène-2-yl)amino]phényl}benzène, tri(terphényl-4-yl)amine, N-(4-(6-((9,9-Dimethyl-9H-fluoren-2-yl)(6-methoxy-[1,1'-biphenyl]-3-yl)amino)-1,3,3-trimethyl-2,3-dihydro-1H-inden-1-yl)phenyl)-N-(6-methoxy-[1,1'-biphenyl]-3-yl)-9,9-dimethyl-9H-fluoren-2-amin, N-([1,1'-Biphenyl]-4-yl)-N-(4-(6-([1,1'-biphe nyl]-4-yl(9,9-dimethyl-9H-fluoren-2-yl)amino)-1,3,3-trimethyl-2,3-dihydro-1H-inden-1-yl)phenyl)-9,9-dimethyl-9H-fluoren-2-amin, N,N-Di([1,1'-biphenyl]-4-yl)-3-(4-(di([1,1'-biphenyl]-4-yl)amino)phenyl)-1,1,3-trimethyl-2,3-dihydro-1H-inden-5-amin, N-(4-(6-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-(4-(6-(9,9'-Spirobi[fluoren]-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9'-spirobi[fluoren]-2-amin, N-(4-(6-(Dibenzo[b,d]furan-2-yl(9,9-diméthyl-9H-fluorène-2-yl)amino)-1,3,3-triméthyl-2,3-dihydro-1H-indène-1-yl)phényl)-N-(9,9-diméthyl-9H-fluorène-2-yl)dibenzo[b,d]furan-2-amin, 9-(4-(6-(9H-Carbazol-9-yl)-1,3,3-trimethyl-2,3-dihydro-1H-inden-1-yl)phenyl)-9H-carbazol, N-([1,1'-biphényl]-4-yl)-3-(4-([1,1'-biphényl]-4-yl(4-méthoxyphényl)amino)phényl)-N-(4-méthoxyphényl)-1,1,3-triméthyl-2,3-dihydro-1H-indène-5-amine, 3-(4-(Bis(6-methoxy-[1,1'-biphenyl]-3-yl)amino)phenyl)-N,N-bis(6-methoxy-[1,1'-biphenyl]-3-yl)-1,1,3-trimethyl-2,3-dihydro-1H-inden-5-amin, N1-([1,1'-Biphenyl]-4-yl)-N1-(4-(6-([1,1'-biphenyl]-4-yl(4-(diphenylamino)phenyl)amino)-1,3,3-trimethyl-2,3-dihydro-1H-inden-1-yl)phenyl)-N4,N4-diphenylbenzene-1,4-diamin, N,N-Di([1,1'-biphenyl]-4-yl)-4'-(6-(4-(di([1,1'-biphenyl]-4-yl)amino)phenyl)-1,3,3-trimethyl-2,3-dihydro-1H-inden-1-yl)-[1,1'-biphenyl]-4-amin, N-(4-(5-(Bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-(4-(6-(Bis(9,9-dimethyl-9H-fluoren-2-yl)amino)-1,3,3-trimethyl-2,3-dihydro-1H-inden-1-yl)phenyl)-N-(9,9-diméthyl-9H-fluorène-2-yl)-9,9-diméthyl-9H-fluorène-2-amine, N,N'-bis(9,9-diméthyl-fluorène-2-yl)-N,N'-diphényl-benzidine (BF-DPB), N,N'-((9H-fluorène-9,9-diyl)bis(4,1 -phénylène))bis(N-([1,1 '-biphényl]-4-yl)-[1,1 '-biphényl]-4-amine) (BPAPF), N4,N4,N4',N4'-tetrakis(9,9-dimethyl-9H-fluoren-2-yl)-[1,1'-biphenyl]-4,4'-diamin (TDMFB), N-([1,1'-biphenyl]-2-yl)-N-(9,9-diméthyl-9H-fluorène-2-yl)-9,9'-spirobi[fluorène]-2-amine, [2,7-bis[N,N-bis(4-méthoxyphényl)amino]-9,9-spirobi[9H-fluorène] (spiro-MeO-TPD), un mélange de N-(4-(5-(bis(9,9-diméthyl-9H-fluorène-2-yl)amino)-1,3,3-triméthyl-2,3-dihydro-1H-indène-1-yl)phényl)-N-(9,9-diméthyl-9H-fluorène-2-yl)-9,9-diméthyl-9H-fluorène-2-amine et de N-(4-(6-(bis(9,9-diméthyl-9H-fluorène-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amin, N-([1,1'-biphenyl]-4-yl)-9,9-dimethyl-N-(4-(9-phenyl-9H-carbazol-3-yl)phenyl)-9H-fluoren-2-amin et leurs mélanges.

15. Utilisation d'un composé (I) ou de ses mélanges, tel que défini dans l'une quelconque des revendications 1 à 9,
- comme semi-conducteur organique,
- comme dopant dans des matériaux de matrice semi-conducteurs organiques, en particulier comme dopant p dans des couches de transport de trous,
- comme injecteur de charge dans une couche d'injection de charge,
- comme couche de transport d'électrons,
- comme matériau de cathode dans les batteries organiques,
- comme matériau électrochrome,
- comme accepteur d'électrons.

16. Utilisation de complexes de Ce(lll) anioniques obtenus par réduction d'un composé (I) tel que défini dans l'une quelconque des revendications 1 à 9, ou de complexes de transfert de charge d'un composé (I) tel que défini dans l'une quelconque des revendications 1 à 9, avec des donneurs d'électrons, comme semi-conducteur organique, comme complexe de transfert de charge, comme matériau électrochromique ou comme ferromagnétique.

17. Composé de formule générale (I)
Ce⁴⁺[L₁L₂]₄⁻ (I),
leurs complexes de transfert de charge, leurs produits de réduction, et leurs mélanges, où L₁ et L₂ sont chacun un ligand tétradenté, qui sont choisis indépendamment l'un de l'autre parmi les ligands de formule (1.1) 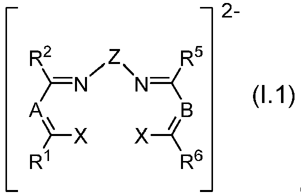 dans laquelle
X représentent O ou S indépendamment l'un de l'autre ;
Z représente un groupe pontant qui comporte 2 ou 3 atomes de carbone entre les atomes d'azote, les atomes de carbone étant à chaque fois non substitués ou substitués par 1, 2, 3, 4, 5 ou 6 radicaux R¹² identiques ou différents, deux atomes de carbone voisins pouvant également être liés l'un à l'autre par une double liaison, ou 2 ou 3 des atomes de carbone faisant partie d'un groupe mononucléaire, 2-nucléaire ou 3-nucléaire aromatique ou hétéroaromatique en Ce -C₁₄ , le système cyclique hétéroaromatique comportant 4 à 13 atomes de carbone et ayant 1, 2 ou 3 hétéroatomes identiques ou différents ou groupes contenant des hétéroatomes comme éléments du cycle, qui sont choisis parmi N, NR⁸ , O, S, SO et SO₂ et où le système cyclique aromatique ou hétéroaromatique est non substitué ou substitué par 1, 2, 3, 4 ou 5 radicaux R⁹ identiques ou différents ;
A et B représentent N ou CR⁷ indépendamment l'un de l'autre;
R¹ et R⁶ sont choisis indépendamment l'un de l'autre parmi l'hydrogène, CN, un alkyle en C₁ -C₆, un haloalkyle en C₁ -C₆, un alcoxy en C₁ - C₆, un haloalcoxy en C₁ -C₆, un cycloalkyle en C₃ -C₇, ledit le cycloalkyle en C₃ -C₇ est non substitué ou substitué par 1, 2, 3, 4, 5 ou 6 radicaux R¹¹ identiques ou différents, l'alkylsulfanyle en C₁ - C₆, l'haloalkylsulfanyle en C₁ -C₆, NR^{4a} R^{4b}, aryle en C⁶ -C¹⁴ et hétéroaryle ayant de 4 à 13 atomes de carbone, l'hétéroaryle ayant 1, 2 ou 3 hétéroatomes ou groupes contenant des hétéroatomes identiques ou différents comme éléments du cycle, choisis parmi N, NR⁸, O, S, SO et SO₂, l'aryle et l'hétéroaryle étant non substitués ou substitués par 1, 2, 3, 4 ou 5 radicaux R⁹ identiques ou différents, ou
R¹ et A forment ensemble, avec l'atome de carbone auquel ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸ , O, S, SO et SO₂, l'aryle et l'hétéroaryle étant non substitués ou substitués par 1, 2, 3 ou 4 radicaux R⁹ identiques ou différents, ou
R⁶ et B forment ensemble avec l'atome de carbone auquel ils sont liés un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, choisis parmi N, NR⁸ , O, S, SO et SO₂, l'aryle et l'hétéroaryle étant non substitués ou substitués par 1, 2, 3 ou 4 radicaux R⁹ identiques ou différents ;
R² et R⁵ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, un cycloalkyle en C₃ -C₇, où dans lequel le cycloalkyle en C₃ -C₇ est non substitué ou substitué par 1, 2, 3, 4, 5 ou 6 radicaux R¹¹ identiques ou différents, l'alkylsulfanyle en C₁ -C₆, l'haloalkylsulfanyle en C₁ -C₆, NR^{4a} R^{4b} aryle en C₆ -C₁₄ et hétéroaryle ayant de 4 à 13 atomes de carbone, l'hétéroaryle ayant 1, 2 ou 3 hétéroatomes ou groupes contenant des hétéroatomes identiques ou différents comme éléments du cycle, choisis parmi N, NR⁸, O, S, SO et SO₂, l'aryle et l'hétéroaryle étant non substitués ou substitués par 1, 2, 3, 4 ou 5 radicaux R⁹ identiques ou différents;
R² et A forment ensemble, avec l'atome de carbone auquel ils sont liés, un groupe aryle en C₆ -C₁₄ ou un groupe hétéroaryle ayant de 4 à 13 atomes de carbone, l'hétéroaryle ayant 1, 2 ou 3 hétéroatomes identiques ou différents ou groupes contenant des hétéroatomes comme éléments du cycle, qui sont choisis parmi N, NR⁸, O, S, SO et SO₂, l'aryle et l'hétéroaryle étant non substitués ou substitués par 1, 2, 3 ou 4 radicaux R⁹ identiques ou différents, ou
R⁵ et B forment ensemble avec l'atome de carbone auquel ils sont liés un groupe aryle en C₆ -C₁₄ ou un groupe hétéroaryle ayant de 4 à 13 atomes de carbone, le groupe hétéroaryle ayant 1, 2 ou 3 hétéroatomes identiques ou différents ou groupes contenant des hétéroatomes comme éléments du cycle, choisis parmi N, NR⁸ , O, S, SO et SO₂, le groupe aryle et le groupe hétéroaryle étant non substitués ou substitués par 1, 2, 3 ou 4 radicaux R⁹ identiques ou différents ;
R^{4a} R^{4b} sont choisis indépendamment l'un de l'autre parmi l'hydrogène, un groupe alkyle en C₁ -C₆ et un groupe aryle en C₆ -C₁₄, le groupe aryle étant non substitué ou substitué par 1, 2, 3, 4 ou 5 groupes R¹⁰ identiques ou différents ;
R⁷ est choisi parmi l'hydrogène, CN, nitro, halogène, alkyle en C₁-C₆, haloalkyle en C₁-C₆, alcoxy en C₁-C₆, haloalcoxy en C₁-C₆, alkylsulfanyle en C₁-C₆, haloalkylsulfanyle en C₁-C₆, NR^{4a} R^{4b} , aryle en C₆ -C₁₄ et hétéroaryle ayant de 4 à 13 atomes de carbone, où l'hétéroaryle a 1, 2 ou 3 hétéroatomes identiques ou différents ou des groupes contenant des hétéroatomes comme éléments du cycle, choisis parmi N, NR⁸, O, S, SO et SO₂, où ledit aryle et ledit hétéroaryle sont non substitués ou substitués par 1, 2, 3, 4 ou 5 radicaux R¹⁰ identiques ou différents ;
R⁸ est choisi parmi l'hydrogène, les groupes alkyle en C₁-C₆, halogénoalkyle en C₁-C₆ et aryle en C₆ -C₁₄, les groupes aryle étant non substitués ou substitués par 1, 2, 3, 4 ou 5 groupes R¹⁰ identiques ou différents ;
R⁹ est choisi parmi CN, halogène, alkyle en C₁-C₆, haloalkyle en C₁-C₆ et aryle en C₆ -C₁₄, où l'aryle est non substitué ou substitué par 1, 2, 3, 4 ou 5 radicaux identiques ou différents choisis parmi alkyle en C₁ -C₄ ou haloalkyle en C₁ -C₄ ;
R¹⁰ est choisi parmi CN, un halogène, un alkyle en C₁ -C₄ et un haloalkyle en C₁ -C₄ ;
R¹¹ est choisi parmi CN, un halogène, un alkyle en C₁ -C₄ et un haloalkyle en C₁ -C₄ ;
R¹² sont choisis indépendamment les uns des autres parmi un halogène, CN, un alkyle en C₁ -C₆, un haloalkyle en C₁ -C₆, un alcoxy en C₁ -C₆, un haloalcoxy en C₁ -C₆, un cycloalkyle en C₃ - C₇, où ledit le cycloalkyle en C₃ -C₇ est non substitué ou substitué par 1, 2, 3, 4, 5 ou 6 radicaux R¹¹ identiques ou différents, l'alkylsulfanyle en C₁ -C₆, l'haloalkylsulfanyle en C₁ -C₆, NR^{4a} R^{4b}, aryle en C₆ -C₁₄ et hétéroaryle ayant de 4 à 13 atomes de carbone, l'hétéroaryle ayant 1, 2 ou 3 hétéroatomes identiques ou différents ou groupes contenant des hétéroatomes comme éléments du cycle, choisis parmi N, NR⁸, O, S, SO et SO₂, l'aryle et l'hétéroaryle étant non substitués ou substitués par 1, 2, 3, 4 ou 5 radicaux R⁹ identiques ou différents ;
les composés suivants et leurs produits de réduction étant exclus :

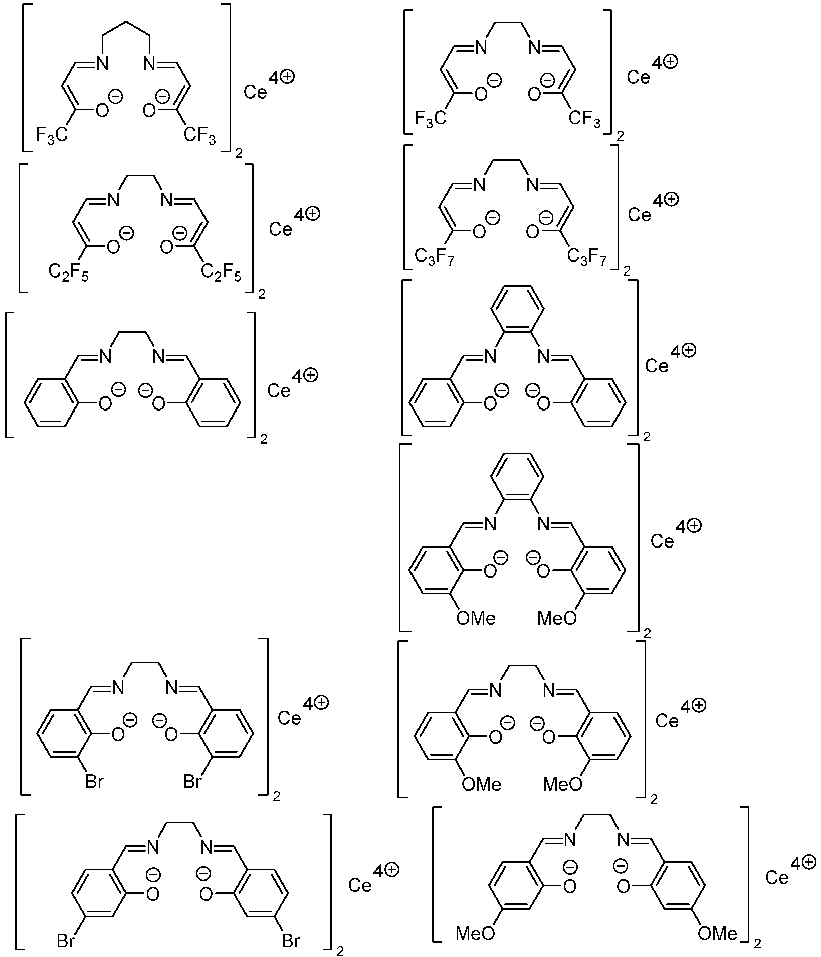

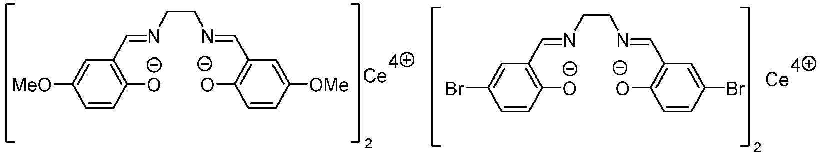

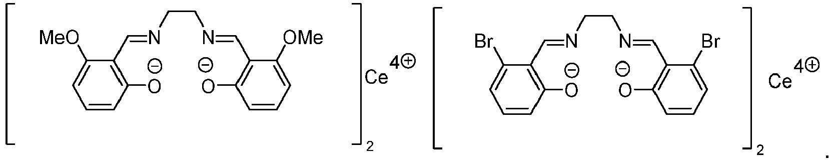

18. Composé selon la revendication 17, de formule générale (I)
Ce⁴⁺[L₁L₂]⁴⁻ (I),
leurs complexes de transfert de charge, leurs produits de réduction, et leurs mélanges, où L₁ et L₂ sont chacun un ligand tétradenté choisi indépendamment l'un de l'autre parmi les ligands de formule (II) 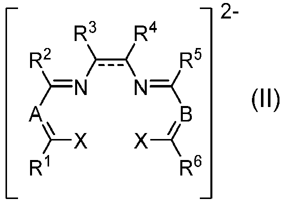 dans laquelle
-̅ -̅ -̅ -̅ -̅ -̅ représente une liaison simple ou double entre le groupe CR³ -CR⁴,
X représentent O ou S indépendamment l'un de l'autre ;
A et B représentent N ou CR⁷ indépendamment l'un de l'autre;
R¹ et R⁶ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, un alkylsulfanyle en C₁ -C₆, un haloalkylsulfanyle en C₁ -C₆, NR^{4a} R^{4b} , un aryle en C₆ -C₁₄ - et hétéroaryle avec 4 à 13 atomes de carbone, où hétéroaryle 1,2 ou 3 hétéroatomes identiques ou différents ou des groupes contenant des hétéroatomes comme éléments du cycle, qui sont choisis parmi N, NR⁸ , O, S, SO et SO₂, où aryle et hétéroaryle sont non substitués ou substitués par 1, 2, 3, 4 ou 5 radicaux R⁶ identiques ou différents, ou
R¹ et A forment ensemble avec l'atome de carbone auquel ils sont liés un groupe aryle C₆ -C₁₄ ou un groupe hétéroaryle ayant de 4 à 13 atomes de carbone, l'hétéroaryle ayant 1, 2 ou 3 hétéroatomes ou groupes contenant des hétéroatomes identiques ou différents comme éléments du cycle, qui sont choisis parmi N, NR⁸, O, S, SO et SO₂, ledit aryle et ledit hétéroaryle sont non substitués ou substitués par 1, 2, 3, ou 4 radicaux R⁹ identiques ou différents, ou
R⁶ et B forment ensemble avec l'atome de carbone auquel ils sont liés un groupe un aryle en C₆ -C₁₄ ou un groupe hétéroaryle ayant de 4 à 13 atomes de carbone, l'hétéroaryle ayant 1, 2 ou 3 hétéroatomes ou groupes contenant des hétéroatomes identiques ou différents comme éléments du cycle, choisis parmi N, NR⁸, O, S, SO et SO₂, l'aryle et l'hétéroaryle étant non substitués ou substitués par 1, 2, 3 ou 4 radicaux R⁹ identiques ou différents ;
R³ et R⁴ sont choisis indépendamment l'un de l'autre parmi l'hydrogène, CN, un alkyle en C₁ -C₆, un haloalkyle en C₁ -C₆, un alcoxy en C₁ -C₆, un haloalcoxy en C₁ -C₆, un alkylsulfanyle en C₁ -C₆, un haloalkylsulfanyle en C₁ -C₆, NR^{4a} R^{4b} , un aryle en C₆ -C₁₄ et hétéroaryle avec 4 à 13 atomes de carbone, où hétéroaryle 1, 2 ou 3 hétéroatomes identiques ou différents ou des groupes contenant des hétéroatomes comme éléments du cycle, qui sont choisis parmi N, NR⁸, O, S, SO et SO₂, où aryle et hétéroaryle sont non substitués ou substitués par 1, 2, 3, 4 ou 5 radicaux R⁹ identiques ou différents, ou
R³ et R⁴ forment ensemble avec les carbones auxquels ils sont liés un groupe aryle C₆ -C₁₄ ou un groupe hétéroaryle ayant de 4 à 13 atomes de carbone, l'hétéroaryle ayant 1, 2 ou 3 hétéroatomes ou groupes contenant des hétéroatomes identiques ou différents comme éléments du cycle, qui sont choisis parmi N, NR⁸, O, S, SO et SO₂, où aryle et hétéroaryle sont non substitués ou substitués par 1, 2, 3 ou 4 radicaux R⁹ identiques ou différents ;
R² et R⁵ sont choisis indépendamment l'un de l'autre parmi l'hydrogène, CN, un alkyle en C₁ -C₆, un haloalkyle en C₁ -C₆, un alcoxy en C₁ - C₆,un haloalcoxy en C₁ -C₆, un alkylsulfanyle en C₁ -C₆, un haloalkylsulfanyle en C₁ -C₆, NR^{4a} R^{4b} , un aryle en C₆ -C₁₄ et hétéroaryle avec 4 à 13 atomes de carbone, où hétéroaryle 1, 2 ou 3 hétéroatomes identiques ou différents ou des groupes contenant des hétéroatomes comme éléments du cycle, qui sont choisis parmi N, NR⁸ , O, S, SO et SO₂, où aryle et hétéroaryle sont non substitués ou substitués par 1, 2, 3, 4 ou 5 radicaux R⁹ identiques ou différents;
R^{4a} R^{4b} sont choisis indépendamment l'un de l'autre parmi l'hydrogène, un alkyle en C₁ -C₆ et un aryle en C₆ -C₁₄, ledit aryle est non substitué ou substitué par 1, 2, 3, 4 ou 5 radicaux R¹⁰ identiques ou différents,
R⁷ est choisi parmi l'hydrogène, CN, nitro, halogène, un alkyle en C₁ - C₆, un haloalkyle en C₁ -C₆, un alcoxy en C₁ -C₆, un haloalcoxy en C₁ -C₆, un alkylsulfanyle en C₁ -C₆, un haloalkyl-sulfanyle C₁-C₆, NR^{4a} R^{4b} , un aryle en C₆ -C₁₄ et hétéroaryle avec 4 à 13 atomes de carbone, où l'hétéroaryle a 1, 2 ou 3 hétéroatomes identiques ou différents ou des groupes contenant des hétéroatomes comme éléments du cycle, choisis parmi N, NR⁸, O, S, SO et SO₂, ledit aryle et hétéroaryle sont non substitués ou substitués par 1, 2, 3, 4 ou 5 radicaux R¹⁰ identiques ou différents ;
R⁸ est choisi parmi l'hydrogène, un alkyle en C₁ -C₆, un haloalkyle en C₁ -C₆ et un aryle en C₆ -C₁₄, ledit aryle est non substitué ou substitué par 1, 2, 3, 4 ou 5 radicaux R¹⁰ identiques ou différents ;
R⁹ est choisi parmi CN, halogène, un alkyle en C₁ -C₆, un haloalkyle en C₁ -C₆ et un aryle en C₆ -C₁₄, ledit aryle est non substitué ou substitué par 1, 2, 3, 4 ou 5 radicaux identiques ou différents choisis parmi un alkyle en C₁ -C₄ ou un haloalkyle en C₁ -C₄;
R¹⁰ est choisi parmi CN, un halogène, - un alkyle en C₁ -C₄ et un haloalkyle en C₁ -C₄,
les composés suivants et leurs produits de réduction étant exclus :
